# EUROPÄISCHE PATENTSCHRIFT

(11) **EP 2 150 556 B1**
(45) Veröffentlichungstag und Bekanntmachung des Hinweises auf die Patenterteilung: **12.01.2011**
(21) Anmeldenummer: 08749630.3
(22) Anmeldetag: 21.04.2008
(51) Int. Cl.: C07F 7/08, H05B 33/14

(54) **SILANE ENTHALTEND PHENOTHIAZIN-S-OXID ODER PHENOTHIAZIN-S,S-DIOXID-GRUPPEN UND DEREN VERWENDUNG IN OLEDS**
SILANES CONTAINING PHENOTHIAZINE-S-OXIDE OR PHENOTHIAZINE-S,S-DIOXIDE GROUPS AND THE USE THEREOF IN OLEDS
SILANES CONTENANT DES GROUPES PHÉNOTHIAZINE-S-OXYDE OU PHÉNOTHIAZINE-S,S-DIOXYDE ET UTILISATION DE CEUX-CI DANS DES DIODES ÉLECTROLUMINESCENTES ORGANIQUES

(30) Priorität: 26.04.2007 EP 07107055
(43) Veröffentlichungstag der Anmeldung: 10.02.2010
(73) Patentinhaber: BASF SE, 67056 Ludwigshafen (DE)
(72) Erfinder: MOONEN, Nicolle, 68163 Mannheim (DE); KAHLE,Klaus, 67069 Ludwigshafen (DE); LENNARTZ, Christian, 67105 Schifferstadt (DE); SCHILDKNECHT, Christian, 68305 Mannheim (DE); NORD, Simon, 76189 Karlsruhe (DE); MOLT, Oliver, 69493 Hirschberg (DE); FUCHS, Evelyn, 68199 Mannheim (DE); RUDOLPH, Jens, 67547 Worms (DE); WAGENBLAST, Gerhard, 67157 Wachenheim (DE)
(86) Internationale Anmeldenummer: PCT/EP2008/054801
(87) Internationale Veröffentlichungsnummer: WO 2008/132085

(56) Entgegenhaltungen:
- US-A1- 2005 214 572
- US-B1- 6 307 083

## Beschreibung

Die vorliegende Erfindung betrifft Silane enthaltend Phenothiazin-S-oxid- oder Phenothiazin-S,S-dioxid-Gruppen, organische Leuchtdioden enthaltend die erfindungsgemäßen Silane, eine Licht-emittierende Schicht enthaltend mindestens ein erfindungsgemäßes Silan und mindestens einen Triplett-Emitter, eine Blockerschicht enthaltend mindestens ein erfindungsgemäßes Silan, Verfahren zur Herstellung der erfindungsgemäßen Silane und die Verwendung der erfindungsgemäßen Silane in organischen Leuchtdioden, bevorzugt als Matrixmaterialien und/oder als Blockermaterialien für Triplett-Emitter.

In organischen Leuchtdioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen. Aufgrund der sehr kompakten Bauweise und des intrinsisch niedrigen Stromverbrauchs eignen sich Vorrichtungen enthaltend OLEDs insbesondere für mobile Anwendungen, z. B. für Anwendungen in Handys, Laptops, usw. sowie zur Beleuchtung.

Die Grundprinzipien der Funktionsweise von OLEDs sowie geeignete Aufbauten (Schichten) von OLEDs sind dem Fachmann bekannt und zum Beispiel in WO 2005/113704 und der darin zitierten Literatur genannt. Als Licht-emittierende Materialien (Emitter) können neben fluoreszierenden Materialien (Fluoreszenz-Emitter) phosphoreszierende Materialien (Phosphoreszenz-Emitter) eingesetzt werden. Bei den Phosphoreszenz-Emittern handelt es sich üblicherweise um metallorganische Komplexe, die im Gegensatz zu den Fluoreszenz-Emittern, die eine Singulett-Emission zeigen, eine Triplett-Emission zeigen (Triplett-Emitter) (M. A. Baldow et al., Appl. Phys. Lett. 1999. 75, 4 bis 6). Aus quantenmechanischen Gründen ist bei Verwendung der Triplett-Emitter (Phosphoreszenz-Emitter) eine bis zu vierfache Quanten-, Energie- und Leistungseffizienz möglich. Um die Vorteile des Einsatzes der metallorganischen Triplett-Emitter (Phosphoreszenz-Emitter) in die Praxis umzusetzen, ist es erforderlich, Device-Kompositionen bereitzustellen, die eine hohe operative Lebensdauer, eine hohe Stabilität gegenüber Temperaturbelastung und eine niedrige Einsatz- und Betriebsspannung aufweisen.

Solche Device-Kompositionen können z. B. Matrixmaterialien enthalten, in denen der eigentliche Lichtemitter in verteilter Form vorliegt. Des Weiteren können die Device-Kompositionen Blockermaterialien enthalten, wobei Loch-, Excitonen- und/oder Elektronenblocker in den Device-Kompositionen vorliegen können. Dabei hat die Auswahl des Matrixmaterials und der eingesetzten Blockermaterialien einen wesentlichen Einfluss u. a. auf die Leuchtdichten- und Quantenausbeuten der OLEDs.

Im Stand der Technik werden zahlreiche verschiedene Materialien für den Einsatz in OLEDs vorgeschlagen. Unter den vorgeschlagenen Materialien sind auch solche, die substituierte, insbesondere Aryl-substituierte, Silane aufweisen.

So betrifft US 2005/0214 572 A1 OLEDs, die, bevorzugt als Matrixmaterial in der lumineszierenden Schicht, mindestens ein Arylsilan enthalten. Das Arylsilan weist mindestens zwei Arylreste auf, die mit einem stickstoffhaltigen Heterozyklus substituiert sind. Der Einsatz von Arylsilanen, die Phenothiazin-S-oxid- oder Phenothiazin-S,S-dioxid-Substituenten aufweisen, ist in US 2005/0214 572 A1 nicht offenbart.

WO 2004/095 598 A2 betrifft OLEDs, deren Licht-emittierende Schicht Matrixmaterialien aufweist, die eine große Energielücke von mindestens 3.2 eV aufweisen. Als Matrixmaterialien sind in WO 2004/095 598 A2 u. a. Arylsilane erwähnt. Der Einsatz von Phenothiazin-S-oxid- bzw. Phenothiazin-S,S-dioxid-substituierten Arylsilanen als Matrixmaterialien und/oder Blockertnaterialien ist in WO 2004/095 598 A2 jedoch nicht erwähnt.

JP 2005/22 00 88 A2 betrifft Arylsilane, deren Arylreste Stickstoff enthaltende Substituenten tragen. Die Verbindungen gemäß JP 2005/22 00 88 A2 weisen eine Energielücke auf, die größer als 3.0 eV ist. Gemäß JP 2005/22 00 88 A2 werden die genannten Arylsilane als Lochtransportmaterialien in OLEDs eingesetzt. Der Einsatz als Matrixmaterialien und/oder in OLEDs ist in JP 2005/22 00 88 A2 nicht erwähnt. Des Weiteren sind in JP 2005/22 00 88 A2 keine Arylsilane offenbart, deren Arylreste Phenothiazin-S-oxid- bzw. Phenothiazin-S,S-dioxid-Substituenten tragen.

JP 2002/30 88 37 A2 betrifft Verbindungen, die Lochtransport-Eigenschaften aufweisen. Bei diesen Verbindungen kann es sich gemäß JP 2002/30 88 37 A2 um Arylsilane handeln, deren Arylgruppen mit Stickstoff-Heterozyklen substituiert sind. Eine Substitution der Arylgruppen mit Phenothiazin-S-oxid- bzw. Phenothiazin-S,S-dioxid-Substituenten ist in JP 2002/30 88 37 A2 nicht erwähnt. Des Weiteren werden die Verbindungen gemäß JP 2002/30 88 37 A2 als Lochtransportmaterialien eingesetzt. Der Einsatz der Verbindungen als Matrixmaterialien in der Licht-emittierenden Schicht und/oder Blockermaterialien ist in JP 2002/30 88 37 A2 nicht erwähnt.

WO 03/017732 A1 betrifft OLEDs, die eine polymerisierbare amorphe Matrix enthalten, worin ein Licht-emittierendes Material vorliegt. Die die polymerisierbare Matrix bildende Grundstruktur enthält Arylsilan-Einheiten, deren Arylreste u. a. mit Heteroarylgruppen substituiert sein können. Eine Substitution der Arylreste mit Phenothiazin-S-oxid- bzw. Phenothiazin-S,S-dioxid-Gruppen ist in WO 03/017732 A1 nicht erwähnt. Des Weiteren handelt es sich bei der Matrix gemäß WO 03/017732 A1 um eine polymerisierbare amorphe Matrix.

In US 6,194,089 B1 ist ein OLED offenbart, das eine organische Licht-emittierende Schicht aufweist, die ein kontinuierliches organisches Medium AₓB_{y}C_{z} aufweist. Dabei ist A ein Elektronen-transportierendes Material, B ein Löcher-transportierendes Material und C ein Löcher-injizierendes Material. Die Materialien A, B und C können in dem kontinuierlichen organischen Medium in verschiedenen Konzentrationsgradienten innerhalb des Mediums vorliegen. Bei der Komponente B kann es sich u. a. um ein Arylsilan handeln, dessen Arylgruppen mit aromatischen tertiären Aminogruppen substituiert sein können. Arylsilane, deren Arylgruppen mit Phenothiazin-S-oxid- oder Phenothiazin-S,S-dioxid-Substituenten substituiert sind, sind in US 6,194,089 B1 nicht erwähnt.

In EP 0 774 883 A2 sind OLEDs offenbart, die eine Lochtransportschicht aufweisen, die 2 oder mehr Lochtransportmaterialien in einer Mischung enthält. Als Lochtransportmaterialien können Arylsilane eingesetzt werden, wobei die Arylgruppen mit tertiären Amineinheiten substituiert sein können. Bezüglich des Einsatzes von Arylsilanen, deren Arylgruppen mit Phenothiazin-S-oxid- oder Phenothiazin-S,S-dioxid-Einheiten substituiert sind, ist EP 0 774 883 A2 keine Information zu entnehmen. Des Weiteren werden die Arylsilane gemäß EP 0 774 883 A2 in der Löcher-transportierenden Schicht als Lochtransportmaterialien eingesetzt und nicht als Matrixmaterialien in der Licht-emittierenden Schicht und/oder als Blockermaterialien.

Aufgabe der vorliegenden Anmeldung gegenüber dem Stand der Technik ist es daher, neuartige Matrixmaterialien und neuartige Blockermaterialien für den Einsatz in OLEDs, insbesondere in den Licht-emittierenden Schichten der OLEDs, bereitzustellen, die bevorzugt als Matrixmaterialien und/oder Blockermaterialien für Triplett-Emitter dienen. Die Materialien sollen leicht zugänglich sein und in Kombination mit dem (den) Emitter(n) gute Leuchtdichten und Quantenausbeuten in OLEDs bewirken.

Diese Aufgabe wird gelöst durch die Bereitstellung von Verbindungen der allgemeinen Formel I 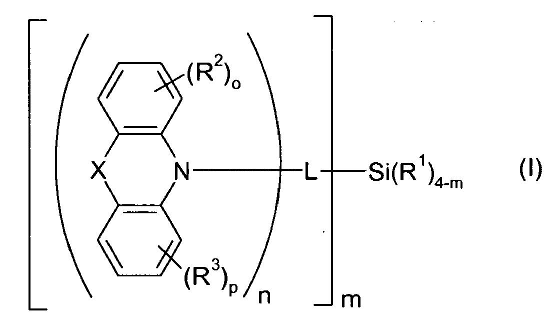 worin bedeuten
- X: SO₂ oder SO, bevorzugt SO₂;
- R¹: jeweils unabhängig voneinander gegebenenfalls substituiertes Aryl, gege- benenfalls substituiertes Heteroaryl oder gegebenenfalls substituiertes Al- kyl;
- R², R³: jeweils unabhängig voneinander gegebenenfalls substituiertes Alkyl, gege- benenfalls substituiertes Aryl, gegebenenfalls substituiertes Heteroaryl; o- der Substituenten mit Donor- oder Akzeptorwirkung, wie Alkoxy, Aryloxy, Arylcarbonyloxy (-C=O(OR)), -C=O(SR), Heteroaryl, Hydroxy, Amino, Ha- logen, -C=O(R), -OC=O(R), -SC=O(R), Amido (-C=O(NR)), -NRC=O(R), Sulfonyl, Sulfonamid, Vinyl, CN, Nitro, Thioalkoxy, Thioaryloxy oder SiR₃, wobei R jeweils unabhängig voneinander Wasserstoff, Alkyl oder Aryl be- deutet;
- m: 1, 2, 3 oder 4, bevorzugt 2, 3 oder 4;
- n: 1 oder 2;
- o, p: unabhängig voneinander 0, 1, 2, 3 oder 4, bevorzugt 0, 1 oder 2, beson- ders bevorzugt 0;
- L: verbrückende Gruppe ausgewählt aus der Gruppe bestehend aus 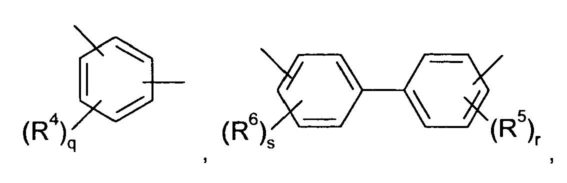 -CH₂-(B)ⱼ- und gegebenenfalls substituiertem Heteroarylen;
- R⁴, R⁵, R⁶: jeweils unabhängig voneinander gegebenenfalls substituiertes Alkyl, gege- benenfalls substituiertes Aryl, gegebenenfalls substituiertes Heteroaryl; o- der Substituenten mit Donor- oder Akzeptorwirkung, wie Alkoxy, Aryloxy, Arylcarbonyloxy (-C=O(OR)), -C=O(SR), Heteroaryl, Hydroxy, Amino, Ha- logen, -C=O(R), -OC=O(R), -SC=O(R), Amido (-C=O(NR)), -NRC=O(R), Sulfonyl, Sulfonamid, Vinyl, CN, Nitro, Thioalko- xy, Thioaryloxy oder SiR₃, wobei R jeweils unabhängig voneinander Was- serstoff, Alkyl oder Aryl bedeutet;
- q, r, s: unabhängig voneinander 0, 1, 2, 3, oder 4, bevorzugt 0, 1 oder 2, beson- ders bevorzugt 0;
- B: eine Alkylengruppe -CₖH₂ₖ-CH₂-, worin eine oder mehrere nicht benachbar- te CH₂-Gruppen der Einheit -CₖH₂ₖ- durch Sauerstoff oder NR⁷ ersetzt sein können;
- R⁷: Aryl oder Alkyl;
- k: 1, 2, 3, 4, 5, 6, 7 oder 8; und
- j: 0 oder 1.

Die erfindungsgemäßen Verbindungen der Formel I sind insbesondere als Matrixmaterialien und/oder Blockermaterialien für den Einsatz in OLEDs geeignet. Bevorzugt werden sie als Matrixmaterialien in der Licht-emittierenden Schicht gemeinsam mit dem eigentlichen Emitter eingesetzt. Besonders bevorzugt ist der Emitter, der mit den Matrixmaterialien gemeinsam in der Licht-emittierenden Schicht eines OLEDs eingesetzt wird, ein Triplett-Emitter. In einer weiteren Ausführungsform werden die Verbindungen der Formel (I) als Loch-/Photonen-Excitonenblocker eingesetzt.

Die Verbindungen der Formel I sind leicht zugänglich und weisen, sowohl bei Einsatz als Matrixmaterialien als auch bei Einsatz als Blockermaterialien in Kombination mit dem (den) eigentlichen Emitter(n), gute Leuchtdichten und Quantenausbeuten bei Einsatz in OLEDs auf.

In Abhängigkeit von ihrem Substitutionsmuster können die Verbindungen der Formel (I) entweder als elektronenleitende Matrix und/oder Loch-/Excitonenblocker oder als Iochleitende bzw. ambipolare Matrix und/oder Elektronen-/Excitonenblocker eingesetzt werden.

### Elektronenleitende Matrix und/oder Loch-/Excitonenblocker

Verbindungen der Formel I, sie keine elektronenschiebenden Substituenten R², R³, R⁴, R⁵, R⁶ (d. h. Substituenten mit + I und/oder +M-Effekt aufweisen), können im Wesentlichen nur Elektronen leiten und werden daher im Allgemeinen als elektronenleitende Matrix und/oder Loch-/Excitonenblocker eingesetzt.

### Ambipolare Matrix und/oder entweder Loch-/Excitonenblocker oder Elektronen-/Excitonenblocker

Verbindungen der Formel I, die elektronenschiebende Substituenten R², R³, R⁴, R⁵, R⁶ aufweisen, können sowohl Elektronen als auch Löcher leiten. Sie können daher als Elektronen- und lochleitende Matrix eingesetzt werden (ambipolare Matrix). In Abhängigkeit von dem System (OLED-Aufbau) können sie als Loch-/Excitonenblocker oder als Elektronen-/Excitonenblocker eingesetzt werden. Dabei ist die Eignung der Verbindungen der Formel I als lochleitende Matrix und/oder Elektronen-/Excitonenblocker im Allgemeinen umso besser, je mehr elektronenschiebende Substituenten in den Verbindungen der Formel vorliegen.

Die Ausdrücke elektronenschiebende Substituenten (+I- und/oder + M-Effekt) und elektronenziehende Substituenten (-I- und/oder -M-Effekt) werden in der vorliegenden Anmeldung im üblichen, dem Fachmann bekannten Sinn verwendet. Geeignete elektronenschiebende und elektronenziehene Substituenten sind z. B. Aminogruppen, Alkoxygruppen, Halogensubstituenten, Aryloxygruppen, Arylcarbonyloxygruppen, Heteroarylgruppen, Hydroxygruppen, -C=O(R), -OC=O(R), -SC=O(R), Amidogruppen, -NRC=O(R), Sulfongruppen, Sulfonamidgruppen, Vinylgruppen, CN, Nitrogruppen, Thioalkoxygruppen, Thioaryloxygruppen oder SiR₃, wobei R jeweils Wasserstoff, Alkyl oder Aryl bedeutet oder halogenierte Alkylgruppen, z. B. CF₃.

Bevorzugt werden die Verbindungen der Formel (I) als elektronenleitende Matrix und/oder Loch-Excitonenblocker eingesetzt. Das bedeutet, in einer bevorzugten Ausführungsform betrifft die vorliegende Erfindung Verbindungen der Formel (I), die keine elektronenschiebenden Substituenten R², R³, R⁴, R⁵, R⁶ aufweisen.

Die Alkylreste sowie die Alkylreste der Alkoxygruppen gemäß der vorliegenden Anmeldung können sowohl geradkettig als auch verzweigt oder cyclisch und/oder optional substituiert sein mit Substituenten ausgewählt aus der Gruppe bestehend aus Aryl, Alkoxy und Halogen. Geeignete Arylsubstituenten sind nachstehend genannt. Beispiele für geeignete Alkylgruppen sind Methyl, Ethyl, Propyl, Butyl, Pentyl, Hexyl, Heptyl und Octyl sowie mit Aryl-, Alkoxy- und/oder Halogen, insbesondere F, substituierte Derivate der genannten Alkylgruppen wie CF₃. Dabei sind sowohl die n-Isomere dieser Reste als auch verzweigte Isomere wie Isopropyl, Isobutyl, Isopentyl, sec-Butyl, tert-Butyl, Neopentyl, 3,3-Dimethylbutyl, 2-Ethylhexyl usw. mit umfasst. Bevorzugte Alkylgruppen sind Methyl, Ethyl, tert-Butyl und CF₃.

Die cyclischen Alkylreste gemäß der vorliegenden Anmeldung können optional substituiert sein mit Substituenten ausgewählt aus der Gruppe bestehend aus Aryl, Alkoxy und Halogen. Bevorzugt sind die cyclischen Alkylreste unsubstituiert. Geeignete Arylsubstituenten sind nachstehend genannt. Beispiele für geeignete cyclische Alkylreste sind Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantyl. Die cyclischen Alkylreste können unsubstituiert oder gegebenenfalls mit einem oder mehreren weiteren Resten, insbesondere Alkyl, Aryl, Alkoxy und/oder Halogen, substituiert sein.

Geeignete Halogensubstituenten im Sinne der vorliegenden Anmeldung sind Fluor, Chlor, Brom und Jod, bevorzugt Fluor, Chlor und Brom, besonders bevorzugt Fluor und Chlor.

Geeignete Alkoxy- und Thioalkoxygruppen leiten sich entsprechend von den Alkylresten ab, wie sie vorstehend definiert wurden. Beispielsweise sind hier zu nennen OCH₃, OC₂H₅, OC₃H₇, OC₄H₉ und OC₈H₁₇ sowie SCH₃, SC₂H₅, SC₃H₇, SC₄Hg₉ und SC₈H₁₇. Dabei sind unter C₃H₇, C₄H₉ und C₈H₁₇ sowohl die n-Isomere als auch verzweigte Isomere wie iso-Propyl, iso-Butyl, sec-Butyl, tert-Butyl und 2-Ethylhexyl umfasst. Besonders bevorzugt sind Methoxy, Ethoxy, n-Octyloxy, 2-Ethylhexyloxy und SCH₃.

Als Aryl werden in der vorliegenden Erfindung Reste bezeichnet, die von monocyclischen, bicyclischen oder tricyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind, möglich. Das heißt, die Bezeichnung Aryl umfasst in der vorliegenden Erfindung beispielsweise auch bicyclische oder tricyclische Reste, in denen sowohl beide oder alle drei Reste aromatisch sind als auch bicyclische oder tricyclische Reste, in denen nur ein Ring aromatisch ist, sowie tricyclische Reste, worin zwei Ringe aromatisch sind. Beispiele für Aryl sind: Phenyl, Naphthyl, Indanyl, 1,2-Dihydronaphth-enyl, 1,4-Dihydronaphthenyl, Indenyl, Anthracenyl, Phenanthrenyl oder 1,2,3,4-Tetrahydronaphthyl. Besonders bevorzugt ist Aryl Phenyl oder Naphthyl, ganz besonders bevorzugt Phenyl.

Die Arylreste können unsubstituiert oder mit einem oder mehreren weiteren Resten substituiert sein. Geeignete weitere Reste sind ausgewählt aus der Gruppe bestehend aus Alkyl, Aryl oder Substituenten mit Donor- oder Akzeptorwirkung wie Alkoxy, Aryloxy, Arylcarbonyloxy, Heteroaryl, Hydroxy, Amino, Halogen, -C=O(R), -OC=O(R), -SC=O(R), Amido(-C=O(NR)), -NRC=O(R), Sulfon, Sulfonamid, Vinyl, CN, Nitro, Thioalkoxy, Thioaryloxy oder SiR₃, wobei R jeweils unabhängig voneinander Wasserstoff, Alkyl oder Aryl bedeutet. Bevorzugt sind die Arylreste unsubstituiert oder mit einer oder mehreren Alkoxygruppen, Cyano oder CF₃ oder F substituiert. Besonders bevorzugt bedeutet Aryl unsubstituiertes Phenyl, 4-Alkylphenyl, 4-Alkoxyphenyl, 2,4,6-Trialkylphenyl, 2,4,6-Trialkoxyphenyl oder N,N-Diarylaminophenyl, bevorzugt 4-Methylphenyl, 4-Methoxyphenyl, 2,4,6-Trimethylphenyl, 2,4,6-Trimethoxyphenyl, 9-Phenylcarbazolyl sowie die entsprechenden benzanellierten Reste.

Geeignete Aryloxy-, Arylthio- und Arylcarbonyloxygruppen leiten sich entsprechend von den Arylresten ab, wie sie vorstehend definiert wurden. Besonders bevorzugt ist Phenoxy, Phenylthio und Phenylcarbonyloxy.

Geeignete Aminogruppen weisen die allgemeine Formel -NR'R" auf, wobei R' und R" unabhängig voneinander Alkyl oder Aryl bedeuten. Geeignete Alkyl und Arylreste, die jeweils gegebenenfalls substituiert sein können, sind vorstehend genannt. Beispiele für geeignete Aminogruppen sind Diarylaminogruppen wie Diphenylamino und Dialkylaminogruppen wie Dimethylamino, Diethylamino- Arylalkylamino wie Phenylmethylamino.

Unter Heteroaryl sind monocyclische, bicyclische oder tricyclische Heteroaromaten zu verstehen, die sich zum Teil vom vorstehend genannten Aryl ableiten lassen, indem im Aryl-Grundgerüst mindestens ein Kohlenstoffatom durch ein Heteroatom ersetzt ist. Bevorzugte Heteroatome sind N, O und S. Insbesondere bevorzugt ist das Grundgerüst ausgewählt aus Systemen wie Pyridin und fünfgliedrigen Heteroaromaten wie Thiophen, Pyrrol, Imidazol oder Furan. Diese Grundgerüste können gegebenenfalls mit einem oder zwei 6-gliedrigen aromatischen Resten anelliert sein. Geeignete Systeme sind Carbazolyl, Benzimidazolyl, Benzofuryl, Dibenzofuryl oder Dibenzothiophenyl. Das Grundgerüst kann an einer, mehreren oder allen substituierbaren Positionen substituiert sein, wobei geeignete Substituenten dieselben sind, die bereits unter der Definition von Aryl genannt wurden. Vorzugsweise sind die Heteroarylreste jedoch unsubstituiert. Insbesondere sind hier zu nennen Pyridin-2-yl, Pyridin-3-yl, Pyridin-4-yl, Thiophen-2-yl, Thiophen-3-yl, Pyrrol-2-yl, Pyrrol-3-yl, Furan-2-yl, Furan-3-yl und Imidazol-2-yl sowie die entsprechenden benzanellierten Reste, insbesondere Benzimidazolyl, Benzofuryl, Dibenzofuryl oder Dibenzothiophenyl.

Unter heterocyclischem Alkyl sind Reste zu verstehen, die sich vom vorstehend genannten cyclischen Alkyl dadurch unterscheiden, dass im cyclischen Alkyl-Grundgerüst mindestens ein Kohlenstoffatom durch ein Heteroatom ersetzt ist. Bevorzugte Heteroatome sind N, O und S. Das Grundgerüst kann an einer, mehreren oder allen substituierbaren Positionen substituiert sein, wobei geeignete Substituenten dieselben sind, die bereits unter der Definition von Aryl genannt wurden. Insbesondere sind hier die stickstoffhaltigen Reste Pyrrolidin-2-yl, Pyrrolidin-3-yl, Piperidin-2-yl, Piperidin-3-yl, Piperidin-4-yl zu nennen.

Unter Gruppen mit Donor- oder Akzeptorwirkung sind im Sinne der vorliegenden Anmeldung die folgenden Gruppen zu verstehen:
Unter Gruppen mit Donorwirkung sind Gruppen zu verstehen, die einen +I- und/oder +M-Effekt aufweisen, und unter Gruppen mit Akzeptorwirkung sind Gruppen zu verstehend, die einen -I- und/oder -M-Effekt aufweisen. Geeignete Gruppen mit Donor- oder Akzeptorwirkung sind Halogenreste, bevorzugt F, Cl, Br, I besonders bevorzugt F, Cl, halogenierte Alkylreste, z. B. CF₃, Alkoxyreste, Aryloxyreste, Carbonylreste, Esterreste, sowohl Oxycarbonyl als auch Carbonyloxy, z. B. Arylcarbonyloxy, Aminreste, Amidreste, -NR(=OCR), CH₂F-Gruppen, CF₃-Gruppen, CN-Gruppen, Thio-Gruppen, Thioalkoxy-Gruppen, Thioaryloxy-Gruppen, Sulfonsäure-Gruppen, Thiocarbonyl, Carbonylthio, Sulfonsäureester-Gruppen, Boronsäure-Gruppen, Boronsäureester-Gruppen, Phosphonsäure-Gruppen, Phosphonsäureester-Gruppen, Phosphinreste, Sulfoxidreste, Sulfonylreste, Sulfonamid-Gruppen, Sulfidreste, Nitro-Gruppen, OCN, Boranreste, Silyl-Gruppen, Stannatreste, Imino-Gruppen, Hydrazinreste, Hydrazolreste, Oximreste, Nitroso-Gruppen, Diazo-Gruppen, SiR₃-Gruppen, Phosphinoxid-Gruppen, Hydroxy-Gruppen, Vinyl-Gruppen, Heteroaryl-Gruppen oder SCN-Gruppen. Bevorzugte Gruppen mit Donor- oder Akzeptorwirkung sind Alkoxy, Aryloxy, Arylcarbonyloxy ((-C=O(OR)), Carbonylthio (-C=O(SR), Heteroaryl, Hydroxy, Amino, Halogen, Carbonyl (-C=O(R)), Oxycarbonyl (-OC=O(R)), Thiocarbonyl (-SC=O(R)), Amido (-C=O(NR)), -NRC=O(R), Sulfonyl, Sulfonamid-Gruppen, Vinyl, Thioalkoxy, Thioaryloxy oder SiR₃, wobei R jeweils unabhägnig Wasserstoff, Alkyl oder Aryl bedeutet. Ganz besonders bevorzugt sind F, Cl, CN, Aryloxy, Alkoxy und halogenierte Alkylreste, z. B. CF₃.

Unter einem Sulfonamidrest ist -SO₂NHR zu verstehen, worin R Wasserstoff, Alkyl oder Aryl bedeutet, bevorzugt Wasserstoff, C₁-C₆-Alkyl, Phenyl oder Benzyl.

Unter Sulfonyl ist -S(O)₂R zu verstehen, worin R Wasserstoff, Alkyl, Aryl oder Amino bedeutet, bevorzugt Wasserstoff, C₁-C₆-Alkyl, Phenyl, Benzyl oder -NR'₂, worin R' jeweils unabhängig voneinander Wasserstoff, Alkyl oder Aryl, bevorzugt Wasserstoff, C₁-C₆-Alkyl oder Benzyl bedeutet.

Unter der Einheit -CₖH₂ₖ- der Alkylenbrücke B sind insbesondere die linearen Alkylenketten -CH₂-, -(CH₂)₂-, -(CH₂)₃-, -(CH₂)₄-, -(CH₂)₅-, -(CH₂)₆-, -(CH₂)₇- und -(CH₂)₈- zu verstehen. Diese können jedoch auch verzweigt sein, so dass beispielsweise auch Ketten -CH(CH₃)-, -C(CH₃)₂-, -CH₂-CH(CH₃)-, -CH(CH₃)-CH(CH₃)-, -C(CH₃)₂-C(CH₃)₂-, -CH(CH₃)-CH₂-CH(CH₃)-, -CH(CH₃)-(CH₂)₂-CH(CH₃)-, -CH(CH₃)-(CH₂)₃-CH(CH₃)-, -CH(CH₃)-(CH₂)₄-CH(CH₃)-, -C(CH₃)₂-CH₂-C(CH₃)₂- oder -C(CH₃)₂-(CH₂)₂-C(CH₃)₂- in Frage kommen. Des Weiteren können in der Einheit -CₖH₂ₖ- der Alkylenbrücke B ein oder mehrere nicht benachbarte CH₂-Gruppen durch Sauerstoff oder NR ersetzt sein. Beispiele hierfür sind insbesondere -O-C₂H₄-O-, -O-(C₂H₄-O-)₂, -NR-C₂H₄-NR- oder -NR-(C₂H₄-NR-)₂, wobei R insbesondere für Alkyl wie Methyl, Ethyl, Propyl, iso-Propyl, Butyl, sec-Butyl oder tert-Butyl oder Aryl wie Phenyl steht.

R¹ in den Verbindungen der Formel 1 ist jeweils unabhängig voneinander ausgewählt aus der Gruppe bestehend aus gegebenenfalls substituiertem Aryl, gegebenenfalls substituiertem Heteroaryl oder gegebenenfalls substituiertem Alkyl, wobei geeignete Aryl-, Heteroaryl- und Alkyl-Gruppen sowie geeignete Substituenten bereits vorstehend genannt sind. Bevorzugt ist R¹ Alkyl, insbesondere Methyl, Ethyl oder Propyl, besonders bevorzugt Methyl, oder unsubstituiertes oder substituiertes Aryl, bevorzugt unsubstituiertes oder substituiertes Phenyl, wobei es sich bei den Substituenten am Phenyl um Alkyl, das bevorzugt mit Halogen substituiert ist, Alkoxy, CN oder einen Aminosubstituenten, z. B. CF₃, OCH₃, CN oder Diarylamino handelt. Bevorzugt ist der Arylrest mit 1 bis 3 Aminosubstituenten, besonders bevorzugt mit Diarylamino-Substituenten, insbesondere mit Diphenylamino-Substituenten substituiert; Heteroaryl, bevorzugt N-Carbazolyl und Derivate davon. Ganz besonders bevorzugt sind die Reste R¹ unabhängig voneinander Phenyl, Methyl, 9-Phenylcarbazolyl oder 4-N,N-Diphenylaminophenyl.

Die Verbindungen der Formel I enthalten 0, 1, 2 oder bis 3 Reste R¹. Das bedeutet, dass m in den Verbindungen der Formel I 1, 2, 3 oder 4 bedeuten kann, bevorzugt ist m 2, 3 oder 4. In dem Fall, wenn m 4 ist, enthält die Verbindung der Formel I keine Reste R¹.

R² und R³ in Formel I bedeuten jeweils unabhängig voneinander gegebenenfalls substituiertes Alkyl, gegebenenfalls substituiertes Aryl oder gegebenenfalls substituiertes Heteroaryl, wobei geeignete Alkyl-, Aryl- und Heteroaryl-Gruppen sowie geeignete Substituenten vorstehend genannt sind oder Substituenten mit Donor- oder Akzeptorwirkung. Geeignete Substituenten mit Donor- oder Akzeptorwirkung sind vorstehend genannt. Bevorzugte Substituenten mit Donor- oder Akzeptorwirkung sind: Halogenierte Alkylreste, z. B. CF₃, Alkoxy, Aryloxy, Arylcarbonyloxy (-C=O(OR)), -C=O(SR), Heteroaryl, Hydroxy, Amino, Halogen, -C=O(R), -OC=O(R), -SC=O(R), Amido (-C=O(NR)), -NRC=O(R), Sulfonyl, Sulfonamid, Vinyl, CN, Nitro, Thioalkoxy, Thioaryloxy oder SiR₃, wobei R jeweils unabhängig voneinander Wasserstoff, Alkyl oder Aryl bedeutet.

o und p sind in Formel I unabhängig voneinander 0, 1, 2, 3 oder 4, bevorzugt 0, 1 oder 2, besonders bevorzugt 0. In dem Fall, wenn o bzw. p 0 sind, liegen in den Verbindungen der Formel I keine Reste R² bzw. R³ vor, d. h. alle substituierbaren Positionen des Phenothiazin-S-Oxid- bzw. Phenothiazin-S,S-Dioxid-Restes sind mit Wasserstoffatomen substituiert.

Bei der verbrückenden Gruppe L handelt es sich um eine Gruppe ausgewählt aus der Gruppe bestehend aus 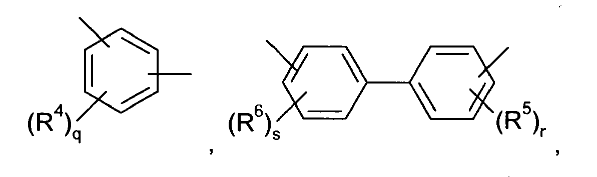 -CH₂-(B)ⱼ- und gegebenenfalls substituiertem Heteroarylen,
wobei die Reste R⁴, R⁵ und R⁶ jeweils unabhängig voneinander gegebenenfalls substituiertes Alkyl, gegebenenfalls substituiertes Aryl, gegebenenfalls substituiertes Heteroaryl oder Substituenten mit Donor- oder Akzeptorwirkung bedeuten. Geeignete Alkyl-, Aryl-, Heteroaryl-Gruppen und Gruppen mit Donor- oder Akzeptorwirkung sind bereits vorstehend genannt.

q, r, s können unabhängig voneinander 0, 1, 2, 3 oder 4 bedeuten, bevorzugt 0, 1 oder 2, besonders bevorzugt 0. In dem Fall, in dem q, r bzw. s 0 bedeuten, enthalten die verbrückenden Gruppen L keine Substituenten R⁴, R⁵ bzw. R⁶, d. h., dass alle substituierbaren Positionen der verbrückenden Gruppen L Wasserstoffatome tragen.

In dem Fall, dass die verbrückende Gruppe L eine Gruppe -CH₂-(B)ⱼ- bedeutet, stellt B eine Alkylengruppe -CₖH₂ₖ-CH₂- dar, worin eine oder mehrere nicht benachbarte CH₂-Gruppen der Einheit -CₖH₂ₖ- durch Sauerstoff oder NR⁷ ersetzt sein können.

R⁷ ist dabei ein Aryl oder Alkyl, wobei geeignete Alkyl-Gruppen bereits vorstehend genannt sind. Besonders bevorzugte Alkyl-Gruppen sind Methyl und Ethyl. Aryl ist bevorzugt Phenyl.

k in der Alkylen-Gruppe B kann 1, 2, 3, 4, 5, 6, 7 oder 8 bedeuten. j ist 0 oder 1.

In einer bevorzugten Ausführungsform ist die Gruppe B eine Alkylengruppe, worin keine der Einheiten -CₖH₂ₖ- durch Sauerstoff oder NR⁷ ersetzt ist. Bei den Alkylen-Gruppen handelt es sich somit bevorzugt um Alkylen-Gruppen der allgemeinen Formel -(CH₂)₁₋₉-. Somit kann die verbrückende Gruppe L eine Alkylen-Gruppe darstellen, die aus 1 bis 10 CH₂-Gruppen aufgebaut ist.

Als Gruppe L bevorzugt eingesetzte gegebenenfalls substituierte Heteroarylen-Gruppen weisen eine der folgenden Formeln auf: 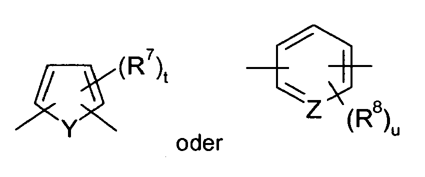 wobei
- R⁷ und R⁸: jeweils unabhängig voneinander gegebenenfalls substituiertes Alkyl, gege- benenfalls substituiertes Heteroaryl oder Substituenten mit Donor- oder Ak- zeptorwirkung bedeuten, wobei geeignete Alkyl-, Aryl-, Heteroaryl-Gruppen und Gruppen mit Donor- oder Akzeptorwirkung bereits vorstehend genannt sind; und
- Y: NR', PR', S, O, wobei R' Alkyl oder Aryl bedeutet und geeignete Alkyl- und Arylgruppen vorstehend genannt sind;
- Z: N
- t: 0, 1 oder 2 bedeutet; und
- u: 0, 1, 2 oder 3 bedeutet,
wobei in dem Fall, wenn t oder u 0 bedeuten, alle substituierbaren Positionen Wasserstoffatome tragen.

In einer bevorzugten Ausführungsform ist die Gruppe L eine verbrückende Gruppe ausgewählt aus der Gruppe bestehend aus 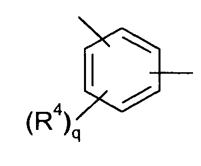 oder-CH₂-(B)ⱼ-,

Besonders bevorzugt ist die verbrückende Gruppe L 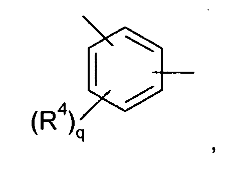 ganz besonders bevorzugt

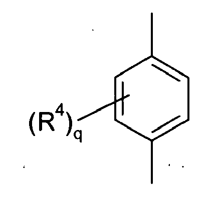

Die Reste R⁴, R⁵ und R⁶ sowie die Indizes q, j, weisen die vorstehend genannten Bedeutungen auf. In einer besonders bevorzugten Ausführungsform sind q, r und s 0, d. h. die substituierbaren Positionen der vorstehend genannten verbrückenden Gruppen L tragen Wasserstoffatome.

Bevorzugt betrifft die vorliegende Erfindung Verbindungen der Formel I worin bedeuten:
- X: SO₂;
- m: 2, 3 oder 4;
- o, p: 0, 1 oder 2, bevorzugt 0;
- L: 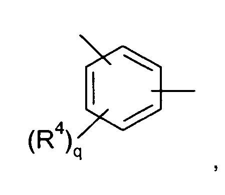 bevorzugt 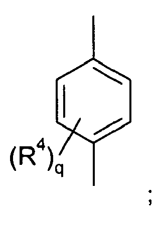 und
- q: 0, 1 oder 2, bevorzugt 0.

In einer weiteren bevorzugten Ausführungsform betrifft die vorliegende Erfindung Verbindungen der Formel I, worin mindestens zwei der an das Si gebundenen Reste oder Gruppen L oder R¹ aromatische Reste oder Gruppen sind.

In einer bevorzugten Ausführungsform betrifft die vorliegende Erfindung Verbindungen der Formel (I), worin bedeuten:
- R², R³: Wasserstoff;
- o, p: 0;
- n: 1 oder 2;
- L: 1,4-Phehylen oder 1,2-Ethylen;
- m: 1, 2, 3 oder 4; und
- R¹: gleich oder verschieden CH₃, Ph, 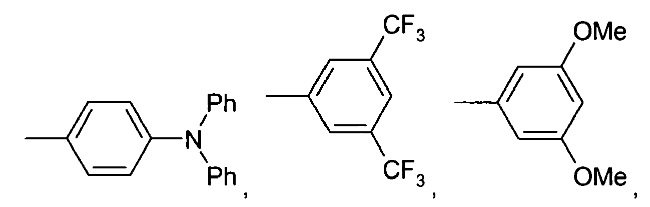 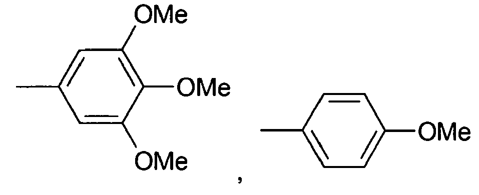
oder

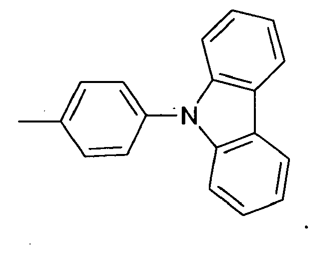

Beispiele für besonders bevorzugte Verbindungen der Formel I sind nachstehend genannt:

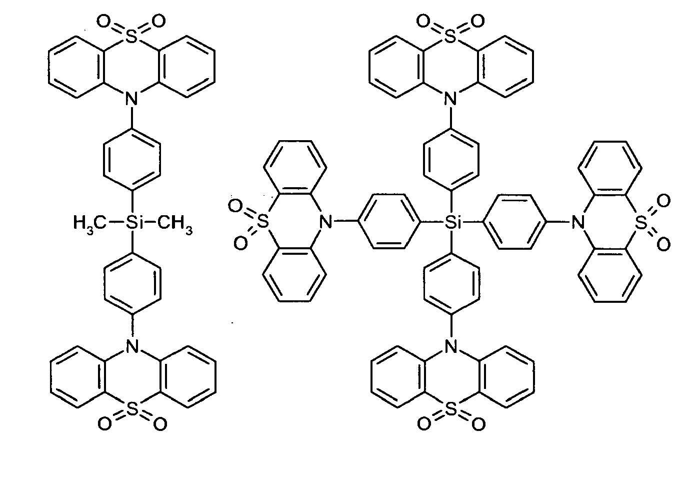

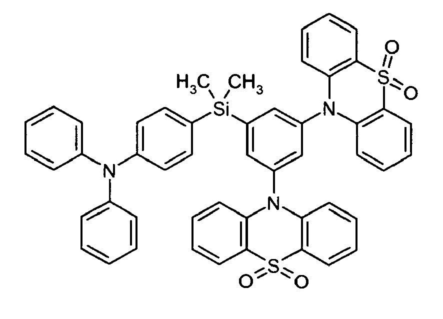

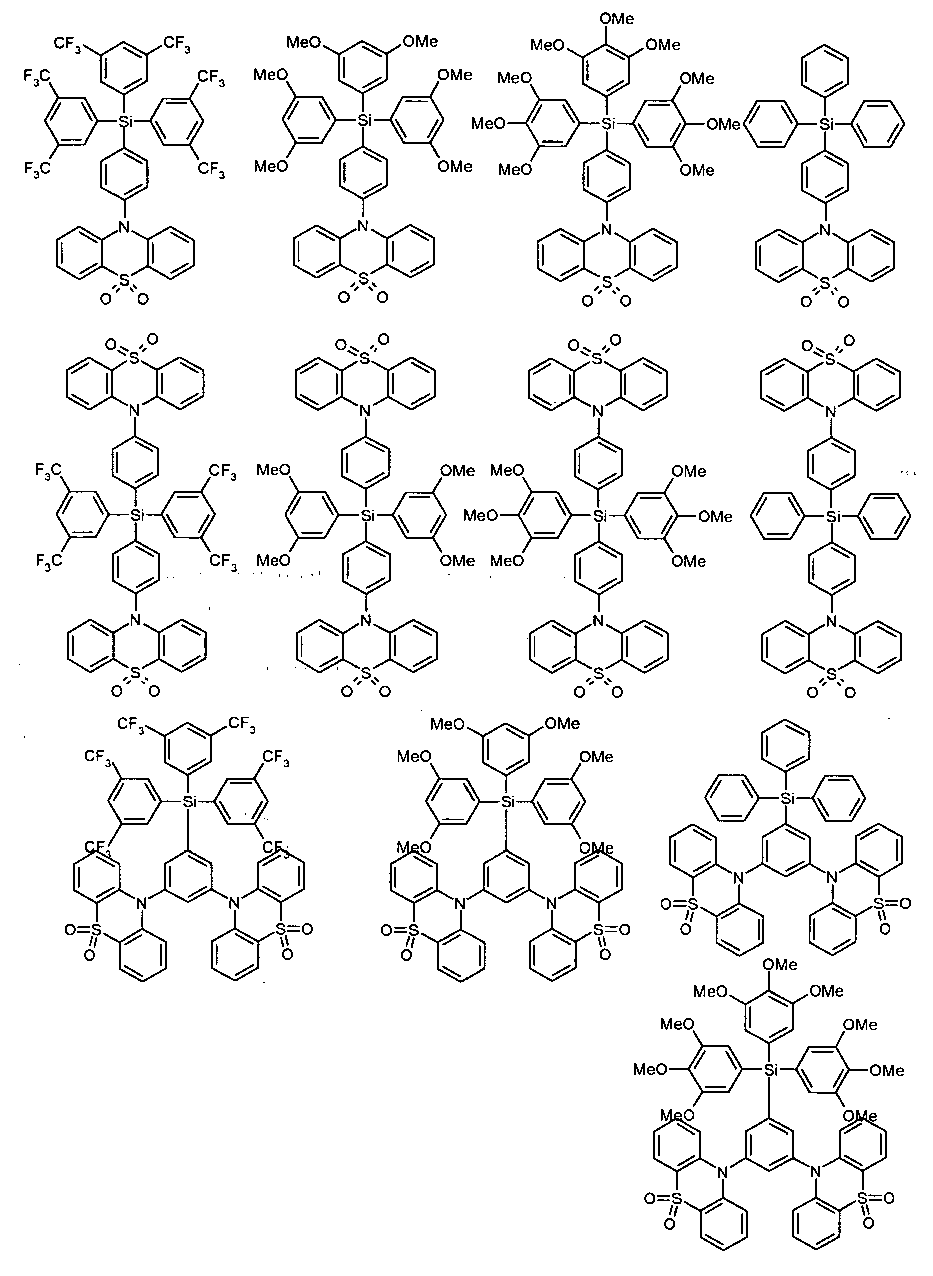

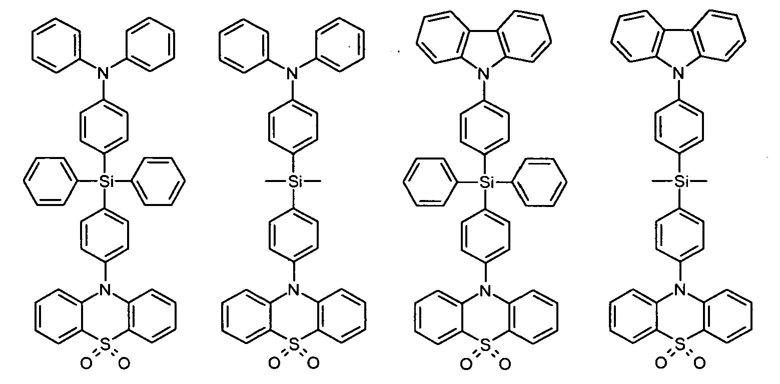

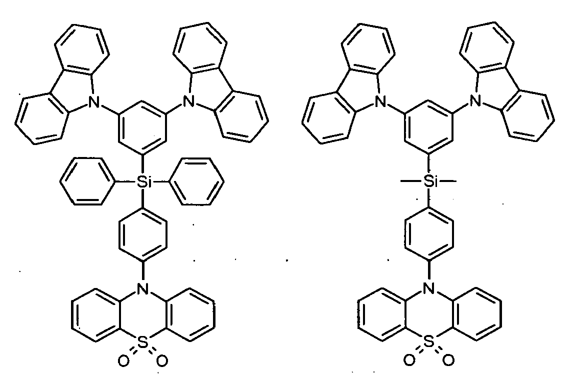

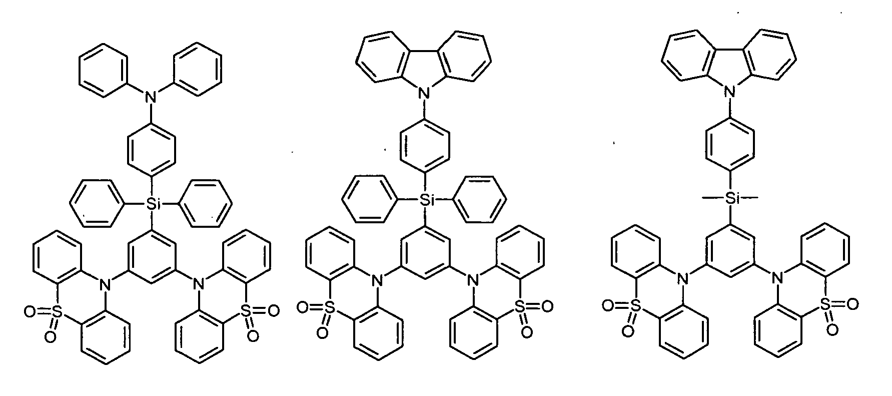

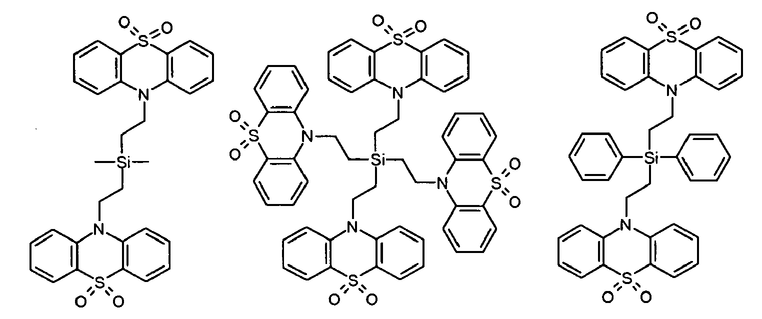

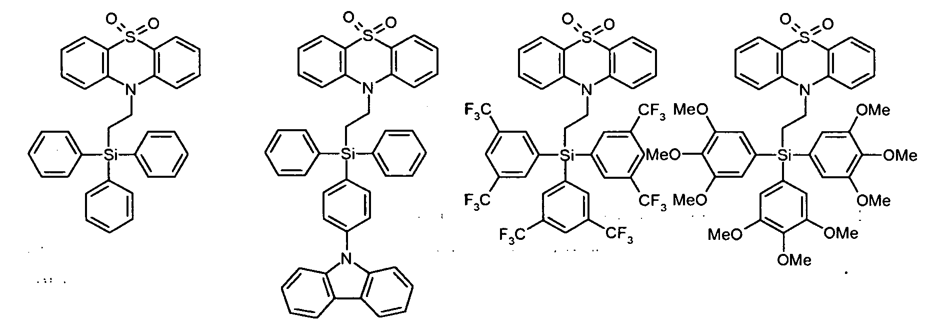

Neben den genannten Verbindungen sind weitere Variationen der Gruppe L und der Indizes n und m möglich. Beispielsweise können die Verbindungen der Formel (I) des Weiteren drei Phenothiazin-S,S-dioxid-Gruppen und einen Rest R¹ aufweisen.

Die Herstellung der erfindungsgemäßen Verbindungen der Formel I kann nach allen geeigneten, dem Fachmann bekannten, Verfahren erfolgen. Bevorzugt erfolgt die Herstellung der Verbindungen der Formel I nach einem Verfahren umfassend die Schritte:
(i) Herstellung eines Phenothiazin-Derivats (II) 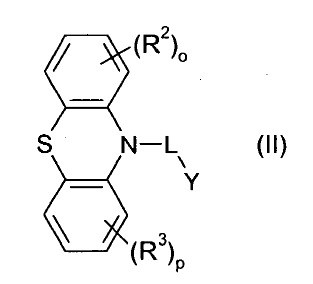 worin bedeuten
   - R², R³: jeweils unabhängig voneinander gegebenenfalls substituiertes Alkyl, gege- benenfalls substituiertes Aryl oder gegebenenfalls substituiertes Heteroaryl oder ein Rest mit Donor- oder Akzeptorwirkung, wie Alkoxy, Aryloxy, Aryl- carbonyloxy (-C=O(OR)), -C=O(SR), Heteroaryl, Hydroxy, Amino, Halo- gen, -C=O(R), -OC=O(R), -SC=O(R), Amido (-C=O(NR)), -NRC=O(R), Sul- fonyl, Sulfonamid, Vinyl, CN, Nitro, Thioalkoxy, Thioaryloxy oder SiR₃, wo- bei R jeweils unabhängig voneinander Wasserstoff, Alkyl, Aryl oder halo- geniertes Alkyl;
   - o, p: unabhängig voneinander 0, 1, 2, 3 oder 4, bevorzugt 0, 1 oder 2, beson- ders bevorzugt 0;
   - L: verbrückende Gruppe ausgewählt aus der Gruppe bestehend aus 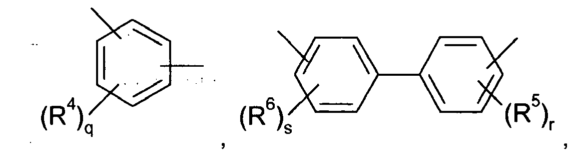 -CH₂-(B)ⱼ- und gegebenenfalls substituiertem Heteroarylen;
   - R⁴, R⁵, R⁶: jeweils unabhängig voneinander gegebenenfalls substituiertes Alkyl, gege- benenfalls substituiertes Aryl, gegebenenfalls substituiertes Heteroaryl oder ein Rest mit Donor- oder Akzeptorwirkung, wie Alkoxy, Aryloxy, Arylcarbo- nyloxy (-C=O(OR)), Carbonylthio (-C=O(SR)), Heteroaryl, Hydroxy, Amino, Halogen, Carbonyl (C=O(R)), -NRC=O(R), Sulfonyl, Sulfonamid-Gruppen, Vinyl, Thioalkoxy, Thioaryloxy oder SiR₃, wobei R jeweils unabhängig von- einander Wasserstoff, Alkyl, halogeniertes Alkyl oder Aryl;
   - q, r, s: unabhängig voneinander 0, 1, 2, 3 oder 4, bevorzugt 0, 1 oder 2, beson- ders bevorzugt 0;
   - B: eine Alkylenbrücke -CₖH₂ₖ-CH₂-, worin eine oder mehrere nicht benachbar- te CH₂-Gruppen der Einheit -CₖH₂ₖ- durch Sauerstoff oder NR⁷ ersetzt sein können;
   - R⁷: Wasserstoff oder Alkyl;
   - k: 1, 2, 3, 4, 5, 6, 7 oder 8; und
   - j: 0 oder 1; und
   - Y: Halogen, bevorzugt ausgewählt aus der Gruppe bestehend aus Cl und Br, besonders bevorzugt Br;
   durch Umsetzung von Phenothiazin oder eines Phenothiazinderivats der Formel (III) 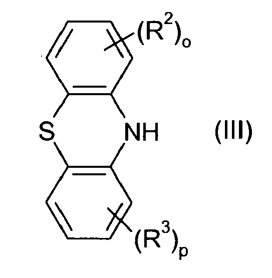 worin R², R³, o und p die vorstehend genannten Bedeutungen aufweisen,
   mit einer bifunktionellen Verbindung der Formel (IV)

   Z-L-Y (IV)

   worin L und Y die vorstehend genannten Bedeutungen aufweisen, und
   - Z: Iod, Fluor, Brom oder Tosyl bedeutet;
(ii) Herstellung von Phenothiazin-Derivaten der Formel (V) 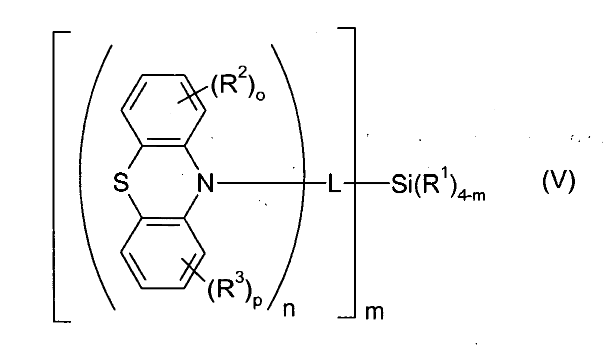 worin die Symbole und Indices die vorstehend genannten Bedeutungen aufweisen und
   - m: 1, 2, 3 oder 4, bevorzugt 2, 3 oder 4, und
   - n: 1 oder 2
   bedeuten;
   durch Umsetzung des Phenothiazinderivats (II) mit einem Halogenalkyl/arylsilan der allgemeinen Formel (VIa) oder mit einem Alkoxysilan der allgemeinen Formel (VIb)

   (R⁸)ₜSi(Hal)₄₋ₜ (VIa)

   (R⁸)ₜ Si(OR⁹)₄₋ₜ (VIb)

   worin bedeuten
   - R⁸: gegebenenfalls substituiertes Aryl, gegebenenfalls substituiertes Heteroaryl oder gegebenenfalls substituiertes Alkyl,
   - Hal: Halogen, bevorzugt Cl; und
   - t-: 1, 2 oder 3; und
   - R⁹: Alkyl, bevorzugt Ethyl oder Methyl
(iii) Herstellung der Phenothiazin-S-oxid- oder -S,S-Dioxid-Derivate der Formel (I)
   durch Umsetzung der Phenothiazin-Derivate der Formel (V) mit einem Oxidationsmittel.

Geeignete bevorzugte Reste und Gruppen der in dem erfindungsgemäßen Verfahren eingesetzten Verbindungen entsprechen den vorstehend bezüglich der Verbindungen der Formel I genannten bevorzugten Resten und Gruppen.

### Schritt (i)

Die zur Herstellung der Phenothiazin-Derivate der Formel (II) eingesetzten Phenothiazine bzw. Phenothiazin-Derivate der Formel (III) sind kommerziell erhältlich bzw. können nach dem Fachmann bekannten Verfahren hergestellt werden. Die bifunktionellen Verbindungen der Formel (IV), die mit den Phenothiazinen bzw. Phenothiazin-Derivaten der Formel (III) umgesetzt werden, sind ebenfalls kommerziell erhältlich bzw. können nach dem Fachmann bekannten Verfahren hergestellt werden.

Die in Schritt (i) des erfindungsgemäßen Verfahrens durchgeführte Substitution des N-Atoms des Phenothiazins bzw. Phenothiazin-Derivats der Formel (III) mit der bifunktionellen Verbindung der Formel (IV) (N-Alkylierung bzw. N-Arylierung) erfolgt bevorzugt in Anwesenheit von Basen, wobei diese dem Fachmann bekannt sind. Vorzugsweise handelt es sich dabei um Alkali- oder Erdalkalihydroxide, wie NaOH, KOH, Ca(OH)₂, Alkalihydride, wie NaH, KH, Alkaliamide, wie NaNH₂, Alkali- oder Erdalkalimetallcarbonate, wie K₂CO₃, oder Alkalimetallalkoxide, wie NaOMe, NaOEt. Des Weiteren sind Gemische der vorstehend genannten Basen geeignet. Besonders bevorzugt sind Na-OH, KOH oder NaH. Besonders bevorzugte Basen sind NaH und K₂CO₃.

Die N-Alkylierung (etwa in M. Tosa et al., Heterocycl. Communications, Vol. 7, No. 3, 2001, S. 277 - 282 beschrieben) bzw. N-Arylierung (etwa in H. Gilman und D. A. Shirley, J. Am. Chem. Soc. 66 (1944) 888; D. Li et al., Dyes and Pigments 49 (2001) 181 - 186 beschrieben) wird bevorzugt in einem Lösungsmittel durchgeführt. Geeignete Lösungsmittel sind z.B. polare aprotische Lösungsmittel, wie Dimethylsulfoxid, Dimethylformamid oder Alkohole. Es ist ebenfalls möglich, einen Überschuss des eingesetzten Alkyl- oder Arylhalogenids als Lösungsmittel einzusetzen, wobei der Einsatz eines Überschusses an Alkyl- oder Aryliodiden bevorzugt ist. Die Umsetzung kann des Weiteren in einem unpolaren aprotischen. Lösungsmittel, z.B. Toluol, durchgeführt werden, wenn ein Phasentransferkatalysator, z.B. tetra-n-Butylammoniumhydrogensulfat, anwesend ist (wie z. B. in I. Gozlan et al., J. Heterocycl. Chem. 21 (1984) 613 - 614 offenbart).

Die N-Arylierung kann aber auch durch Kupfer-katalysierte Kupplung der Verbindung der Formel (III) mit einem Arylhalogenid, bevorzugt einem Aryliodid erfolgen (Ullmann-Reaktion). Ein geeignetes Verfahren zur N-Arylierung von Phenothiazin in Anwesenheit von Kupferbronze ist zum Beispiel in H. Gilman et al., J. Am. Chem. Soc. 66 (1944) 888 - 893 offenbart.

Das molare Verhältnis der Verbindung der Formel (III) zu der bifunktionellen Verbindung der Formel (IV) beträgt im Allgemeinen 1 :: 1 bis 1 : 2, bevorzugt 1 : 1 bis 1 : 1,5.

Die N-Alkylierung bzw. N-Arylierung wird üblicherweise bei Normaldruck und in einem Temperaturbereich von 0 bis 220 °C bzw. bis zum Siedepunkt des verwendeten Lösungsmittels durchgeführt. Die Reaktionsdauer beläuft sich in der Regel auf 0,5 bis 48 Stunden.

Die geeigneten Bedingungen für die N-Alkylierung bzw. N-Arylierung der Verbindung der Formel (III) lassen sich in jedem Fall vom Fachmann ohne Probleme in Vorversuchen ermitteln. Beispielsweise lässt sich der Fortschritt der N-Alkylierung bzw. N-Arylierung mit analytischen Methoden, etwa IR-spetroskopisch, verfolgen.

Das erhaltene Rohprodukt wird im Allgemeinen gemäß dem Fachmann bekannten Verfahren aufgearbeitet.

### Schritt (ii)

In Schritt (ii) erfolgt die Herstellung von Phenothiazin-Derivaten der Formel (V) durch Umsetzung der in Schritt (i) hergestellten Phenothiazin-Derivate der Formel (II) mit einem Halogenalkyl/arylsilan der Formel (IVa) oder einem Alkoxysilan der Formel (Vlb). Die Halogenalkyl/arylsilane der Formel (VIa) und die Alkoxysilane der Formel (VIb) sind im Allgemeinen kommerziell erhältlich oder können nach dem Fachmann bekannten Verfahren hergestellt werden. Als Halogenalkyl/arylsilane sind Halogenalkylsilane, Halogenarylsilane oder gemischte Halogenalkyl/Halogenarylsilane geeignet. Als geeignetes Halogenalkyl/arylsilan kann zum Beispiel Dichlordimethylsilan, Dichlordiphenylsilan, Dichlormethylphenylsilan oder Tetrachlorsilan eingesetzt werden.

Die Umsetzung des Phenothiazins bzw. Phenothiazin-Derivats der Formel (II) mit dem Halogenalky/arylsilan der Formel (VIa) oder dem Alkoxysilan (VIb) erfolgt im Allgemeinen in Anwesenheit eines Metalls bzw. eines Metallsalzes wie Magnesium oder BuLi (n, sec, tert.). Geeignete Reaktionsbedingungen für die Umsetzung in Schritt (ii) des erfindungsgemäßen Verfahrens sind dem Fachmann bekannt bzw. für den Fachmann leicht zu ermitteln.

Üblicherweise wird das Verfahren in Schritt (ii) bei Normaldruck durchgeführt. Die Temperatur beträgt im Allgemeinen -78°C bis +100°C. Die Reaktionsdauer beträgt im Allgemeinen 1 Stunde bis 24 Stunden.

Das erhaltene Rohprodukt wird im Allgemeinen gemäß dem Fachmann bekannten Verfahren aufgearbeitet.

### Schritt (iii)

In Schritt (iii) des erfindungsgemäßen Verfahrens erfolgt die Herstellung der erfindungsgemäßen Phenothiazin-S-oxid- oder -S,S-dioxid-Derivate der Formel (I) ausgehend von den gemäß Schritt (ii) hergestellten Phenothiazin-Derivaten der Formel (V). Die erfindungsgemäßen Phenothiazin-S-oxid- oder -S,S-dioxid-Derivate der Formel (I) werden durch Umsetzung der Phenothiazin-Derivate der Formel (V) mit einem Oxidationsmittel erhalten. Geeignete Oxidationsmittel sind davon abhängig, ob Phenothiazin-S-oxid- oder Phenothiazin-S,S-dioxid-Derivate hergestellt werden. Zur Herstellung der jeweiligen Derivate geeignete Oxidationsmittel sind dem Fachmann bekannt. Beispiele für geeignete Oxidationsmittel sind nachstehend genannt.

Geeignete Verfahren zur Oxidation der Phenothiazine zu den erfindungsgemäß verwendeten Phenothiazin-S-oxiden und Phenothiazin-S,S-dioxiden sind dem Fachmann bekannt und zum Beispiel in M. Tosa et al. Heterocyclic Communications, Vol. 7, No. 3, 2001, S. 277 bis 282 angegeben.

Die Oxidation zu Phenothiazin-S-oxid-Derivaten erfolgt zum Beispiel mittels H₂O₂ in Ethanol, Ethanol-Aceton-Mischungen oder Oxalsäure, mittels Ammoniumpersulfat, Salpetersäure, salpetriger Säure, anorganischen Stickstoffoxiden gegebenenfalls zusammen mit (Luft-)Sauerstoff, NO⁺BF₄⁻/O₂, CrO₃ in Pyridin, Ozon, Tetramethyloxiran, Perfluoroalkyloxaziridinen oder mittels elektrochemischen Methoden. Des Weiteren kann die Oxidation der entsprechend funktionalisierten Phenothiazine der Formel V zu den entsprechenden Phenoxazin-S-oxid-Derivaten der Formel I mittels m-Chlorperbenzoesäure in CH₂Cl₂ bei Temperaturen von 0 bis 5 °C oder mittels einer Mischung aus rauchender Salpetersäure und Eisessig in CCl₄ erfolgen (siehe etwa M. Tosa et al. Heterocyclic Communications, Vol. 7, No. 3, 2001, S. 277 bis 282).

Die Oxidation zu Phenothiazin-S,S-dioxid-Derivaten erfolgt zum Beispiel mittels Persäuren, wie Peressigsäure, die beispielsweise aus H₂O₂ und AcOH zugänglich ist, oder m-Chlorperbenzoesäure, Natriumperborat, NaOCl oder Schwermetallsystemen wie KMnO₄/H₂O, Et₃PhN⁺MnO₄⁻ in organischen Medien, OsO₄/N-Methylmorpholin-N-oxid. So kann die Oxidation der entsprechend funktionalisierten Phenothiazine der Formel V zu den entsprechenden Phenothiazin-S,S-dioxid-Derivaten der Formel I mittels einer wässrigen Lösung von KMnO₄ und C₁₆H₃₅N(CH₃)₃⁺Cl⁻ in CHCl₃ bei Raumtemperatur oder mittels m-Chlorperbenzoesäure in CH₂Cl₂ bei Raumtemperatur erfolgen (siehe etwal M. Tosa et al. Heterocyclic Communications, Vol. 7, No. 3, 2001, S. 277 - 282).

Zur Herstellung der Phenothiazin-S,S-dioxid-Derivate werden das Phenothiazin-Derivat der Formel V und das Oxidationsmittel, bevorzugt m-Chlorperbenzoesäure, in einem molaren Verhältnis von im Allgemeinen 1 : 1,8 bis 1 : 4, bevorzugt 1 : 1,9 bis 1 : 3,5, besonders bevorzugt 1 : 1,9 bis 1 : 3 eingesetzt.

Zur Herstellung von Phenothiazin-S-oxid-Derivaten werden das Phenothiazin-Derivat der Formel V und das Oxidationsmittel in einem molaren Verhältnis von im Allgemeinen 1 : 0,8 bis 1 : 1,5, bevorzugt 1 : 1 bis 1 : 1,3 eingesetzt. Oxidationsmittel, mit denen keine Weiteroxidation zu den entsprechenden S,S-Dioxid-Derivaten erfolgt, z.B. H₂O₂, können in einem größeren Überschuss als dem vorstehend angegebenen in Bezug auf das Phenothiazin-Derivat eingesetzt werden.

Die Oxidation erfolgt im Allgemeinen in einem Lösungsmittel, bevorzugt in einem Lösungsmittel ausgewählt aus der Gruppe bestehend aus halogenierten Kohlenwasserstoffen und dipolar aprotischen Lösungsmitteln. Beispiele für erstere bzw. letztere sind Methylenchlorid bzw. Acetonitril und Sulfolan.

Abhängig vom Oxidationsmittel erfolgt die Oxidation zu den Phenothiazin-S-oxid-Derivaten üblicherweise bei Normaldruck in einem Temperaturbereich von -10 °C bis +50 °C und die Oxidation zu den Phenothiazin-S,S-dioxid-Derivaten üblicherweise bei Normaldruck in einem Temperaturbereich von 0 bis + 100°C. Die Reaktionsdauer der Oxidation beträgt im Allgemeinen 0,25 bis 24 Stunden.

Die geeigneten Bedingungen für die Oxidation der jeweiligen Phenothiazin-Derivate zu den entsprechenden Phenothiazin-S-oxid- bzw. Phenothiazin-S,S-dioxid-Derivaten lassen sich aber in jedem Fall vom Fachmann ohne Probleme in Vorversuchen ermitteln. Beispielsweise kann der Fortschritt der Oxidation mit analytischen Methoden, etwa IR-spektroskopisch, verfolgt werden.

In einer bevorzugten Variante werden die Phenothiazin-S-oxid-Derivate der Formel I durch Oxidation der entsprechenden Phenothiazin-Derivate der Formel V mit m-Chlorperbenzoesäure als Oxidationsmitel in CH₂Cl₂ bei 0 bis 20 °C hergestellt.

Die Phenothiazin-S,S-dioxid-Derivate der Formel I werden bevorzugt durch Oxidation der entsprechenden Phenothiazin-Derivate der Formel V mit m-Chlorperbenzoesäure als Oxidationsmittel in CH₂Cl₂ bei 0 bis 40 °C hergestellt.

Die Isolierung und Aufarbeitung der erhaltenen Phenothiazin-S-oxide und Phenothiazin-S,S-dioxide erfolgt nach dem Fachmann bekannten Verfahren.

Die erfindungsgemäßen Verbindungen der Formel (I) können mit Hilfe des erfindungsgemäßen Verfahrens in hoher Reinheit und in guten Ausbeuten erhalten werden. Die hohe Reinheit wird gegebenenfalls dadurch erzielt, dass das in dem erfindungsgemäßen Verfahren erhaltene Produkt zum Beispiel durch Umkristallisation gereinigt wird.

Die Verbindungen der Formel (I) sind hervorragend für den Einsatz als Matrixmaterialien in organischen Leuchtdioden geeignet. Insbesondere sind sie als Matrixmaterialien in der Licht-emittierenden Schicht der OLEDs geeignet, wobei die Licht-emittierende Schicht als Emitter-Verbindungen bevorzugt einen oder mehrere Triplett-Emitter enthält.

Des Weiteren sind die Verbindungen der Formel (I) als Blockermaterialien geeignet, insbesondere als Blockermaterialien in OLEDs, wobei sie bevorzugt als Blockermaterialien für Triplett-Emitter eingesetzt werden. Die Verbindungen der Formel(I) können - in Abhängigkeit von ihrem Substitutionsmuster - als Loch-/Excitonenblocker oder Elektronen-/Excitionenblocker eingesetzt werden, wie vorstehend ausgeführt wurde.

Ein weiterer Gegenstand der vorliegenden Erfindung ist daher ein OLED enthaltend mindestens eine Verbindung der Formel (I). Bevorzugt wird die Verbindung der Formel (I) in einer Ausführungsform als Matrixmaterial eingesetzt, wobei das Matrixmaterial besonders bevorzugt gemeinsam mit einem Triplett-Emitter eingesetzt wird. In einer weiteren Ausführungsform wird die Verbindung der Formel (I) bevorzugt als Blockermaterial, besonders bevorzugt als Blockermaterial für Triplett-Emitter, eingesetzt.

Weiterhin können die Verbindungen der Formel (I) in OLEDs sowohl als Matrixmaterial als auch als Blockermaterial eingesetzt werden. Dabei kann es sich bei dem Matrixmaterial und dem Blockermaterial um die gleichen oder verschiedene Verbindungen der Formel (I) handeln.

Ein weiterer Gegenstand der vorliegenden Erfindung ist eine Licht-emittierende Schicht, enthaltend mindestens eine erfindungsgemäße Verbindung der Formel (I) und mindestens eine Emitter-Verbindung, wobei es sich bei der Emitter-Verbindung bevorzugt um einen Triplett-Emitter handelt.

In einer weiteren Ausführungsform betrifft die vorliegende Erfindung die Verwendung der Verbindungen der Formel (I) als Blockermaterialien in OLEDs. Weiterhin kann ein OLED die Verbindungen der Formel (I) sowohl als Matrixmaterialien als auch als Blockermaterialien enthalten.

Des Weiteren betrifft die vorliegende Erfindung die Verwendung der erfindungsgemäßen Verbindungen der Formel (I) in OLEDs, bevorzugt als Matrixmaterialien, insbesondere als Matrixmaterialien für Emitter-Verbindungen, wobei es sich bei den Emitter-Verbindungen besonders bevorzugt um Triplett-Emitter handelt.

Die Verwendung der erfindungsgemäßen Verbindungen der Formel (I) als Matrixmaterialien in der Licht-emittierenden Schicht eines OLEDs ist ebenfalls ein weiterer Gegenstand der vorliegenden Erfindung.

Die Verwendung der Verbindungen der Formel I als Matrixmaterialien und/oder Blockermaterialien soll hierbei nicht ausschließen, dass diese Verbindungen selbst auch Licht emittieren. Die erfindungsgemäß verwendeten Matrixmaterialien und/oder Blockermaterialien bewirken aber, dass bei Verbindungen, welche als Emitter in OLEDs eingesetzt werden, eine Zunahme der Leuchtdichte und Quantenausbeute gegenüber sonst üblichen Matrixmaterialien und/oder Blockermaterialien erreicht werden kann, wenn sie in das Matrixmaterial eingebettet sind bzw. wenn die OLEDs die Verbindungen der Formel (I) als Blockermaterial enthalten.

Viele der bevorzugt eingesetzten Emitter-Verbindungen basieren auf Metallkomplexen, wobei insbesondere die Komplexe der Metalle Ru, Rh, Ir, Pd und Pt, vor allem die Komplexe des Ir Bedeutung erlangt haben. Die erfindungsgemäß verwendeten Verbindungen der Formel I sind besonders als Matrixmaterialien und/oder Blockermaterialien für Emitter auf Basis solcher Metallkomplexe geeignet. Insbesondere sind sie für die Verwendung als Matrixmaterialien und/oder Blockermaterialien zusammen mit Komplexen des Ru, Rh, Ir, Pd und Pt, besonders bevorzugt für die Verwendung zusammen mit Komplexen des Ir geeignet.

Geeignete Metallkomplexe zur Verwendung zusammen mit den Verbindungen der Formel I als Matrixmaterialien und/oder Blockermaterialien in OLEDs sind z.B. in den Schriften WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1 und WO 2006/056418 beschrieben.

Weitere geeignete Metallkomplexe sind die kommerziell erhältlichen Metallkomplexe Tris(2-phenylpyridin)iridium(III), Iridium(III)tris(2-(4-tolyl)pyridinato-N,C²'), Iridium(III)tris (1-phenylisochinolin), Iridium(III)bis(2-(2'-benzothienyl)pyridinato-N,C³') (acetylacetonat), Iridium(III)bis(2-(4,6-difluorophenyl)pyridinato-N,C²)picolinat, Iridium(III)bis(1-phenylisochinolin)(acetylacetaonat), Iridium(III)bis(di-benzo[f,h]chinoxalin) (acetylacetaonat), Iridium(III)bis(2-methyldi-benzo[f,h]chinoxalin)(acetylacetonat) und Tris(3-methyl-1-phenyl-4-trimethyl-acetyl-5-pyrazolin)terbium(III).

Des Weiteren sind die folgenden kommerziell erhältlichen Materialien geeignet: Tris(dibenzoylacetonato)-mono(phenanthrolin)-europium(III), Tris(dibenzoylmethan)-mono(phenanthrolin)europium(III), Tris(dibenzoylmethan)-mono(5-aminophenanthrolin)europium(III), Tris(di-2-naphthoylmethan)-mono(phenanthrolin)-europium(III), Tris(4-bromobenzoylmethan)-mono(phenanthrolin)-europium(III), Tris(di(biphenylmethan))-mono(phenanthrolin)-europium(III), Tris(dibenzoylmethan)-mono(4,7-diphenylphenanthrolin)-europium(III), Tris(dibenzoylmethan)-mono(4,7-dimethylphenanthrolin)-europium(III), Tris(dibenzoylmethan)-mono(4,7-dimethylphenanthrolin-disulfonsäure)europium(III)-dinatriumsalz, Tris[di(4-(2-(2-ethoxy-ethoxy)ethoxy)benzoylmethan)]mono(phenanthrolin)-europium(III) und Tris[di[4-(2-(2-ethoxyethoxy)ethoxy)benzoylmethan)]mono-(5-aminophenanthrolin)-europium(III).

In einer bevorzugten Ausführungsform der vorliegenden Erfindung werden die erfindungsgemäßen Verbindungen der Formel (I) in der Licht-emittierenden Schicht als Matrixmaterial gemeinsam mit Carbenkomplexen als Triplett-Emitter eingesetzt, d.h. besonders bevorzugte Triplett-Emitter sind Carbenkomplexe. Geeignete Carbenkomplexe sind dem Fachmann bekannt und in einigen der vorstehend genannten Anmeldungen und nachstehend genannt. In einer weiteren bevorzugten Ausführungsform werden die erfindungsgemäßen Verbindungen der Formel (I) als Blockermaterial gemeinsam mit Carbenkomplexen als Triplett-Emitter eingesetzt. Die erfindungsgemäßen Verbindungen können des Weiteren sowohl als Matrixmaterialien als auch als Blockermaterialien gemeinsam mit Carbenkomplexen als Triplett-Emitter eingesetzt werden.

Geeignete Metallkomplexe zur Verwendung zusammen mit den Verbindungen der Formel I als Matrixmaterialien und/oder Blockermaterialien in OLEDs sind somit z.B. auch Carben-Komplexe, wie sie in WO 2005/019373 A2, WO 2006/056418 A2 und WO 2005/113704 und in den älteren nicht vorveröffentlichten Europäischen Anmeldungen EP 06 112 228.9 und EP 06 112 198.4 beschrieben sind. Auf die Offenbarung der genannten WO- und EP-Anmeldungen wird hierbei explizit Bezug genommen und diese Offenbarungen sollen in den Inhalt der vorliegenden Anmeldung als mit einbezogen gelten. Insbesondere enthalten geeignete Metallkomplexe zur Verwendung zusammen mit den Verbindungen der Formel I als Matrixmaterialien und/oder Blockermaterialien in OLEDs Carbenliganden der nachfolgenden, unter anderem in WO 2005/019373 A2 offenbarten Strukturen (die Bezeichnung der im Folgenden verwendeten Variablen wurde aus der Anmeldung WO 2005/019373A2 übernommen; im Hinblick auf die genauere Definition der Variablen wird ausdrücklich auf diese Anmeldung verwiesen): 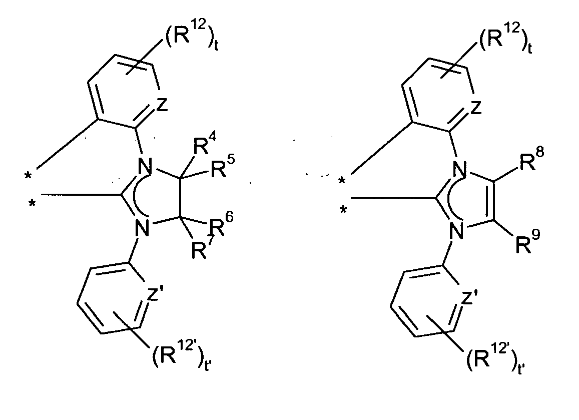 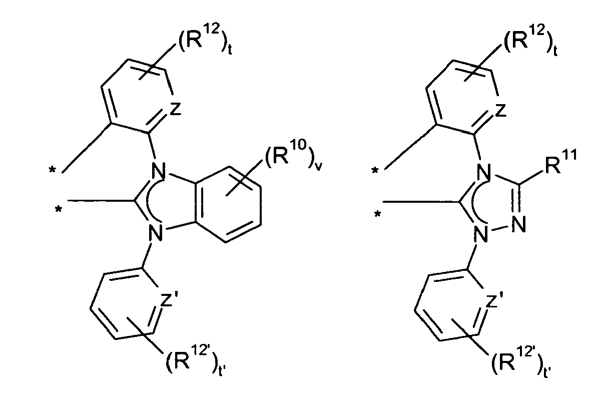 worin bedeuten:
- *: die Anbindungsstellen des Liganden an das Metallzentrum;
- z, z: gleich oder verschieden, CH oder N;
- R¹², R^{12'}: gleich oder verschieden, ein Alkyl-, Aryl-, Heteroaryl- oder Alkenylrest, be- vorzugt ein Alkyl- oder Arylrest oder jeweils 2 Reste R¹² bzw. R^{12'} bilden gemeinsam einen anellierten Ring, der gegebenenfalls mindestens ein He- teroatom, bevorzugt N, enthalten kann, bevorzugt bilden jeweils 2 Reste R¹² bzw. R^{12'} gemeinsam einen anellierten aromatischen C₆-Ring, wobei an die- sen, bevorzugt sechsgliedrigen, aromatischen Ring gegebenenfalls ein oder mehrere weitere aromatische Ringe anelliert sein können, wobei jede denk- bare Anellierung möglich ist, und die anellierten Reste wiederum substituiert sein können; oder R¹² bzw. R^{12'} bedeutet einen Rest mit Donor- oder Ak- zeptorwirkung, bevorzugt ausgewählt aus der Gruppe bestehend aus Halo- genresten, bevorzugt F, Cl, Br, besonders bevorzugt F; Alkoxy-, Aryloxy-, Carbonyl-, Ester-, Aminogruppen, Amidresten, CHF₂, CH₂F, CF₃, CN, Thiogruppen und SCN; t und t' gleich oder verschieden, bevorzugt gleich, 0 bis 3, wobei, wenn t bzw. t' > 1 ist, die Reste R¹² bzw. R^{12'} gleich oder verschieden sein kön- nen, bevorzugt ist t bzw. t' 0 oder 1, der Rest R¹² bzw. R^{12'} befindet sich, wenn t bzw. t' 1 ist, in ortho-, meta- oder para-Position zur Verknüpfungs- stelle mit dem dem Carbenkohlenstoffatom benachbarten Stickstoffatom;
- R⁴, R⁵, R⁶, R⁷, R⁸, R⁹ und R¹¹: Wasserstoff, Alkyl, Aryl, Heteroaryl, Alkenyl oder ein Substituent mit Donor- oder Akzeptorwirkung, bevorzugt ausgewählt aus Halogenresten, bevorzugt F, Cl, Br, besonders bevorzugt F, Alkoxyresten, Aryloxyresten, Carbonylresten, Esterresten, Aminresten, Amidresten, CH₂F-Gruppen, CHF₂-Gruppen, CF₃-Gruppen, CN-Gruppen, Thiogruppen und SCN- Gruppen, bevorzugt Wasserstoff, Alkyl, Heteroaryl oder Aryl,
- R¹⁰: Alkyl, Aryl, Heteroaryl oder Alkenyl, bevorzugt Alkyl, Heteroaryl oder Aryl, oder jeweils 2 Reste R¹⁰ bilden gemeinsam einen anellierten Ring, der ge- gebenenfalls mindestens ein Heteroatom, bevorzugt Stickstoff, enthalten kann, bevorzugt bilden jeweils 2 Reste R¹⁰ gemeinsam einen anellierten a- romatischen C₆-Ring, wobei an diesen, bevorzugt sechsgliedrigen, aromati- schen Ring gegebenenfalls ein oder mehrere weitere aromatische Ringe anelliert sein können, wobei jede denkbare Anellierung möglich ist, und die anellierten Reste wiederum substituiert sein können; oder R¹⁰ bedeutet ei- nen Rest mit Donor- oder Akzeptorwirkung, bevorzugt ausgewählt aus der Gruppe bestehend aus Halogenresten, bevorzugt F, Cl, Br, besonders be- vorzugt F; Alkoxy-, Aryloxy-, Carbonyl-, Ester-, Aminogruppen, Amidresten, CHF₂, CH₂F, CF₃, CN, Thiogruppen und SCN
- v: 0 bis 4, bevorzugt 0, 1 oder 2, ganz besonders bevorzugt 0, wobei, wenn v 0 ist, die vier Kohlenstoffatome des Arylrests in Formel c, die gegebenen- falls mit R¹⁰ substituiert sind, Wasserstoffatome tragen.

Insbesondere enthalten geeignete Metallkomplexe zur Verwendung zusammen mit den Verbindungen der Formel I als Matrixmaterialien und/oder Blockermaterialien in OLEDs Ir-Carbenkomplexe der nachfolgenden, in WO 2005/019373 A2 offenbarten Strukturen: 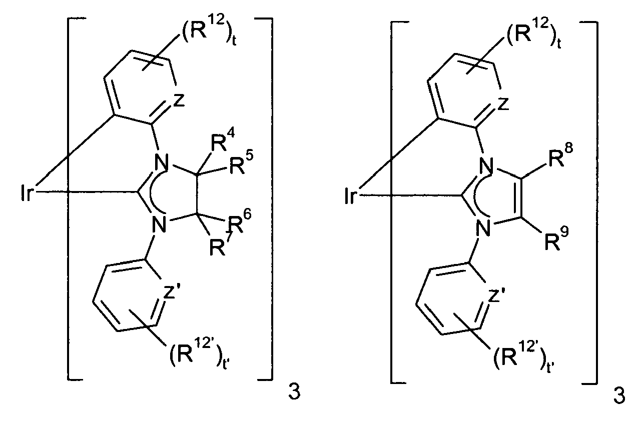 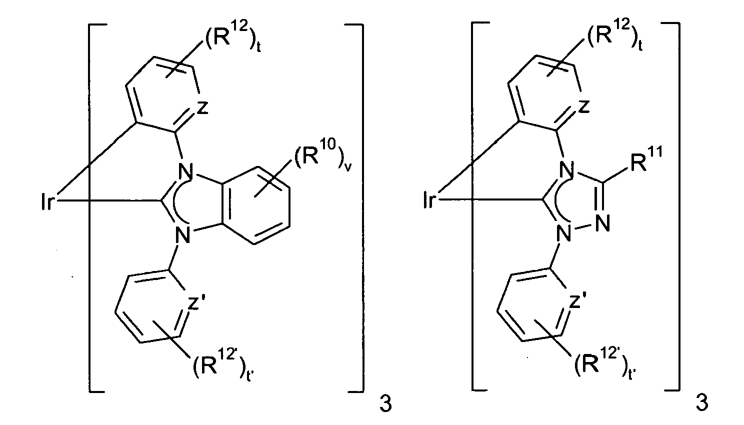 wobei die Variablen die bereits vorstehend genannten Bedeutungen aufweisen.

Weitere geeignete Metallkomplexe zur Verwendung zusammen mit den Verbindungen der Formel I als Matrixmaterialien und/oder Blockermaterialien in OLEDs sind insbesondere auch in WO 2006/056418 A2 offenbarte Strukturen (die Bezeichnung der im Folgenden verwendeten Variablen wurde aus der Anmeldung WO 2006/056418 A2 übernommen; im Hinblick auf die genauere Definition der Variablen wird ausdrücklich auf diese Anmeldung verwiesen): 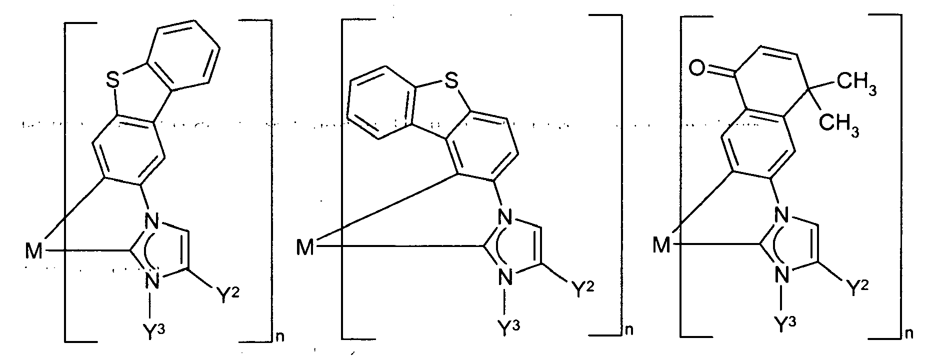 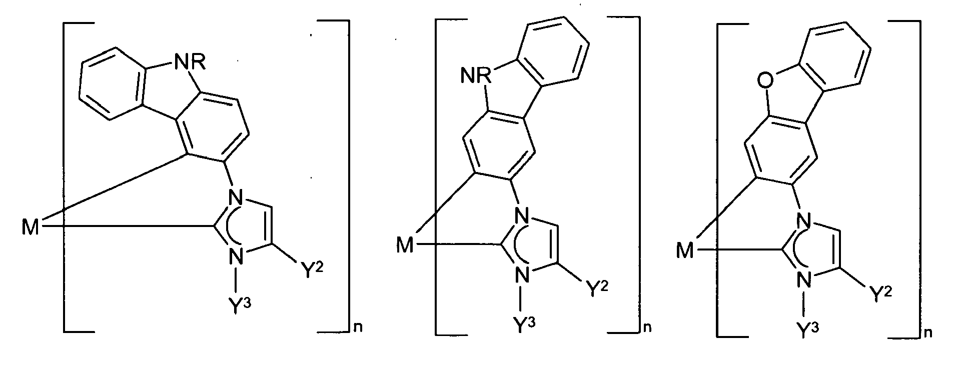 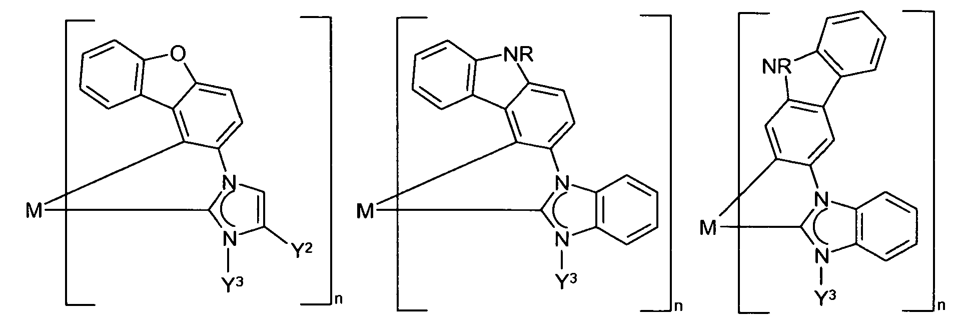 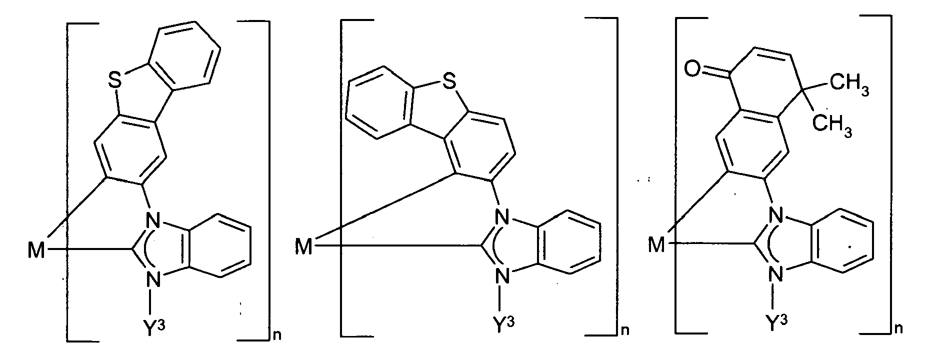 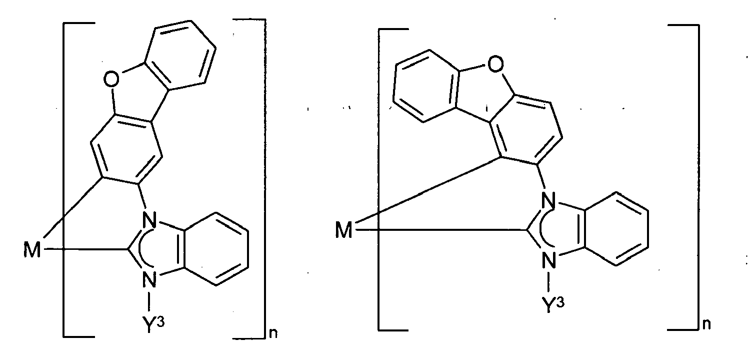 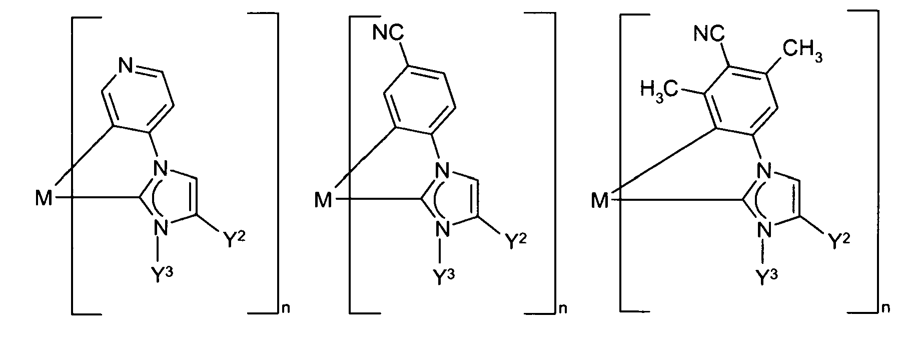 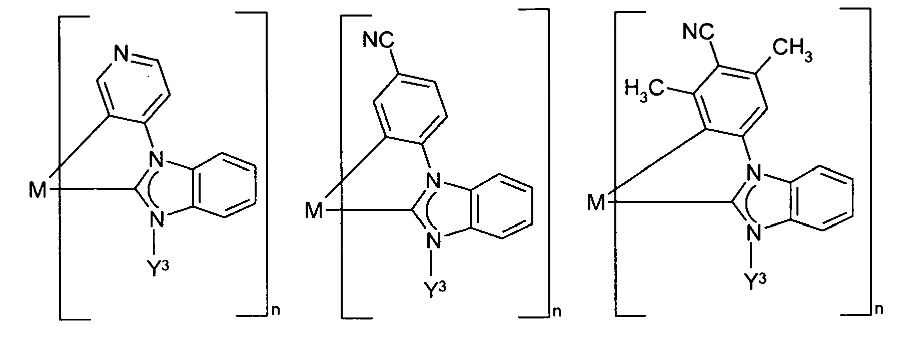 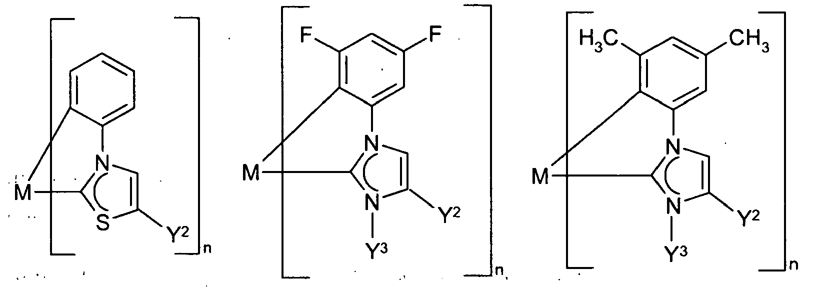 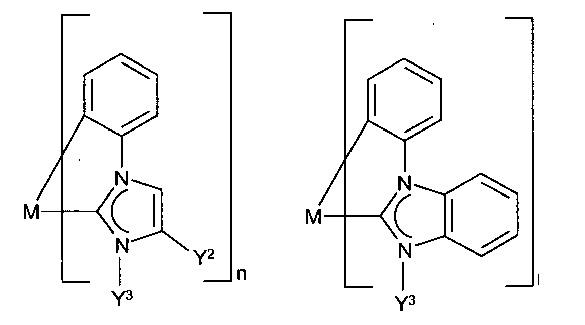 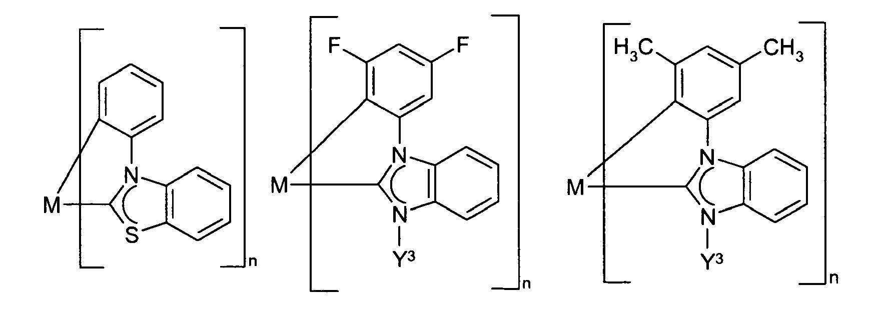 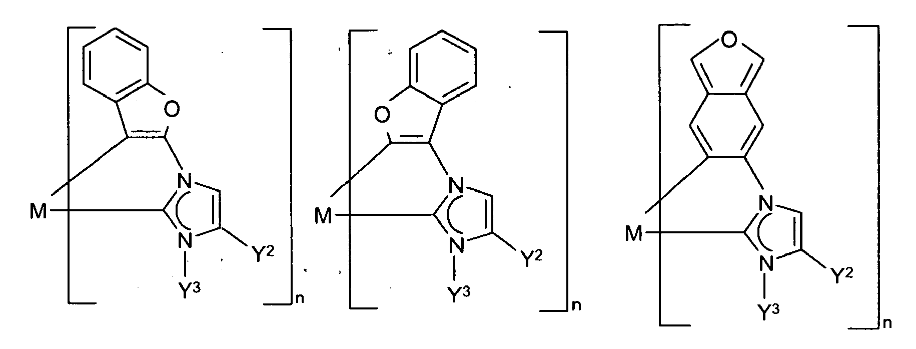 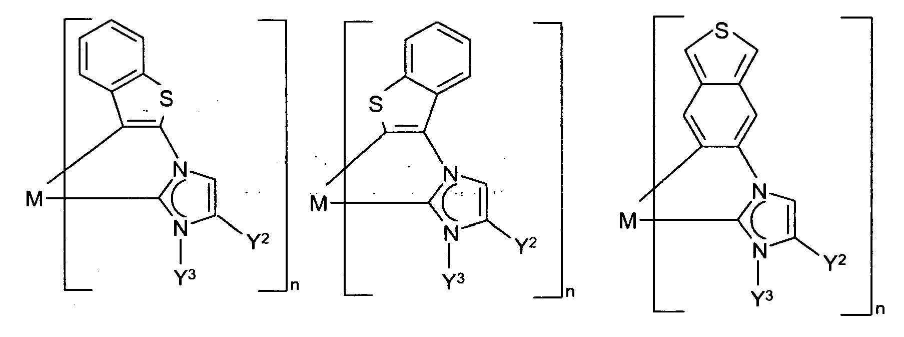 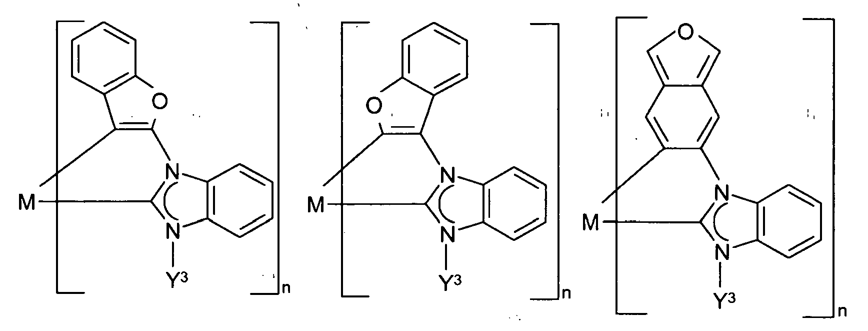 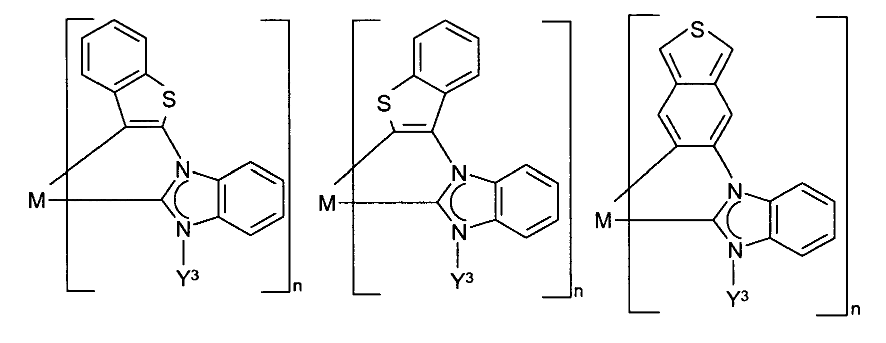 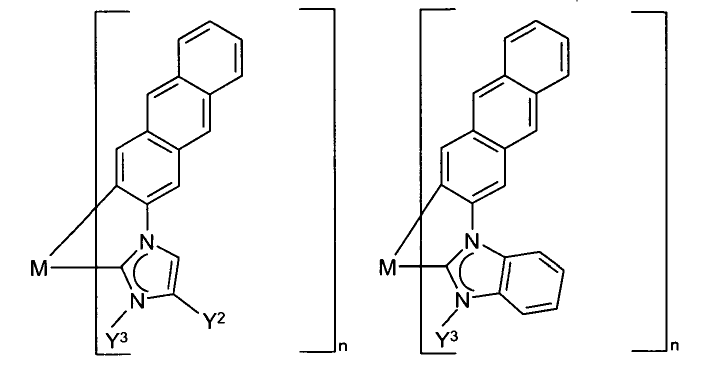 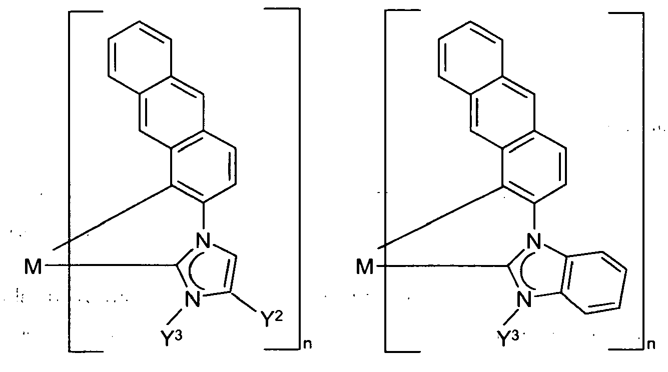 worin M für Ru(III), Rh(III), Ir(III), Pd(II) oder Pt(II) steht, n für Ru(III), Rh(III) und Ir(III) den Wert 3, für Pd(II) und Pt(II) den Wert 2 annimmt und Y² und Y³ Wasserstoff, Methyl, Ethyl, n-Propyl, iso-Propyl oder tert.-Butyl bedeuten. Bevorzugt handelt es sich bei M um Ir(III) mit n gleich 3. Y³ bedeutet vorzugsweise Methyl, Ethyl, n-Propyl, iso-Propyl oder tert.-Butyl.

Weitere geeignete Metallkomplexe zur Verwendung zusammen mit den Verbindungen der Formel I als Matrixmaterialien und/oder Blockermaterialien in OLEDs sind insbesondere auch: 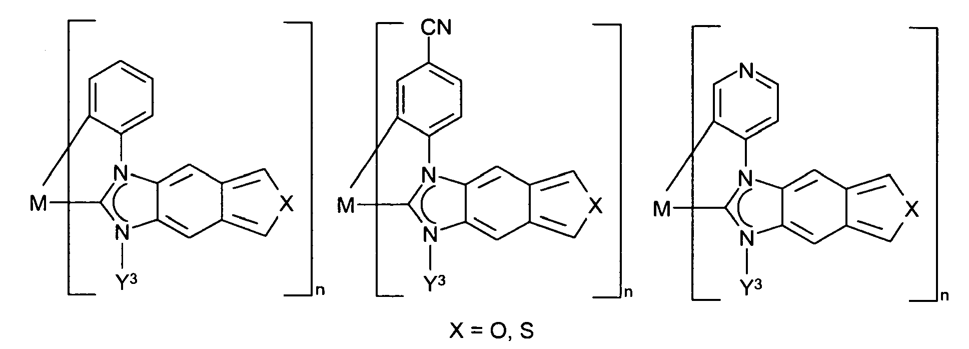 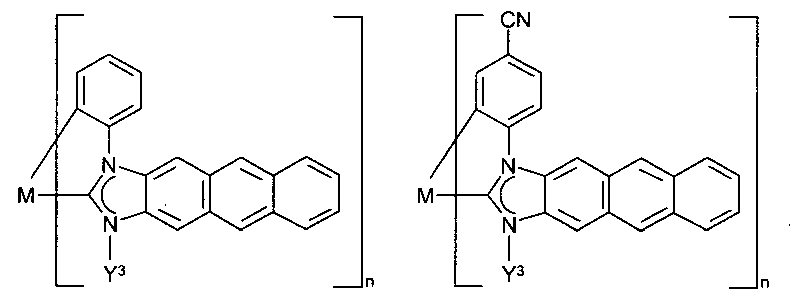 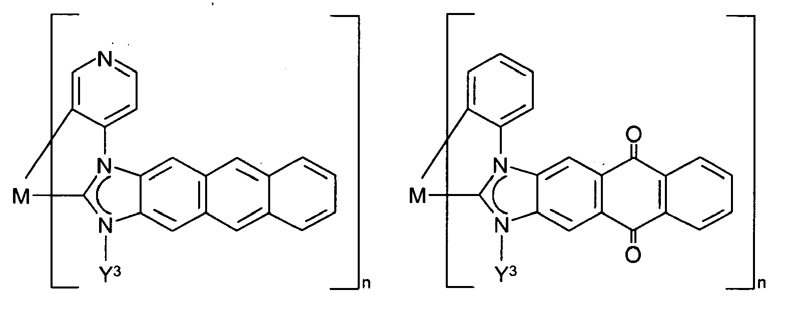 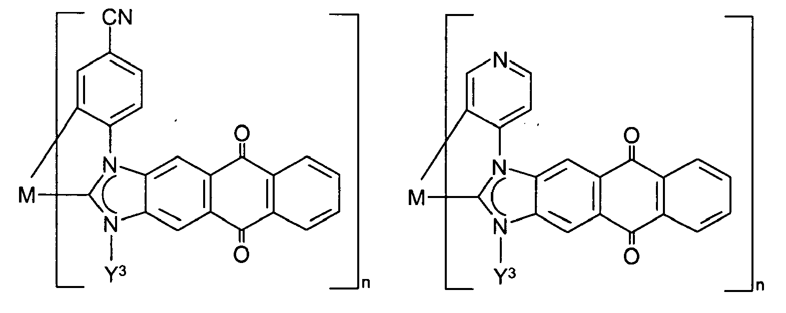 worin M für Ru(III), Rh(III), Ir(III), Pd(II) oder Pt(II) steht, n für Ru(III), Rh(III) und Ir(III) den Wert 3, für Pd(II) und Pt(II) den Wert 2 annimmt und Y³ Wasserstoff, Methyl, Ethyl, n-Propyl, iso-Propyl oder tert.-Butyl bedeuten. Bevorzugt handelt es sich bei M um Ir(III) mit n gleich 3. Y³ bedeutet vorzugsweise Methyl, Ethyl, n-Propyl, iso-Propyl oder tert.-Butyl.

Weitere geeignete Metallkomplexe zur Verwendung zusammen mit den Verbindungen der Formel I als Matrixmaterialien und/oder Blockermaterialien in OLEDs sind insbesondere auch: 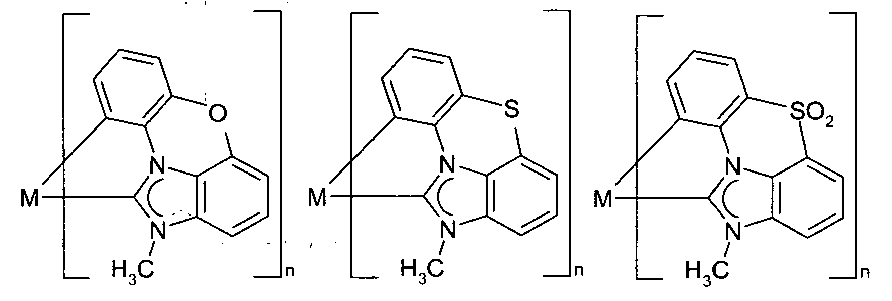 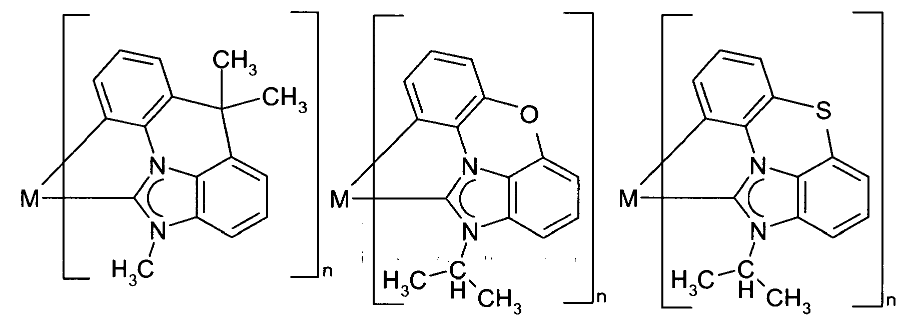 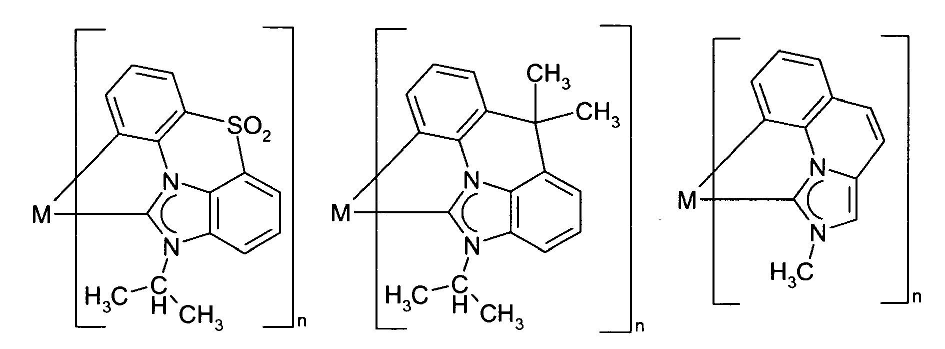 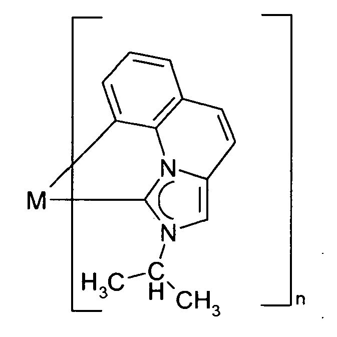 worin M für Ru(III), Rh(III) und insbesondere Ir(III), Pd(II) oder Pt(II) steht, n für Ru(III), Rh(III)und Ir(III) den Wert 3 und für Pd(II) und Pt(II) den Wert 2 annimmt.

Weitere geeignete Metallkomplexe zur Verwendung zusammen mit den Verbindungen der Formel I als Matrixmaterialien und/oder Blockermaterialien in OLEDs sind insbesondere auch: 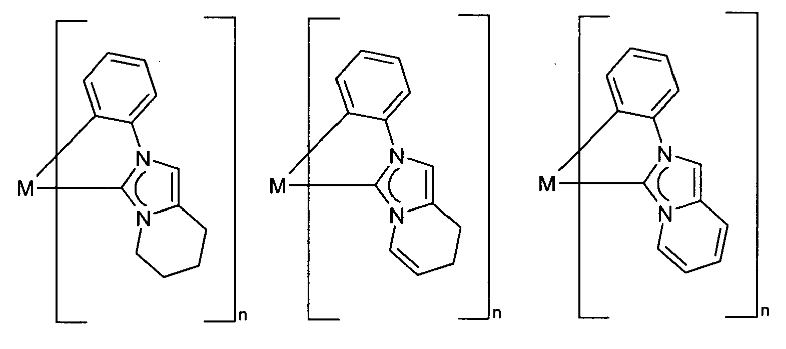 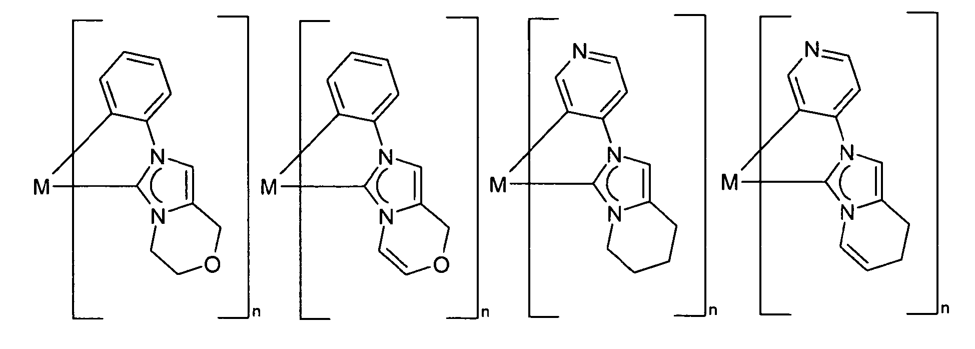 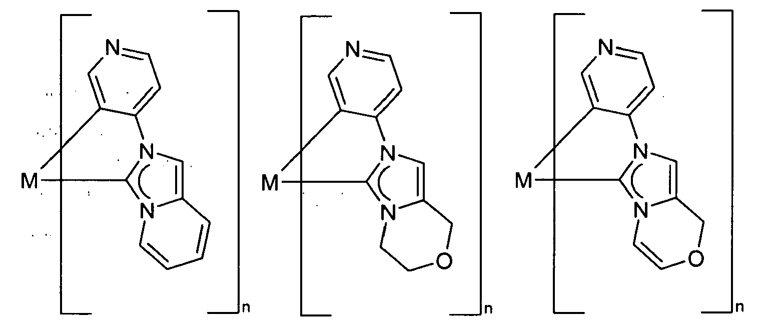 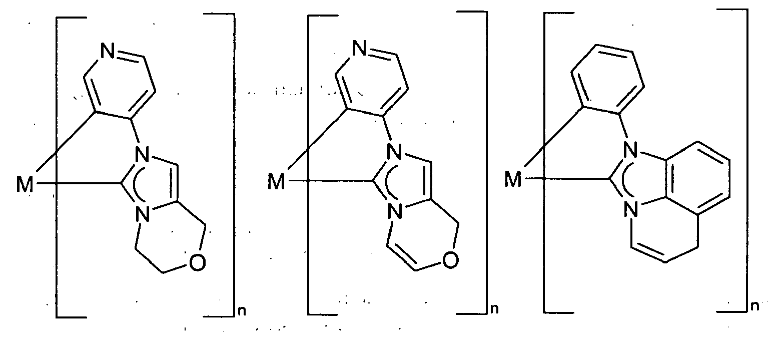 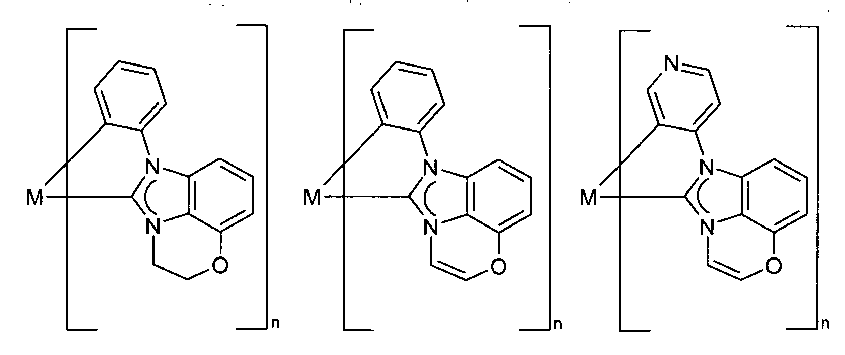 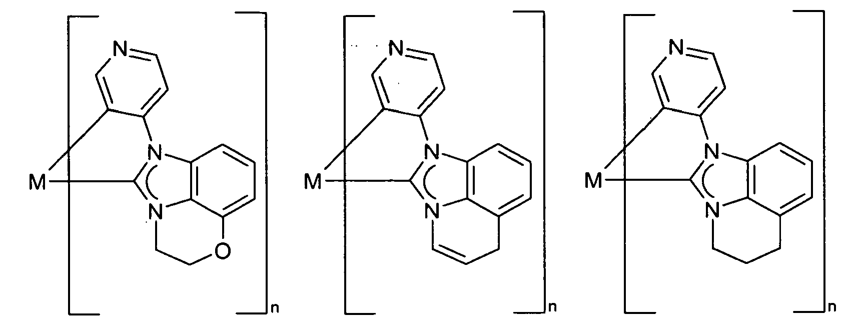 worin M für Ru(III), Rh(III) und insbesondere Ir(III), Pd(II) oder Pt(II) steht, n für Ru(III), Rh(III) und Ir(III) den Wert 3 und für Pd(II) und Pt(II) den Wert 2 annimmt.

Des Weiteren kommen auch Komplexe mit verschiedenen Carbenliganden und/oder mit mono- oder dianionischen Liganden, die sowohl mono- als auch bidentat sein können, in Frage.

Anhand der nachfolgenden Tabelle seien schematisch Komplexe ML'(L")₂ mit dreiwertigen Metallzentren und zwei verschiedenen Carbenliganden L' und L" genannt.

| | | | | | | | |
|---|---|---|---|---|---|---|---|
| L' | L" | L' | L" | L' | L" | L' | L" |
| L¹ | L² | L³ | L⁴ | L⁷ | L⁵ | L⁵ | L³ |
| L¹ | L³ | L³ | L⁵ | L⁷ | L⁴ | L⁵ | L² |
| L¹ | L⁴ | L³ | L⁶ | L⁷ | L³ | L⁵ | L¹ |
| L¹ | L⁵ | L³ | L⁷ | L⁷ | L² | L⁴ | L³ |
| L¹ | L⁶ | L⁴ | L⁵ | L⁷ | L¹ | L⁴ | L² |
| L¹ | L⁷ | L⁴ | L⁶ | L⁶ | L⁵ | L⁴ | L¹ |
| L² | L³ | L⁴ | L⁷ | L⁶ | L⁴ | L³ | L² |
| L² | L⁴ | L⁵ | L⁶ | L⁶ | L³ | L³ | L¹ |
| L² | L⁵ | L⁵ | L⁷ | L⁶ | L² | L² | L¹ |
| L² | L⁶ | L⁶ | L⁷ | L⁶ | L¹ | | |
| L² | L⁷ | L⁷ | L⁶ | L⁵ | L⁴ | | |

wobei M beispielsweise für Ru(III), Rh(III) oder Ir(III), insbesondere Ir(III), und L' und L" beispielsweise für Liganden ausgewählt aus der Gruppe der Liganden L¹ bis L⁷ 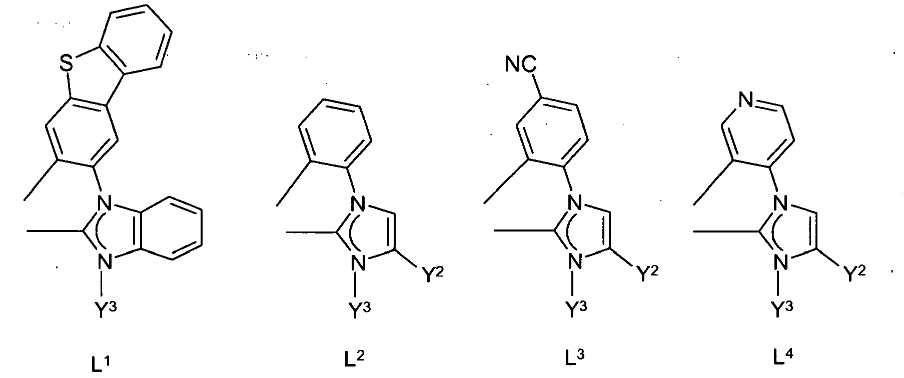 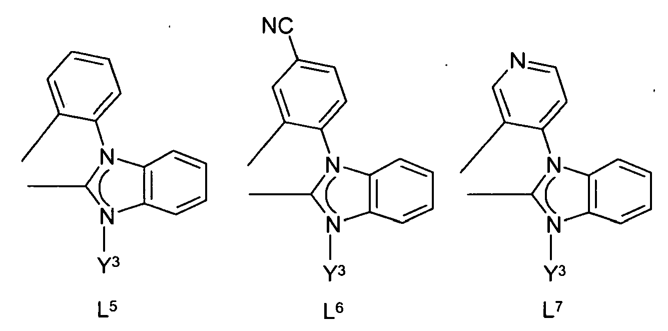 stehen, Y² Wasserstoff, Methyl, Ethyl, n-Propyl, iso-Propyl oder tert.-Butyl und Y³ Methyl, Ethyl, n-Propyl, iso-Propyl oder tert.-Butyl, bezeichnet.

Ein Vertreter dieser Komplexe mit verschiedenen Carbenliganden (L' = L⁴ mit Y² = Wasserstoff und Y³ = Methyl; L" = L² mit Y² = Wasserstoff und Y³ = Methyl) ist beispielsweise:

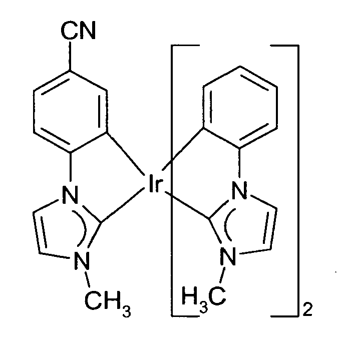

Selbstverständlich können in den als Emitter in den Matrixmaterialien und/oder gemeinsam mit den Blockermaterialien der Formel 1 verwendeten Komplexen dreiwertiger Metallzentren (etwa im Falle von Ru(III), Rh(III) oder Ir(III)) auch alle drei Carbenliganden verschieden voneinander sein.

Beispiele für Komplexe dreiwertiger Metallzentren M mit Liganden L (hier monoanionischer, bidentater Ligand) als "Zuschauerliganden" sind LML'L", LM(L')₂ und L₂ML', worin M etwa für Ru(III), Rh(III) oder Ir(III), insbesondere Ir(III), steht, und L' und L" die zuvor aufgeführte Bedeutung besitzen. Für die Kombination von L' und L" in den Komplexen LML'L" ergibt sich hierbei:

| | | | |
|---|---|---|---|
| L' | L" | L' | L" |
| L¹ | L¹ | L³ | L⁴ |
| L¹ | L³ | L³ | L⁵ |
| L¹ | L⁴ | L³ | L⁶ |
| L¹ | L⁵ | L³ | L⁷ |
| L¹ | L⁶ | L⁴ | L⁵ |
| L¹ | L⁷ | L⁴ | L⁶ |
| L² | L³ | L⁴ | L⁷ |
| L² | L⁴ | L⁵ | L⁶ |
| L² | L⁵ | L⁵ | L⁷ |
| L² | L⁶ | L⁶ | L⁷ |
| L² | L⁷ | | |

Als Liganden L kommen vor allem das Acetylacetonat und dessen Derivate, das Picolinat, Schiffsche Basen, Aminosäuren sowie die in WO 02/15645 A1 genannten bidentaten monoanionischen Liganden in Frage; insbesondere sind das Acetylacetonat und Picolinat von Interesse. Im Falle der Komplexe L₂ML' können die Liganden L gleich oder verschieden sein.

Ein Vertreter dieser Komplexe mit verschiedenen Carbenliganden (L' = L⁴ mit Y² = Wasserstoff und Y³ = Methyl; L" = L² mit Y² = Wasserstoff und Y³ = Methyl) ist beispielsweise: 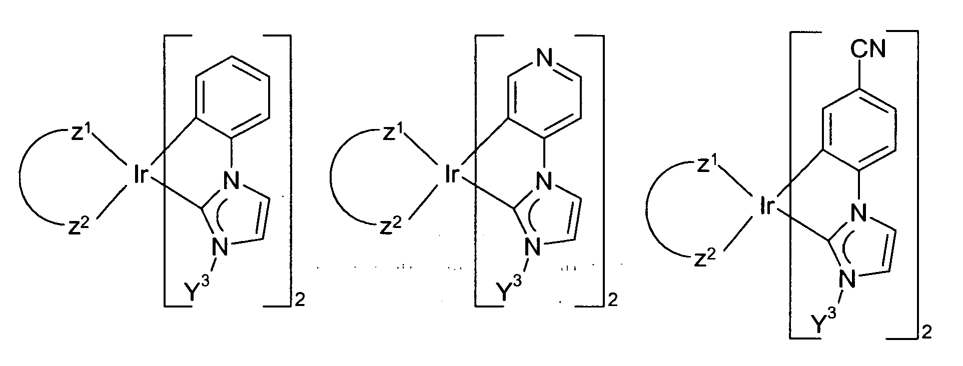 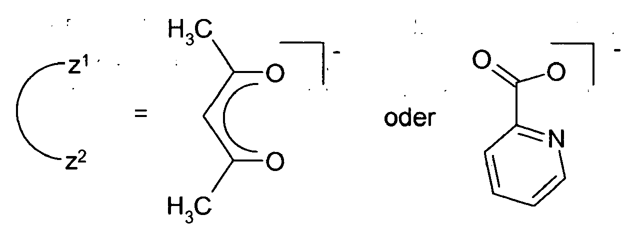 worin z¹ und z² im Symbol 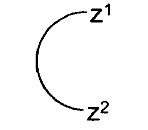 für die beiden Zähne des Liganden L stehen. Y³ bezeichnet Wasserstoff, Methyl, Ethyl, n-Propyl, iso-Propyl oder tert.-Butyl, insbesondere Methyl, Ethyl, n-Propyl oder iso-Propyl.

Weitere insbesondere als Emitter-Verbindungen geeignete Metallkomplexe zur Verwendung gemeinsam mit den Verbindungen der Formel I als Matrixmaterialien in OLEDs sind: 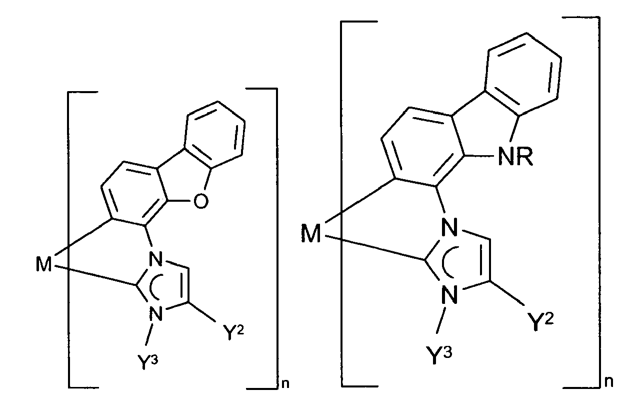 worin R Wasserstoff, Alkyl oder Aryl, bevorzugt Methyl, Ethyl, n-Propyl, iso-Propyl, tert.-Butyl oder Phenyl bedeutet,
sowie 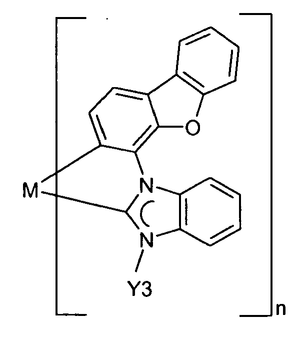 worin M für Ru(III), Rh(III), Ir(III), Pd(II) oder Pt(II) steht, n für den Fall, dass M Ru(III), Rh(III) und Ir(III) bedeutet, den Wert 3 und für den Fall, dass M Pd(II) und Pt(II) bedeutet, den Wert 2 annimmt und Y² und Y³ Wasserstoff, Methyl, Ethyl, n-Propyl, iso-Propyl oder tert.-Butyl bedeuten. Bevorzugt handelt es sich bei M um Ir(III) mit n gleich 3. Y³ bedeutet vorzugsweise Methyl, Ethyl, n-Propyl, iso-Propyl oder tert.-Butyl.

Des Weiteren sind die folgenden speziellen Metallkomplexe für den Einsatz in OLEDs, insbesondere als Emitter-Verbindungen, gemeinsam mit den Verbindungen der Formel I als Matrixmaterialien geeignet: 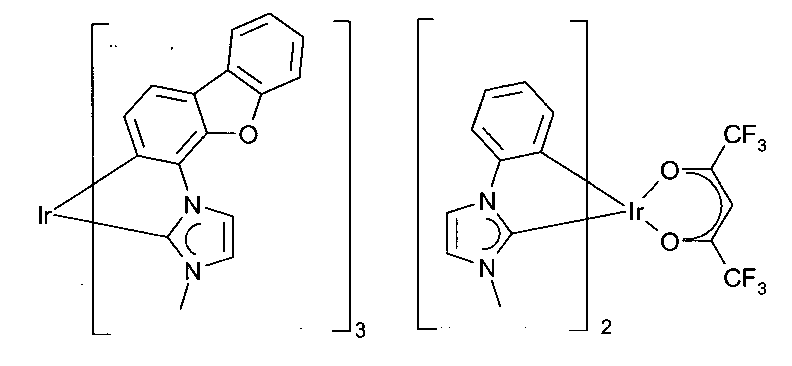 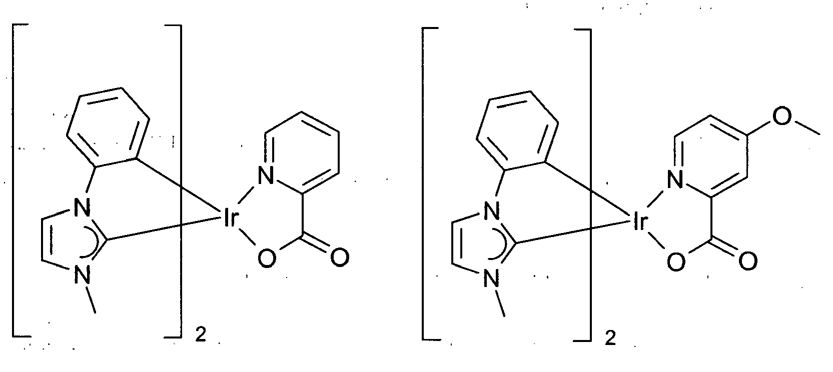 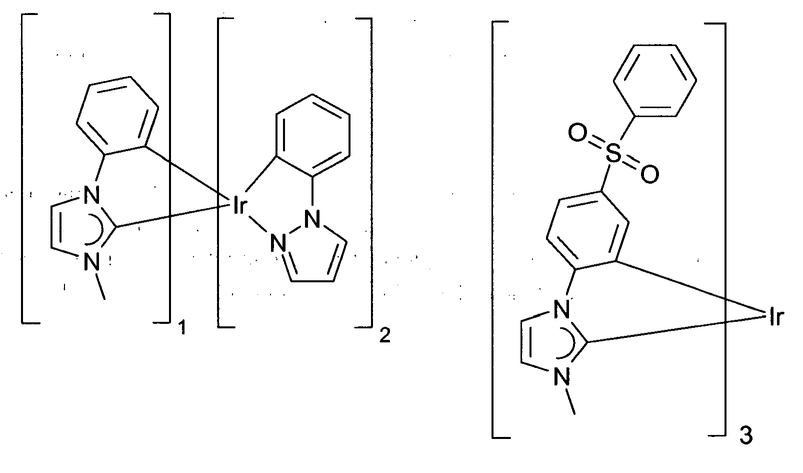 und

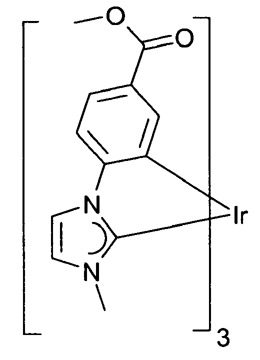

Die Herstellung der vorstehend genannten Carbenkomplexe erfolgt gemäß dem Fachmann bekannten Verfahren. Die Stöchiometrien und Reaktionsbedingungen sind für den Fachmann auf Basis der vorstehend genannten Patentanmeldungen betreffend Carbenkomplexe und deren Herstellungsverfahren problemlos zu ermitteln. Zusätzlich sind im Beispielteil der vorliegenden Anmeldung Verfahren zur Herstellung einiger der vorstehend genannten Carbenkomplexe angegeben. Die nicht ausdrücklich in den Beispielen beschriebenen Carbenkomplexe können in Analogie zu den im Beispielteil beschriebenen Verfahren hergestellt werden.

Wird mindestens eine erfindungsgemäße Verbindung der Formel (I) gemeinsam mit einer Emitter-Verbindung, bevorzugt gemeinsam mit einem Triplett-Emitter, in der Licht-emittierenden Schicht eines OLEDs eingesetzt, was besonders bevorzugt ist, beträgt der Anteil der mindestens einen Verbindung der Formel (I) in der Licht-emittierenden Schicht im Allgemeinen 10 bis 99 Gew.-%, bevorzugt 50 bis 99 Gew.-%, besonders bevorzugt 70 bis 97 Gew.-%. Der Anteil der Emitter-Verbindung in der Licht-emittierenden Schicht beträgt im Allgemeinen 1 bis 90 Gew.-%, bevorzugt1 bis 50 Gew.-%, besonders bevorzugt 3 bis 30 Gew.-%, wobei die Anteile an der mindestens einen Verbindung der Formel (I) und der mindestens einen Emitter-Verbindung im Allgemeinen 100 Gew.-% ergeben. Es ist jedoch auch möglich, dass die Licht-emittierende Schicht neben der mindestens einen Verbindung der Formel (I) und der mindestens einen Emitter-Verbindung weitere Substanzen enthält, zum Beispiel weiteres Verdünnungsmaterial, wobei geeignetes Verdünnungsmaterial nachstehend genannt wird.

Organische Leuchtdioden (OLEDs) sind grundsätzlich aus mehreren Schichten aufgebaut, z.B.:
1. Anode
2. Löcher-transportierende Schicht
3. Licht-emittierende Schicht
4. Elektronen-transportierende Schicht
5. Kathode

Es sind auch von dem vorstehend genannten Aufbau verschiedene Schichtenfolgen möglich, die dem Fachmann bekannt sind. Beispielsweise ist es möglich, dass das OLED nicht alle der genannten Schichten aufweist, zum Beispiel ist ein OLED mit den Schichten (1) (Anode), (3) (Licht-emittierende Schicht) und (5) (Kathode) ebenfalls geeignet, wobei die Funktionen der Schichten (2) (Löcher-transprotierende Schicht) und (4) (Elektronen-transprotierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Die Verbindungen der Formel I können als ladungstransportierende, insbesondere Elektronen-transportierende Materialien eingesetzt werden, sie finden aber vorzugsweise als Matrixmaterialien in der Licht-emittierenden Schicht Verwendung oder als Loch/Excitonenblockerschicht. Mit elektronenschiebenden Substituenten substituierte Verbindungen der Formel (I) können des Weiteren als Elektronen/Excitonenblockerschicht verwendet werden.

Die erfindungsgemäßen Verbindungen der Formel I können als alleiniges Matrixmaterial - ohne weitere Zusätze - in der Licht-emittierenden Schicht vorliegen. Es ist jedoch ebenfalls möglich, dass neben den erfindungsgemäß eingesetzten Verbindungen der Formel I weitere Verbindungen in der Licht-emittierenden Schicht vorliegen. Beispielsweise kann ein fluoreszierender Farbstoff anwesend sein, um die Emissionsfarbe des vorhandenen Emittermoleküls zu verändern. Des Weiteren kann ein Verdünnungsmaterial eingesetzt werden. Dieses Verdünnungsmaterial kann ein Polymer sein, zum Beispiel Poly(N-vinylcarbazol) oder Polysilan. Das Verdünnungsmaterial kann jedoch ebenfalls ein kleines Molekül sein, zum Beispiel 4,4'-N,N'-Dicarbazolbiphenyl (CBP = CDP) oder tertiäre aromatische Amine. Wenn ein Verdünnungsmaterial eingesetzt wird, beträgt der Anteil der erfindungsgemäß eingesetzten Verbindungen der Formel I in der Licht-emittierenden Schicht im Allgemeinen immer noch mindestens 40 Gew.-%, bevorzugt 50 bis 100 Gew.-% bezogen auf das Gesamtgewicht der Verbindungen der Formel I und Verdünnungsmittel.

Die einzelnen der vorstehend genannten Schichten des OLEDs können wiederum aus 2 oder mehreren Schichten aufgebaut sein. Beispielsweise kann die Löcher-transportierende Schicht aus einer Schicht aufgebaut sein, in die aus der Elektrode Löcher injiziert werden, und einer Schicht, die die Löcher von der Loch injizierenden Schicht weg in die Licht-emittierende Schicht transportiert. Die Elektronen-transportierende Schicht kann ebenfalls aus mehreren Schichten bestehen, zum Beispiel einer Schicht, worin Elektronen durch die Elektrode injiziert werden, und einer Schicht, die aus der Elektronen-injizierenden Schicht Elektronen erhält und in die Licht-emittierende Schicht transportiert. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäß als Emittersubstanzen verwendeten organischen Verbindungen angepasst ist.

Um besonders effiziente OLEDs zu erhalten, sollte das HOMO (höchstes besetztes Molekülorbital) der Loch-transportierenden Schicht mit der Arbeitsfunktion der Anode angeglichen sein und das LUMO (niedrigstes unbesetztes Molekülorbital) der Elektronen-transportierenden Schicht sollte mit der Arbeitsfunktion der Kathode angeglichen sein.

Die Anode (1) ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen Ib, IVa, Va und VIa des Periodensystems der Elemente sowie die Übergangsmetalle der Gruppe VIIIa. Wenn die Anode lichtdurchlässig sein soll, werden im Allgemeinen gemischte Metalloxide der Gruppen IIb, IIIb und IVb des Periodensystems der Elemente (alte IUPAC-Version) eingesetzt, zum Beispiel Indium-Zinn-Oxid (ITO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können.

Geeignete Lochtransportmaterialien für die Schicht (2) des erfindungsgemäßen OLEDs sind zum Beispiel in Kirk-Othmer Encyclopedia of Chemical Technologie, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus tris-[N-(1-naphthyl)-N-(phenylamino)]triphenylamin (1-NaphDATA), 4,4'-Bis[N-(1-naphthyl)-N-phenyl[amino]biphenyl (α-NPD), N,N'-Diphenyl-N,N'-bis(3-methylphenyl)-[1,1'-biphenyl]-4,4'-diamin (TPD), 1,1-Bis[(di-4-tolylamino)phenyl]-cyclohexan (TAPC), N,N'-Bis(4-methylphenyl)-N,N'-Bis(4-ethylphenyl)-[1,1'-(3,3'-dimethyl)biphenyl]-4,4'-diamin (ETPD), Tetrakis-(3-methylphenyl)-N,N,N',N'-2,5-phenylendiamin (PDA), α-Phenyl-4-N,N-diphenylaminostyrol (TPS), p-(Diethylamino)-benzaldehyddiphenylhydrazon (DEH), Triphenylamin (TPA), Bis[4-(N,N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-Tetrakis(4-methylphenyl)-(1,1'-biphenyl)-4,4`-diamin (TTB), 4,4',4"-tris(N,N-Diphenylamino)triphenylamin (TDTA), Porphyrinverbindungen und Phthalocyaninen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen und Polyanilinen. Es ist ebenfalls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Weiterhin können die vorstehend als Emittermaterialien genannten Carben-Komplexe als Lochtransportmaterialien eingesetzt werden, wobei die Bandlücke des mindestens einen Lochtransportmaterials im Allgemeinen größer ist als die Bandlücke des eingesetzten Emittermaterials. Dabei ist unter Bandlücke im Sinne der vorliegenden Anmeldung die Triplett-Energie zu verstehen.

Geeignete Elektronentransportmaterialien für die Schicht (4) der erfindungsgemäßen OLEDs umfassen mit oxinoiden Verbindungen chelatisierte Metalle wie 2,2', 2"-(1,3,5-phenylen)tris-[1-phenyl-1H-benzimidazol] (TPBI), Tris(8-chinolinolato)aluminium (Alq₃), Verbindungen auf Phenanthrolinbasis wie 2,9-Dimethyl-4,7-diphenyl-1,10-phenanthrolin (DDPA = BCP) oder 4,7-Diphenyl-1,10-phenanthrolin (DPA) und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ). Dabei kann die Schicht (4) sowohl zur Erleichterung des Elektronentransports dienen als auch als Pufferschicht oder als Sperrschicht, um ein Quenchen des Excitons an den Grenzflächen der Schichten des OLEDs zu vermeiden. Vorzugsweise verbessert die Schicht (4) die Beweglichkeit der Elektronen und reduziert ein Quenchen des Excitons.

Von den vorstehend als Lochtransportmaterialien und Elektronentransportmaterialien genannten Materialien können einige mehrere Funktionen erfüllen. Zum Beispiel sind einige der Elektronen leitenden Materialien gleichzeitig Löcher blockende Materialien, wenn sie ein tief liegendes HOMO aufweisen.

Wie bereits vorstehend erwähnt, können die erfindungsgemäßen Verbindungen der Formel (I) ebenfalls als Ladungstransport oder Blockermaterialien, bevorzugt als Blockermaterialien eingesetzt werden.

Die Ladungstransportschichten können auch elektronisch dotiert sein, um die Transporteigenschaften der eingesetzten Materialien zu verbessern, um einerseits die Schichtdicken großzügiger zu gestalten (Vermeidung von Pinholes/Kurzschlüssen) und um andererseits die Betriebsspannung des Devices zu minimieren. Beispielsweise können die Lochtransportmaterialien mit Elektronenakzeptoren dotiert werden, zum Beispiel können Phthalocyanine bzw. Arylamine wie TPD oder TDTA mit Tetrafluortetracyanchinodimethan (F4-TCNQ) dotiert werden. Die Elektronentransportmaterialien können zum Beispiel mit Alkalimetallen dotiert werden, beispielsweise Alq₃ mit Lithium. Die elektronische Dotierung ist dem Fachmann bekannt und zum Beispiel in W. Gao, A. Kahn, J. Appl. Phys., Vol. 94, No. 1, 1 July 2003 (p-dotierte organische Schichten); A. G. Werner, F. Li, K. Harada, M. Pfeiffer, T. Fritz, K. Leo. Appl. Phys. Lett., Vol. 82, No. 25, 23 June 2003 und Pfeiffer et al., Organic Electronics 2003, 4, 89 - 103 offenbart.

Die Kathode (5) ist eine Elektrode, die zur Einführung von Elektronen oder negativen Ladungsträgern dient. Geeignete Materialien für die Kathode sind ausgewählt aus der Gruppe bestehend aus Alkalimetallen der Gruppe Ia, zum Beispiel Li, Cs, Erdalkalimetallen der Gruppe IIa, zum Beispiel Calcium, Barium oder Magnesium, Metallen der Gruppe IIb des Periodensystems der Elemente (alte IUPAC-Version), umfassend die Lanthaniden und Aktiniden, zum Beispiel Samarium. Des Weiteren können auch Metalle wie Aluminium oder Indium, sowie Kombinationen aller genannten Metalle eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Das OLED gemäß der vorliegenden Erfindung kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einer bevorzugten Ausführungsform enthält das erfindungsgemäße OLED zusätzlich zu den Schichten (1) bis (5) mindestens eine der im Folgenden genannten weiteren Schichten:
- eine Loch-Injektionsschicht zwischen der Anode (1) und der Löcher-transportierenden Schicht (2);
- eine Blockschicht für Elektronen zwischen der Löcher-transportierenden Schicht (2) und der Licht-emittierenden Schicht (3);
- eine Blockschicht für Löcher zwischen der Licht-emittierenden Schicht (3) und der Elektronen-transportierenden Schicht (4);
- eine Elektronen-Injektionsschicht zwischen der Elektronen-transportierenden Schicht (4) und der Kathode (5).

Die erfindungsgemäßen Verbindungen der Formel (I) können - in Abhängigkeit von ihrem Substituionsmuster als Blockermaterial in der Blockschicht für Elektronen oder in der Blockschicht für Löcher als Blockermaterial eingesetzt werden.

Es ist jedoch auch möglich, dass das OLED nicht alle der genannten Schichten (1) bis (5) aufweist, zum Beispiel ist ein OLED mit den Schichten (1) (Anode), (3) (Licht-emittierende Schicht) und (5) (Kathode) ebenfalls geeignet, wobei die Funktionen der Schichten (2) (Löcher-transportiende Schicht) und (4) (Elektronen-transportierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Dem Fachmann ist bekannt, wie er (zum Beispiel auf Basis von elektrochemischen Untersuchungen) geeignete Materialien auswählen muss. Geeignete Materialien für die einzelnen Schichten sind dem Fachmann bekannt und z.B. in WO 00/70655 offenbart.

Des Weiteren kann jede der genannten Schichten des erfindungsgemäßen OLEDs aus zwei oder mehreren Schichten aufgebaut sein. Des Weiteren ist es möglich, dass einige oder alle der Schichten (1), (2), (3), (4) und (5) oberflächenbehandelt sind, um die Effizienz des Ladungsträgertransports zu erhöhen. Die Auswahl der Materialien für jede der genannten Schichten ist bevorzugt dadurch bestimmt, ein OLED mit einer hohen Effizienz und Lebensdauer zu erhalten.

Die Herstellung des erfindungsgemäßen OLEDs kann nach dem Fachmann bekannten Methoden erfolgen. Im Allgemeinen wird das erfindungsgemäße OLED durch aufeinander folgende Dampfabscheidung (Vapor deposition) der einzelnen Schichten auf ein geeignetes Substrat hergestellt. Geeignete Substrate sind zum Beispiel Glas oder Polymerfilme. Zur Dampfabscheidung können übliche Techniken eingesetzt werden wie thermische Verdampfung, Chemical Vapor Deposition und andere. In einem alternativen Verfahren können die organischen Schichten aus Lösungen oder Dispersionen in geeigneten Lösungsmitteln beschichtet werden, wobei dem Fachmann bekannte Beschichtungstechniken angewendet werden.

Im Allgemeinen haben die verschiedenen Schichten folgende Dicken: Anode (1) 500 bis 5000 Å, bevorzugt 1000 bis 2000 Å; Löcher-transportierende Schicht (2) 50 bis 1000 Å, bevorzugt 200 bis 800 Å, Licht-emittierende Schicht (3) 10 bis 1000 Å, bevorzugt 100 bis 800 Å, Elektronen transportierende Schicht (4) 50 bis 1000 Å, bevorzugt 200 bis 800 Å, Kathode (5) 200 bis 10.000 Å, bevorzugt 300 bis 5000 Å. Die Lage der Rekombinationszone von Löchern und Elektronen in dem erfindungsgemäßen OLED und somit das Emissionsspektrum des OLED können durch die relative Dicke jeder Schicht beeinflusst werden. Das bedeutet, die Dicke der Elektronentransportschicht sollte bevorzugt so gewählt werden, dass die Elektronen/Löcher Rekombinationszone in der Licht-emittierenden Schicht liegt. Das Verhältnis der Schichtdicken der einzelnen Schichten in dem OLED ist von den eingesetzten Materialien abhängig. Die Schichtdicken von gegebenenfalls eingesetzten zusätzlichen Schichten sind dem Fachmann bekannt.

Durch Einsatz der erfindungsgemäßen Verbindungen der Formel I als Matrixmaterialien in der Licht-emittierenden Schicht der erfindungsgemäßen OLEDs können OLEDs mit hoher Effizienz erhalten werden. Die Effizienz der erfindungsgemäßen OLEDs kann des Weiteren durch Optimierung der anderen Schichten verbessert werden. Beispielsweise können hoch effiziente Kathoden wie Ca oder Ba, gegebenenfalls in Kombination mit einer Zwischenschicht aus LiF, eingesetzt werden. Geformte Substrate und neue Löcher-transpor-tierende Materialien, die eine Reduktion der Operationsspannung oder eine Erhöhung der Quanteneffizienz bewirken, sind ebenfalls in den erfindungsgemäßen OLEDs einsetzbar. Des Weiteren können zusätzliche Schichten in den OLEDs vorhanden sein, um die Energielevel der verschiedenen Schichten einzustellen und um Elektrolumineszenz zu erleichtern.

Die erfindungsgemäßen OLEDs können in allen Vorrichtungen eingesetzt werden, worin Elektrolumineszenz nützlich ist. Geeignete Vorrichtungen sind bevorzugt ausgewählt aus stationären und mobilen Bildschirmen und Beleuchtuhgseinheiten. Stationäre Bildschirme sind z.B. Bildschirme von Computern, Fernsehern, Bildschirme in Druckern, Küchengeräten sowie Reklametafeln, Beleuchtungen und Hinweistafeln. Mobile Bildschirme sind z.B. Bildschirme in Handys, Laptops, Digitalkameras, Fahrzeugen sowie Zielanzeigen an Bussen und Bahnen.

Weiterhin können die erfindungsgemäßen Verbindungen der Formel I in OLEDs mit inverser Struktur eingesetzt werden. Bevorzugt werden die erfindungsgemäß eingesetzten Verbindungen der Formel I in diesen inversen OLEDs wiederum als Matrixmaterialien in der Licht-emittierenden Schicht eingesetzt. Der Aufbau von inversen OLEDs und die üblicherweise darin eingesetzten Materialien sind dem Fachmann bekannt.

Die nachfolgenden Beispiele erläutern die Erfindung zusätzlich.

### Beispiele

### A: Herstellung von erfindungsgemäßen Verbindungen der Formel (I)

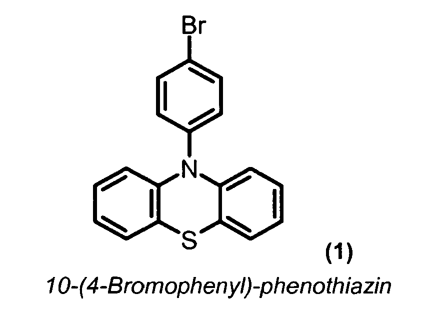

Phenothiazin (31,4 g, 157 mmol), 1-Brom-4-iodbenzol (50,0 g, 173 mmol), Kaliumcarbonat (32,9 g, 238 mmol) und Kupferpulver (2,0 g, 31 mmol) wurden auf 170 °C erhitzt und 7 h bei dieser Temperatur gerührt. Die Reaktionsschmelze wurde auf 130°C abgekühlt, mit Essigester (80 ml) versetzt und 30 min unter Rückfluß erhitzt. Die anfallende Suspension wurde in einen Soxhlet-Extraktor überführt und 16 h bei Rückfluß extrahiert. Die Extraktionslösung wurde unter rühren auf Raumtemperatur abkühlt. Dreiviertel des Lösungsmittels wurde abdestilliert und mit Ethanol (30 ml) wurde das Wertprodukt ausgefällt und filtriert. Es wurden 26,7 g (48 % d. Th.) farblose Kristalle mit einem Schmelzpunkt von 132 - 137 °C erhalten.

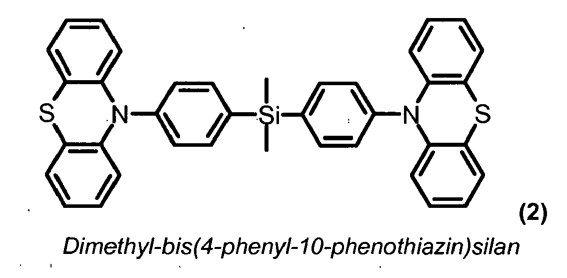

Zu einer Mischung aus Magnesium (0,56 g, 22,6 mmol) und trockenem THF (6 ml) wurde eine Lösung aus 10-(4-Bromophenyl)-phenothiazin (8,0 g, 22,6 mmol) in trockenem THF (24 ml) gegeben. Nach 2h Rückfluß wurde SiMe₂Cl₂ (1,46 g, 11,3 mmol) zugegeben. Nach 2 h Rückfluß, wurde die Lösung abgekühlt, filtriert und mit Eis und Diethylether versetzt. Die organische Phase wurde abgetrennt und eingeengt. Der Rückstand wurde aus DMF kristalliert. Es wurden 3,00 g (44% d. Th) elementaranalysereine farblose Kristalle erhalten.

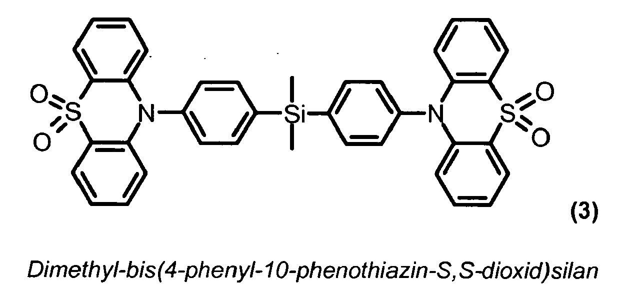

Dimethyl-bis(4-phenyl-10-phenothiazin)silan (2,97 g, 4,90 mmol) wurde in Methylenchlorid (280 ml) gelöst. Nach 15 min Rühren bei Raumtemperatur wurden 77 %ige m-Chlorperbenzoesäure (5,80 g, 23,4 mmol) portionsweise zugegeben. Die Reaktionslösung wurde 24 h bei Raumtemperatur gerührt. Die organische Phase wurde mit 10%iger Natronlauge (50 ml), 5%iger Salzsäure (50 ml) und mit gesättigter Natriumhydrogencarbonatlösung (50 ml) gewaschen und eingeengt. Der Rückstand wurde aus DMF kristallisiert. Es wurden 2,8 g (85 % d. Th.) farblose Kristalle mit einem Schmelzpunkt von 293-298°C erhalten.

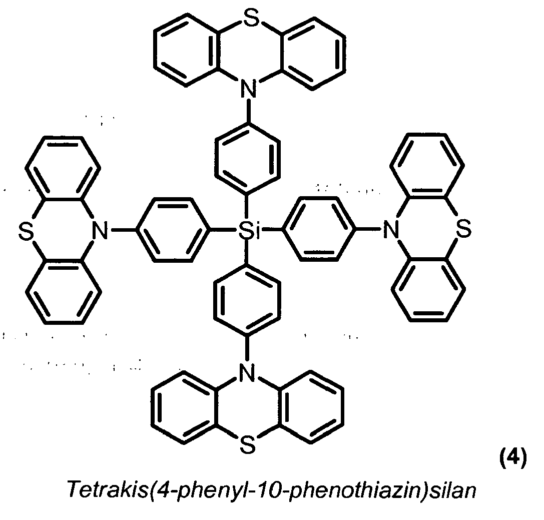

Zu einer Lösung aus 10-(4-Bromophenyl)-phenothiazin (8,0 g, 22,6 mmol) in trockenem THF (120 ml) wurde bei -78°C nBuLi (14,8 ml, 1,6 M in Hexan) gegeben. Nach 1 h rühren wurde SiMe₂Cl₂ (0,93 g, 5,4 mmol) in trockenem THF (12 ml) zugegeben. Nach 2 h bei -78°C wurde die Lösung auf Raumtemperatur erwärmt und über Nacht gerührt. Der Niederschlag wurde filtriert und der Rückstand mit gesättigter Ammoniumchloridlösung und VE-Wasser gewaschen. Der Rückstand wurde aus DMF kristalliert. Es wurden 3,44 g (57% d. Th) des Zielprodukts erhalten. 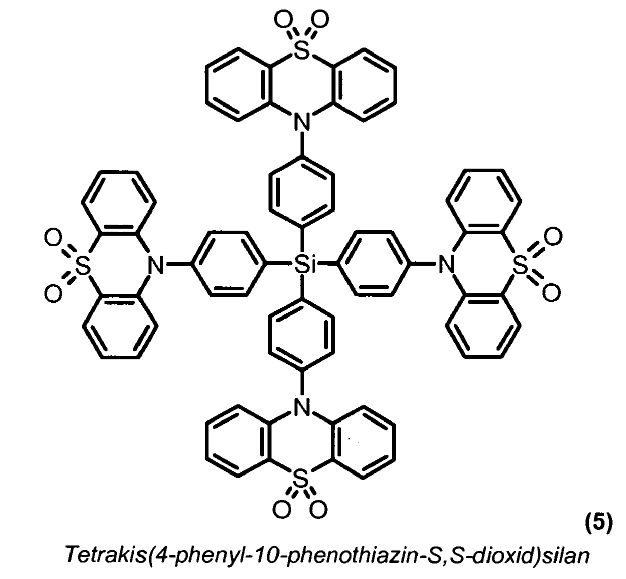 Tetrakis(4-phenyl-phenothiazin)silan (3,47 g, 3,08 mmol) wurde in Methylenchlorid (1500 ml) suspendiert. Nach 15 min rühren bei Raumtemperatur wurde 77 %ige m-Chlorperbenzoesäure (7,30 g, 29,6 mmol) portionsweise zugegeben. Die entstandene Lösung wurde 20 h bei Raumtemperatur gerührt. Die organische Phase wurde mit 10%iger Natronlauge (3 x 30 ml), 5%iger Salzsäure (30 ml) und mit gesättigter Natriumhydrogencarbonatlösung (30 ml) gewaschen und eingeengt. Der Rückstand wurde aus Methylenchlorid/Aceton kristallisiert. Es wurden 1,74 g (45 % d. Th.) farblose Kristalle erhalten.

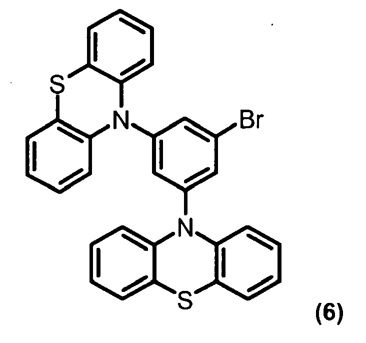

Natriumhydrid (60%ige Dispersion in Parafinöl) (28,0 g, 700,0 mmol) wurde mit trockenem DMF (700 ml) unter N₂ gemischt. Unter Rühren wird Phenothiazin (140,7 g, 700,0 mmol) in 10 min zugefügt. Nach Ende der Wasserstoffentwicklung (20 min) wurde 1-Brom-3,5-Difluorbenzol (68,95 g, 350,0 mmol) in DMF (70 ml) in 15 min zugrefügt. Das Gemisch wurde 18 h bei 100°C gerührt. Natriumhydrid (60%ige Dispersion in Parafinöl) wurde zugegeben (4,0 g,1 00,0 mmol) und weitere 5 h bei 100°C wurde gerührt. Natriumhydrid (60%ige Dispersion in Parafinöl) wurde zugegeben (4,0 g, 100,0 mmol) und weitere 18 h bei 100°C wurde gerührt. Das Gemisch wurde abgekühlt auf Raumtemperatur, filtriert und mit DMF gewaschen. Der rückstand wurde mit Cyclohexan/Essigester (5:2, 500 ml) versetzt und abfiltriert. Es wurden 38,9 g (20 % d. Th.) hellgelber Feststoff (6) erhalten.

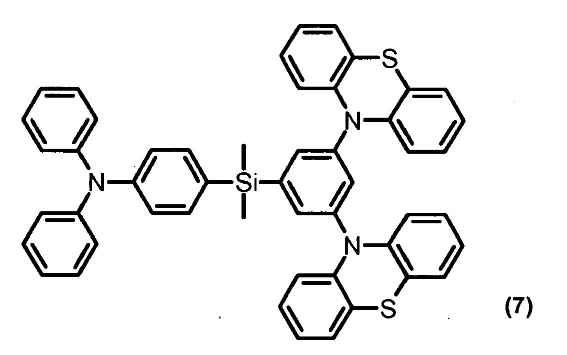

Zu einer Lösung aus (4-Bromphenyl)diphenylamin (8,40g, 25,20 mmol) in trockenem THF (105 ml) auf -78°C wurde nBuLi (16,8 ml, 26,6 mmol, 1,6 M in Hexan) zugetropft. Nach 60 min bei -78°C rühren wurde diese Lösung an einer Lösung aus Dichlordimethylsilan (3,47 g, 26,6 mmol) in THF (140ml) bei -78°C zugetropft. Das Reaktionsgemisch wurde in 40min auf 0°C erwärmt und 1 h bei 0°C gerührt. Der Ansatz wurde erneut auf -78°C gekühlen und eine Lösung aus 1-Lithium-3,5-bis(10-phenothiazin)phenylat (aus (6) (14,0 g, 25,2 mmol) und nBuLi (16,8 ml, 26,6 mmol, 1,6 M in Hexan)) in THF (105 ml) wurde zugegeben. Nach 1 h rühren bei -78°C wurde das Gemisch auf Raumtemperatur erwärmt und übernacht gerührt. Das Gemisch wure mit gesättigter Ammoniumchloridlösung (70 ml) versetzt und filtriert. Die organische Phase wurde mit Wasser gewaschen und getrocknet (Na₂SO₄). Nach Säulenchromatographie (SiO₂, Hexan/Essigester 40:1) und Umkristallisation aus Essigester wurde 2,88 g (14 % d. Th.) des Zielprodukts (7) erhalten.

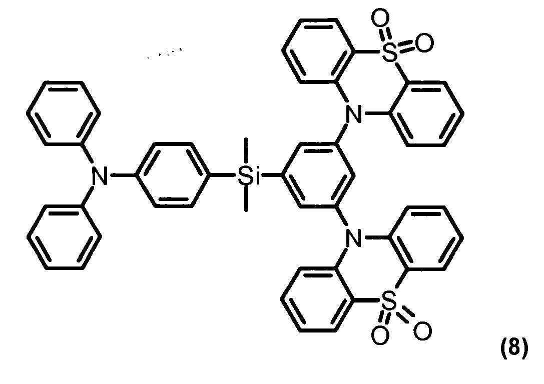

(7) (2,8 g, 3,6 mmol) wurde in Methylenchlorid (100 ml) gelöst. Nach 15 min rühren bei Raumtemperatur wurde 77 %ige m-Chlorperbenzoesäure (3,55 g, 14,4 mmol) in Methylenchlorid (40 ml) bei 0-5°C langsam zugetropft. Die entstandene Lösung wurde 20 h bei 0-5°C gerührt. Die organische Phase wurde mit 10%iger Natronlauge (3 x 20 ml), 5%iger Salzsäure (30 ml) und mit gesättigter Natriumhydrogencarbonatlösung (30 ml) gewaschen, getrocknet (Na₂SO₄) und eingeengt. Nach Säulenchromatographie (SiO₂, CH₂Cl₂) wurden 1,1 g (38 % d. Th.) des Zielprodukts (8) erhalten.

### B: Anwendungsbeispiel: Herstellung eines OLED

### B1: Verwendung der Verbindung (3) als Matrixmaterial

Das als Anode verwendete ITO-Substrat wird zuerst mit kommerziellen Reinigungsmitteln für die LCD-Produktion (Deconex^{®} 20NS und Neutralisationsmittel 250RGAN-ACID^{®}) und anschließend in einem Aceton/Isopropanol-Gemisch im Ultraschallbad gesäubert. Zur Beseitigung möglicher organischer Rückstände wird das Substrat in einem Ozonofen weitere 25 Minuten einem kontinuierlichen Ozonfluss ausgesetzt. Diese Behandlung verbessert auch die Lochinjektionseigenschaften des ITOs.

Danach werden die nachfolgend genannten organischen Materialien mit einer Rate von ca. 0.5-5 nm/min bei etwa 10⁻⁹ mbar auf das gereinigte Substrat aufgedampft. Als Lochleiter und Excitonenblocker wird Ir(dpbic)₃ (V1) mit einer Dicke von 20 nm auf das Substrat aufgebracht. 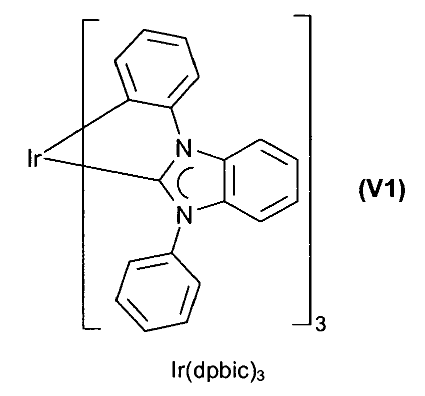 (Zur Herstellung siehe Ir-Komplex (7) in der Anmeldung WO 2005/019373 A2).

Anschließend wird eine Mischung aus 16 Gew.-% der Verbindung CN-PMBIC (V2): 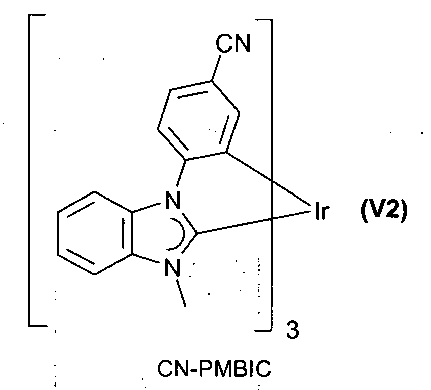 (Zur Herstellung siehe Beispiel 3 in WO 2006/056418 A2).

und 84 Gew.-% der Verbindung Dimethyl-bis(4-phenyl-phenothiazin-S,S-dioxid)silan (3) in einer Dicke von 20 nm aufgedampft, wobei erstere Verbindung als Emitter, letztere als Matrixmaterial fungiert.

Anschließend wird das Material mPTO2 (1,3-Phenylen-10,10'-bis(phenothiazin)-5,5'-dioxid (V3)) mit einer Dicke von 10 nm als Excitonen- und Lochblocker aufgedampft. 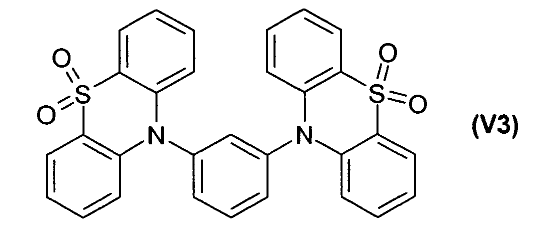 Als nächstes wird ein Elektronentransportmaterial TPBI (1,3,5-tris(N-phenylbenzylimidazol-2-yl)benzen) in einer Dicke von 65 nm, eine 0,75 nm dicke Lithiumfluorid-Schicht und abschließend eine 110 nm dicke Al-Elektrode aufgedampft.

Zur Charakterisierung des OLEDs werden Elektrolumineszenz-Spektren bei verschiedenen Strömen bzw. Spannungen aufgenommen. Weiterhin wird die Strom-Spannungs-Kennlinie in Kombination mit der abgestrahlten Lichtleistung gemessen. Die Lichtleistung kann durch Kalibration mit einem Luminanzmeter in photometrische Größen umgerechnet werden.

Für das beschriebene OLED ergeben sich die folgenden elektrooptischen Daten:

| | |
|---|---|
| Emissionsmaximum | 455 nm |
| CIE(x,y) | 0,17; 0,13 |
| Photometrischer Wirkungsgrad bei 3 V | 6,0 cd/A |
| Leistungseffizienz bei 3 V | 6,3 Im/W |
| Externe Quantenausbeute bei 3 V | 5,4 % |
| Leuchtdichte bei 10V | 400 cd/m² |

### B2: Verwendung der Verbindng (3) als Excitonen- und Lochblocker

Das als Anode verwendete ITO-Substrat wird zuerst mit kommerziellen Reinigungsmitteln für die LCD-Produktion (Deconex^{®} 20NS und Neutralisationsmittel 250RGAN-ACID^{®}) und anschließend in einem Aceton/Isopropanol-Gemisch im Ultraschallbad gesäubert. Zur Beseitigung möglicher organischer Rückstände wird das Substrat in einem Ozonofen weitere 25 Minuten einem kontinuierlichen Ozonfluss ausgesetzt. Diese Behandlung verbessert auch die Lochinjektionseigenschaften des ITOs.

Danach werden die nachfolgend genannten organischen Materialien mit einer Rate von ca. 0.5-5 nm/min bei etwa 10⁻⁹ mbar auf das gereinigte Substrat aufgedampft. Als Lochleiter und Excitonenblocker wird Ir(dpbic)₃ (V1) mit einer Dicke von 30 nm auf das Substrat aufgebracht. 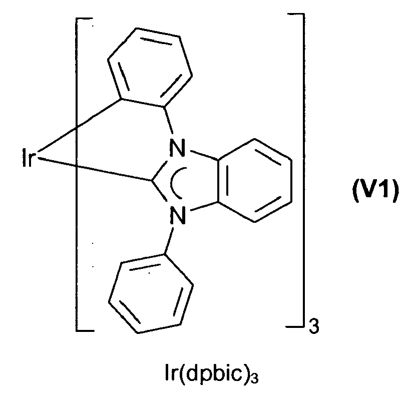 (Zur Herstellung siehe Ir-Komplex (7) in der Anmeldung WO 2005/019373 A2).

Anschließend wird eine Mischung aus 30 Gew.-% der Verbindung CN-PMBIC 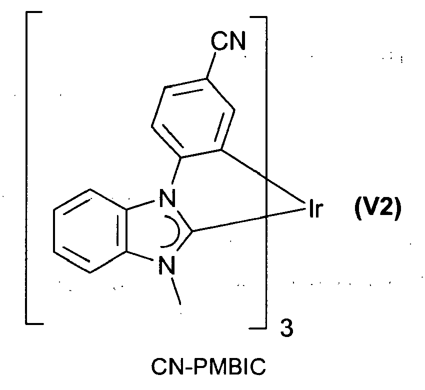 (Zur Herstellung siehe Beispiel 3 in WO 2006/056418 A2).
und 70 Gew.-% der Verbindung (V4) in einer Dicke von 20 nm aufgedampft, wobei erstere Verbindung als Emitter, letztere als Matrixmaterial fungiert.

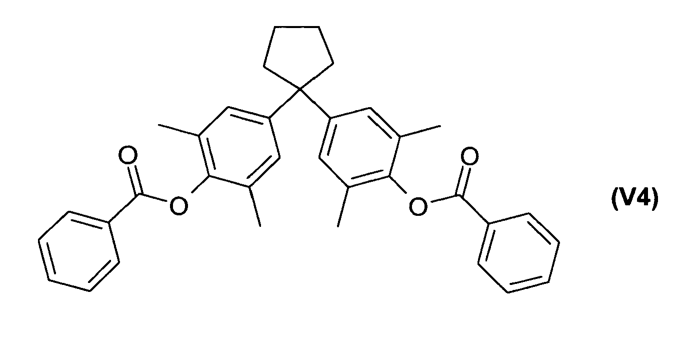

Anschließend wird Verbindung (3) mit einer Dicke von 10 nm als Excitonen- und Lochblocker aufgedampft.

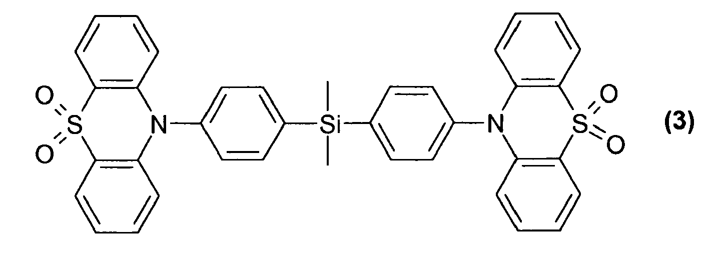

Als nächstes wird ein Elektronentransportmaterial TPBI (1,3,5-tris(N-phenylbenzylimidazol-2-yl)benzen) in einer Dicke von 40 nm, eine 0,75 nm dicke Lithiumfluorid-Schicht und abschließend eine 110 nm dicke Al-Elektrode aufgedampft.

Zur Charakterisierung des OLEDs werden Elektrolumineszenz-Spektren bei verschiedenen Strömen bzw. Spannungen aufgenommen. Weiterhin wird die Strom-Spannungs-Kennlinie in Kombination mit der abgestrahlten Lichtleistung gemessen. Die Lichtleistung kann durch Kalibration mit einem Luminanzmeter in photometrische Größen umgerechnet werden.

Für das beschriebene OLED ergeben sich die folgenden elektrooptischen Daten:

| | |
|---|---|
| Emissionsmaximum | 455 nm |
| Blocker | |
| CIE(x,y) | 0,16; 0,12 |
| Photometrischer Wirkungsgrad bei 4,5 V | 8,8 cd/A |
| Leistungseffizienz bei 4,5 V | 6,1 Im/W |
| Externe Quantenausbeute bei 4,5 V | 8,8 % |

### C: Verfahren zur Herstellung von ausgewählten Carbenkomplexen

### Ir(DBF-MIC)₃ (V5)

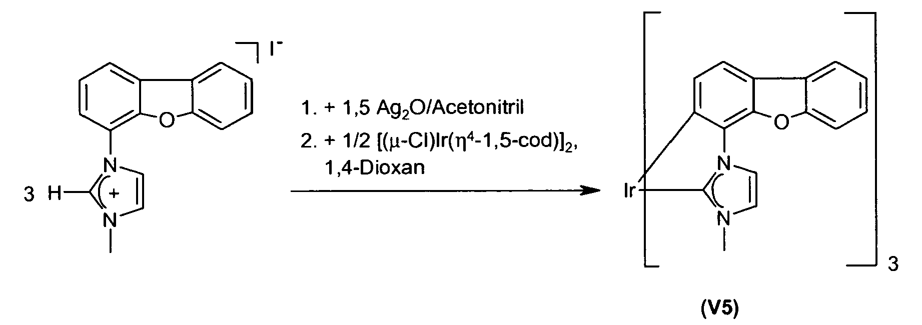

3.48 g (9.2 mmol) Imidazoliumiodid und 1.07 g (4.6 mmol) Silberoxid werden in 60 ml Acetonitril suspendiert und bei Raumtemperatur über Nacht gerührt. Anschließend wird die Suspension zur Trockene eingeengt, mit 100 ml 1,4-Dioxan aufgenommen und zu einer Lösung aus 0.62 g (0.92 mmol) [(µ-Cl)(η⁴-1,5-cod)Ir]₂ und 60 ml 1,4-Dioxan innerhalb von einer halben Stunde dosiert. Im Anschluss wird eine Stunde bei Raumtemperatur, zwei Stunden bei 70°C und 18 Stunden unter Rückfluss gerührt. Nach dem Abkühlen wird das Reaktionsgemisch zur Trockene eingeengt, mit Dichlormethan extrahiert und der Extrakt einer säulen-chromatographischen Aufreinigung (Eluent: 1. Methylenchlorid: Cyclohexan 2:1 zur Isolierung des Isomerengemisches und 2. Essigsäureethylester: Cyclohexan 1:1 zur Trennung der Isomeren, Verhältnis der Isomeren im Reaktionsgemisch : *mer*/*fac* ca. 3/1) unterzogen. Man erhält ca. 0.61 g (35%) *mer-*Isomer und 0.1 g (6%) fac-Isomer als hellgelbes Pulver.

### meridionales Isomer:

¹H-NMR: (DMSO, 500 MHz): 8.40 (d, *J*= 2.0 Hz, 1H, CH), 8.34 (d, *J*= 2.1Hz, 1H, CH), 8.22 (d, *J* = 2.1 Hz, 1H, CH), 7.90 - 7.85 (m, 1H, CH), 7.69 - 7.65 (m, 1H, CH), 7.43 - 7.13 (m, 16H, CH), 6.75 (d, ³*J*_{H},_{H} = 7.5 Hz, 1H, CH), 6.68 (d, ³*J*_{H},_{H} = 7.7 Hz, 1H, CH), 6.56 (d, ³*J*_{H},_{H} = 7.6 Hz, 1H, CH), 3.13 (s, 3H, CH₃), 3.05 (s, 3H, CH₃), 2.99 (s, 3H, CH₃).

### faciales Isomer:

¹H-NMR: (DMSO, 500 MHz): 8.28 (d, *J*= 1.95 Hz, 3H, CH), 7.88-7.89 (m, 3H, CH), 7.67-7.68 (m, 3H, CH), 7.43 - 7.12 (m, 12H, CH), 6.50 (d, *J*_{H},_{H}= 7.5 Hz, 3H, CH), 3.14 (s, 9H, CH₃).

### [(PMIC)₂IrCl]₂ (V6)

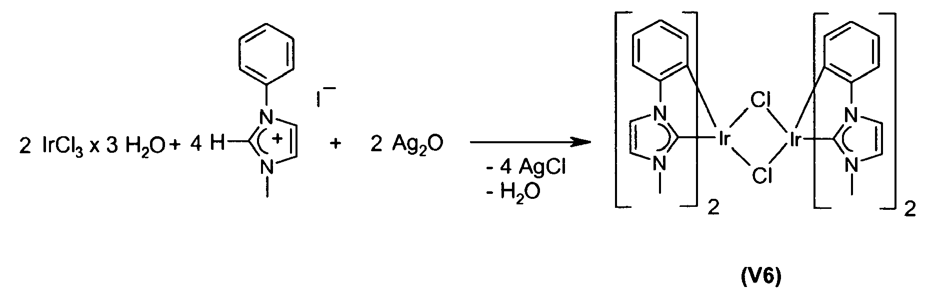

4.29 g (18.5 mmol) Silberoxid, 9.47g (33.1 mmol) Imidazoliumiodid und 3.56 g (10.1 mmol) Iridiumtrichloridtrihydrat werden in 350 ml 2-Ethoxyethanol suspendiert und 15 h bei 120°C im Dunkeln gerührt. Danach entfernt man das Lösungsmittel im Vakuum und extrahiert den Rückstand mit Methylenchlorid. Der Extrakt wird auf ca. ein Viertel seines Volumens eingeengt und mit Methanol versetzt. Der ausfallende Niederschlag wird abfiltriert und getrocknet. Man erhält 1.7 g [(PMIC)₂IrCl]₂(31%).

¹ H-NMR: (CD₂Cl₂, 500 MHz): δ = 7.59 (d, *J* = 2.3 Hz, 4H, CH), 7.17 (d, *J* = 1.7 Hz, 4H, CH), 6.99 (d, ³*J*_{H},_{H} = 7.2 Hz, 4H, CH), 6.73 (pt, ³*J*_{H},_{H} = 7.5 Hz, 4H, CH), 6.45 (pt, ³*J*_{H},_{H} = 7.9 Hz, 4H, CH), 6.09 (d, ³*J*_{H},_{H} = 7.3 Hz, 4H, CH), 3.91 (s, 12H, CH₃).

### (PMIC)₂Irpic (V7)

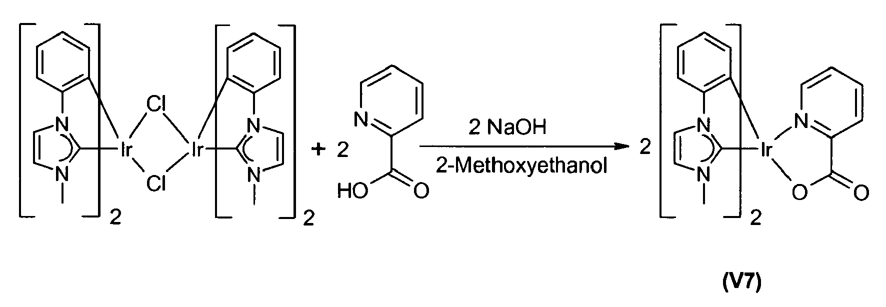

Eine Lösung von 0.41 g (3.32 mmol) Picolinsäure in Methoxyethanol (30 ml) wird innerhalb von 10 min mit 3.32 ml Natronlauge (1 M, 3.32 mmol) versetzt und 15 min bei Raumtemperatur gerührt. Danach wird das Reaktionsgemisch innerhalb von 10 min zu einer Suspension von 0.9 g (0.83 mmol) [(PMIC)₂IrCl]₂ in Methoxyethanol (80 ml) gegeben. Es wird 15 min bei Raumtemperatur gerührt und anschließend 21 h am Rückfluss erhitzt. Nach dem Abkühlen wird das Reaktionsgemisch mit Wasser (300 ml) versetzt. Der dabei entstandene Niederschlag wird abfiltriert, getrocknet und einer säulenchromatographischen Reinigung unterworfen (Eluent: Ethylacetat/Methanol = 1/0.25). Man erhält 0.64 g (PMIC)₂IrPic (61%).

¹H-NMR: (CD₂Cl₂, 500 MHz): δ = 3.00 (s, 3H, CH₃), 3.86 (s, 3H, CH₃), 6.31-6.33 (m, 1H, CH), 6.42-6.44 (m, 1H, CH), 6.59-6.63 (m, 2H, CH), 6.83-6.88 (m, 2H, CH), 6.90-6.91 (m, 1H, CH), 6.98-6.99 (m, 1H, CH), 7.08 (d, *J* = 7.8 Hz, 2H, CH), 7.21-7.24 (m, 1H, CH), 7.46-7.47 (m, 2H, CH), 7.80-7.83 (dt, *J* = 7.7 Hz, *J* = 1.5 Hz, 1H, CH), 7.92-7.93 (m, 1H, CH), 8.13-8.15 (m, 1H, CH).

### (PMIC)₂IrPicOMe (V8)

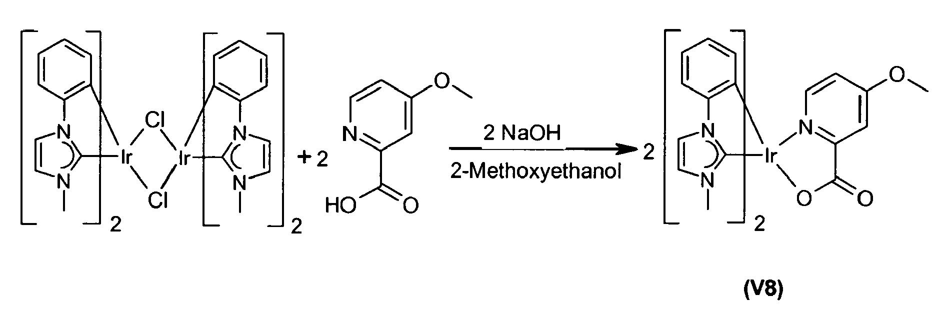

Eine Suspension von 0.68 g (4.44 mmol) 4-Methoxypicolinsäure in 70 ml 2-Methoxyethanol wird innerhalb von 10 min mit 4.44 ml Natronlauge (1M, 4.44 mmol) versetzt. Es wird 15 min bei Raumtemperatur gerührt, bevor man das Gemisch langsam zu einer Suspension aus 1.2 g (1.11 mmol) [(PMIC)₂IrCl]₂ und 80ml 2-Methoxyethanol gibt. Es wird 15 min bei Raumtemperatur gerührt und anschließend 21 h am Rückfluss erhitzt. Nach dem Abkühlen wird das Reaktionsgemisch mit Wasser (600 ml) versetzt. Der dabei entstandene Niederschlag wird abfiltriert, getrocknet und säulenchromatographisch gereinigt (Eluent: Ethylacetat/Methanol = 1/0.25). Man erhält 0. 93 g PMIC₂IrPicOMe (64%).

¹H-NMR: (CD₂Cl₂, 500 MHz): δ = 3.07 (s, 3H, CH₃), 3.85 (s, 3H, CH₃), 3.91 (s, 3H, CH₃), 6.33 (dd, *J* = 7.3 Hz, *J* = 1.4 Hz, 1H, CH), 6.42 (dd, *J* = 7.4 Hz, *J* = 1.4 Hz, 1H, CH), 6.57-6.61 (m, 2H, CH), 6.74 (dd, *J* = 6.3 Hz, *J* = 2.9 Hz, 1H, CH), 6.81-6.86 (m, 2H, CH), 6.92 (d, *J* = 2.1 Hz, 1H, CH), 6.98 (d, *J* = 2.1 Hz, 1H, CH), 7.07 (dd, *J* = 4.8 Hz, *J* = 1.3 Hz, 1H, CH), 7.08 (dd, *J* = 4.6 Hz, *J* = 1.3 Hz, 1H, CH), 7.46 (dd, *J* = 4.1 Hz, *J* = 2.1 Hz, 2H, CH), 7.65 (d, *J* = 6.2 Hz, 1H, CH), 7.72 (d, *J* = 2.5 Hz, 1H, CH).

### (PMIC)₂Ir(acac-F₆) (V9)

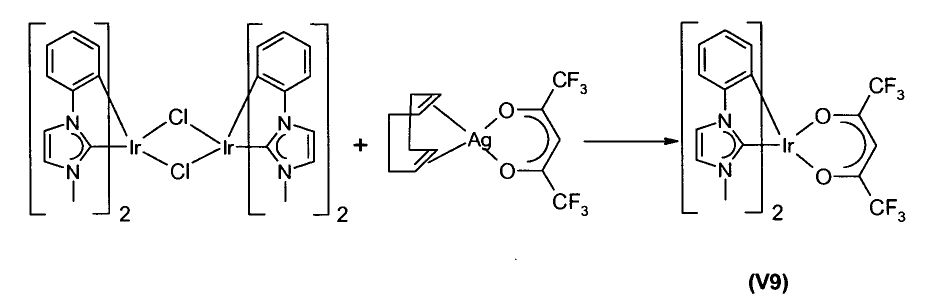

Eine Lösung von 0.22 g (0.2 mmol) [(PMIC)₂lrCl]₂ in 60 ml Methylenchlorid wurden mit einer Lösung von 0.17 g (0.4 mmol) (cod)Ag(acac-F₆) in 30 ml Methylenchlorid versetzt. Es wurde 2 h unter Rückfluss und 18 h bei Raumtemperatur gerührt. Dann wurde das Lösungsmittel im Vakuum entfernt und der Rückstand säulenchromatographisch gereinigt (Eluent CH₂Cl₂). Man erhielt 0.28 g (**96%**) rotes Pulver.

¹H-NMR: (CD₂Cl₂, 500 MHz): δ = 7.50 (s, 2H), 7.11 (s, 2H), 7.03 (m, 2H), 6.81 (m, 2H), 6.56 (m, 2H), 6.19 (m, 2H), 5.98 (s, 1H), 3.79 (s, 6H).

### mer-Tris-[1-(4'-phenylsulfonylphenyl)-3-methylbenzimidazol-2-yliden-C2, C2']-iridium(III)(V10)

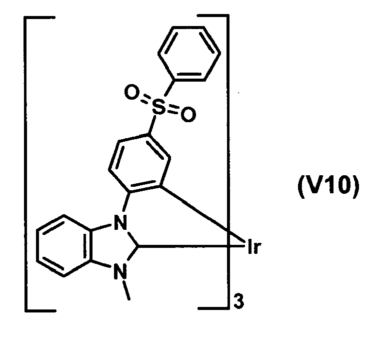

### 1-(4'-Phenylsulfonylphen yl)-benzimidazol

Zu einer Lösung von Benzimidazol (11.8 g, 0.10 mol) in DMF (500 mL) wird bei Raumtemperatur unter Stickstoff Natriumhydrid (60% in Mineralöl, 4.4 g, 0.11 mol) gegeben und 10 min gerührt. Die Mischung wird mit 4-Chlorphenyl-phenylsulfon (26.1 g, 0.10 mol) versetzt und 16 h bei 100 °C gerührt. Nach erneuter Zugabe von Natriumhydrid (60% in Mineralöl, 2.0 g, 0.05 mol) bei Raumtemperatur wird die Mischung 16 h bei 130°C gerührt. Nach Abkühlung auf Raumtemperatur wird die Mischung auf Eiswasser gegeben. Ausgefallenes Produkt wird abfiltriert und mit Wasser gewaschen. Ausbeute: 91%.

¹H-NMR (*d*₆-DMSO, 400 MHz): δ = 7.35 (m_{c}, 2H), 7.64-7.82 (m, 5H), 7.98 (d, 2H), 8.06 (d, 2H), 8.20 (d, 2H), 8.67 (s, 1H).

### 1-(4'-Phenylsulfonylphenyl)-3-methylbenzimidazolium-tetrafluoroborat

Eine Lösung von 1-(4'-Phenylsulfonylphenyl)-benzimidazol (6.7 g, 20 mmol) in Dichlormethan (100 mL) wird bei -10 °C mit Trimethyloxonium-tetrafluoroborat (3.3 g, 22 mmol) versetzt und 16 h unter Argon gerührt. Nach Zugabe von Ethanol wird der entstandene Niederschlag abfiltriert und mit kaltem Petrolether gewaschen. Ausbeute: 80%.

¹H-NMR (*d*₆-DMSO, 400 MHz): δ = 4.17 (s, 3H), 7.67-7.83 (m, 5H), 7.94 (d, 1H), 8.04-8.12 (m, 4H), 8.15 (d, 1H), 8.36 (d, 2H), 10.14 (s, 1H).

### mer-Tris-[1-(4 '-phenylsulfonylphenyl)-3-methylbenzimidazol-2-yliden-C2, C2']-iridium(III)(V11)

Eine Suspension von 1-(4'-Phenylsulfonylphenyl)-3-methylbenzimidazoliumtetrafluoroborat (4.4g, 10 mmol) in Dioxan (100mL) wird unter Argon bei Raumtemperatur mit KHMDS (0.5 M in Toluol, 20mL, 10 mmol) versetzt und 15 min gerührt. Nach Zugabe von 1,5-Cyclooctadien-iridium(I)chlorid-Dimer (0.7 g, 1 mmol) wird die Mischung 16 h unter Rückfluß gerührt. Nach Abkühlung auf Raumtemperatur wird der Niederschlag abfiltriert und mit Methyl-tert-butylether gewaschen. Die vereinigten Filtrate werden zur Trockne eingeengt und säulenchromatographisch aufgereinigt (Aluminiumoxid, Dichlormethan, Butanon). Ausbeute: 56%.

¹H-NMR (*d*₆-DMSO, 400 MHz): δ = 2.82 (s, 3H), 2.99 (s, 3H), 3.15 (s, 3H), 6.61 (d, 1H), 6.98 (d, 1H), 7.01 (d, 1H), 7.30-7.72 (m, 27H), 8.09-8.16 (m, 3H), 8.35-8.44 (m, 3H).

### Tris-[1-(4'-methoxycarbonylphenyl)-3-methylimidazol-2-yliden-C2,C2']-iridium(III)

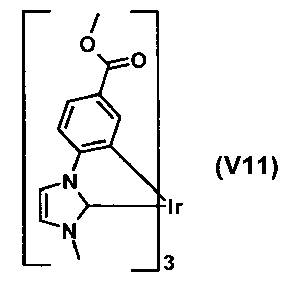

### 1-(4 '-Methoxycarbonylphenyl)-imidazol

Eine Mischung von Imidazol (132 g, 1.9 mol), 4-Fluorbenzoesäuremethylester (170 mL, 1.3 mol) und Kaliumcarbonat (357 g, 2.6 mol) in DMSO (200 mL) wird 3 h bei 120 °C gerührt. Nach Abkühlung auf Raumtemperatur wird die Mischung auf Eiswasser gegeben. Ausgefallenes Produkt wird abfiltriert und mit Wasser gewaschen. Ausbeute: 59%.

¹H-NMR (*d*₆-DMSO, 400 MHz): δ = 3.89 (s, 3H), 7.17 (m_{c}, 1H), 7.86 (d, 2H), 7.90 (m_{c}, 1H), 8.08 (d, 2H), 8.44 (m_{c}, 1H).

### 1-(4'-Methoxycarbonylphenyl)-3-methylimidazolium-iodid

Eine Lösung von 1-(4'-Methoxycarbonylphenyl)-imidazol (153 g, 0.76 mol) in THF/Methanol 1:1 (600 mL) wird mit Methyliodid (196 mL, 2.27 mol) versetzt und 16 h bei Raumtemperatur unter Argon gerührt. Nach Aufkonzentartion der Lösung wird das ausgefallene Produkt abfiltriert und mit THF gewaschen. Ausbeute: 59%.

¹H-NMR (*d*₆-DMSO, 400 MHz): δ = 3.90 (s, 3H), 3.95 (s, 3H), 7.94 (d, 2H), 7.98 (m_{c}, 1H), 8.21 (d, 2H), 8.38 (m_{c}, 1H), 9.89 (s, 1H).

### Tris-(1-(4'-methoxycarbonylphenyl)-3-methylimidazol-2-yliden-C2,C2']-iridium(III)

Eine Suspension von 1-(4'-Methoxycarbonylphenyl)-3-methylimidazolium-iodid (148.0 g, 431 mmol) und Silber(I)oxid (50.4 g, 216 mmol) in Dioxan (400 mL) wird 16 h bei Raumtemperatur unter Argon gerührt. Die Mischung wird mit 1,5-Cyclooctadien-iridium(I)chlorid-Dimer (33.2 g, 43 mmol) versetzt und 16 h unter Rückfluß gerührt. Nach Abkühlung auf Raumtemperatur wird der Niederschlag abfiltriert und mit Dichlormethan gewaschen. Die vereinigten Filtrate werden zur Trockne eingeengt und säulenchromatographisch aufgereinigt (Aluminiumoxid, Essigester/Methanol 1:1). Mischfraktionen, die das Produkt als mer- und fac-Isomer enthalten, werden zur Trockne eingeengt und in Aceton/Methanol 1:1 gelöst. Die Lösung wird mit 1 M Salzsäure versetzt und 16 h unter Rückfluß gerührt. Das ausgefallene mer-Isomer wird abfiltriert und mit wenig Aceton gewaschen. Ausbeute: 45%.

*mer*-isomer: ¹H-NMR (*d*₆-DMSO, 400 MHz): δ = 2.89 (s, 3H), 2.96 (s, 3H), 2.99 (s, 3H), 3.60 (s, 3H), 3.67 (s, 3H), 3.69 (s, 3H), 7.12 (s, 1H), 7.18 (s, 1H), 7.24 (s, 1H), 7.27 (s, 1H), 7.29 (s, 1H), 7.31 (s, 1H),7.38-7.49 (m, 6H), 7.98 (s, 1H), 8.04 (s, 1H), 8.06 (s, 1H).

*fac*-isomer: ¹H-NMR (*d*₆-DMSO, 400 MHz): δ = 3.00 (s, 9H), 3.60 (s, 9H), 7.06 (d, 3H), 7.21 (d, 3H), 7.43-7.51 (m, 6H), 7.99 (d, 3H).

### Iridium(III)bis (2-phenyl)-2-pyrazolinato-N,C²)] (1-phenyl-3-methyl-imidazolin-2-yliden-C, C² (V12)

Die Verbindung (V12) wird in Analogie zu Beispiel 3 in US 2005/0260441 A1 hergestellt mit dem Unterschied, dass anstelle von [(F2ppz)₂)IrCl]₂ [(ppz)_{2I}rCl]₂ eingesetzt wird.

## Patentansprüche

1. Verbindungen der allgemeinen Formel I 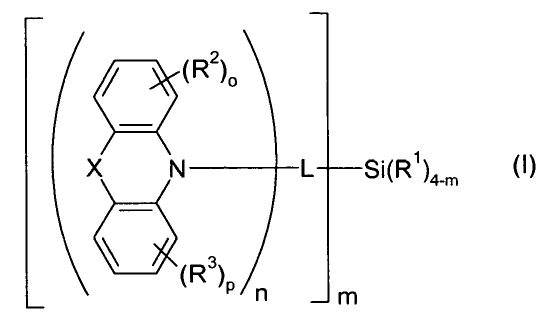 worin bedeuten
X SO₂ oder SO, bevorzugt SO₂;
R¹ jeweils unabhängig voneinander gegebenenfalls substituiertes Aryl, gegebenenfalls substituiertes Heteroaryl oder gegebenenfalls substi- tuiertes Alkyl;
R², R³ jeweils unabhängig voneinander gegebenenfalls substituiertes Alkyl, gegebenenfalls substituiertes Aryl, gegebenenfalls substituiertes He- teroaryl oder Substituenten mit Donor- oder Akzeptorwirkung;
m 1, 2, 3 oder 4, bevorzugt 2, 3 oder 4;
n 1 oder 2;
o, p unabhängig voneinander 0, 1, 2, 3 oder 4, bevorzugt 0, 1 oder 2;
L verbrückende Gruppe ausgewählt aus der Gruppe bestehend aus 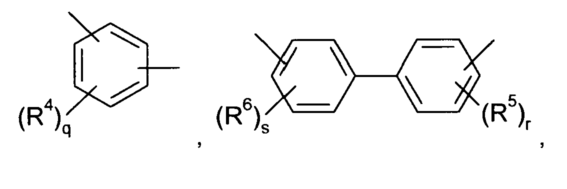 -CH₂-(B)ⱼ- und gegebenenfalls substituiertem Heteroarylen;
R⁴, R⁵, R⁶ jeweils unabhängig voneinander gegebenenfalls substituiertes Alkyl, gegebenenfalls substituiertes Aryl, gegebenenfalls substituiertes He- teroaryl oder Substituenten mit Donor- oder Akzeptorwirkung;
q, r, s unabhängig voneinander 0, 1, 2, 3 oder 4, bevorzugt 0, 1 oder 2;
B eine Alkylenbrücke -CₖH₂ₖ-CH₂-, worin eine oder mehrere nicht be- nachbarte CH₂-Gruppen der Einheit -CₖH₂ₖ- durch Sauerstoff oder NR⁷ ersetzt sein können;
R⁷ Wasserstoff oder Alkyl;
k 1, 2, 3, 4, 5, 6, 7 oder 8; und
j 0 oder 1.

2. Verbindungen nach Anspruch 1, worin bedeuten:
X SO₂;
m 2, 3 oder 4;
o, p 0, 1 oder 2;
L 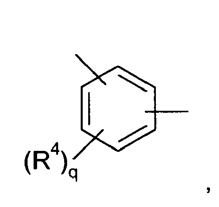 bevorzugt 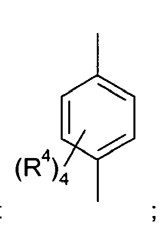 und
q 0, 1 oder 2.

3. Verbindungen nach Anspruch 1 oder 2, **dadurch gekennzeichnet, dass** mindestens zwei der an das Si gebundenen Reste oder Gruppen L oder R¹ aromatische Reste oder Gruppen sind.

4. Verbindungen nach Anspruch 1, worin bedeuten:
L 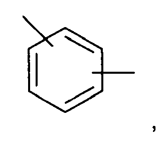 bevorzugt 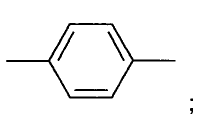 und
o, p und q 0.

5. OLED enthaltend mindestens eine Verbindung nach einem der Ansprüche 1 bis 4.

6. OLED nach Anspruch 5, **dadurch gekennzeichnet, dass** die mindestens eine Verbindung nach einem der Ansprüche 1 bis 4 als Matrixmaterial und/oder Blockermaterial eingesetzt wird.

7. OLED nach Anspruch 6, **dadurch gekennzeichnet, dass** das Matrixmaterial und/oder Blockermaterial gemeinsam mit einem Triplett-Emitter eingesetzt wird.

8. Licht-emittierende Schicht enthaltend mindestens eine Verbindung nach einem der Ansprüche 1 bis 4 und mindestens einen Triplett-Emitter.

9. Loch- und/oder Excitonenblockerschicht enthaltend mindestens eine Verbindung nach einem der Ansprüche 1 bis 4.

10. Verfahren zur Herstellung von Verbindungen nach einem der Ansprüche 1 bis 4 umfassend die Schritte:
(i) Herstellung eines Phenothiazinderivats (II) 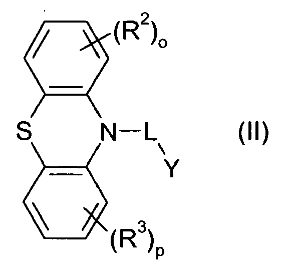 worin bedeuten
R², R³ jeweils unabhängig voneinander gegebenenfalls substituiertes Alkyl, gegebenenfalls substituiertes Aryl oder gegebenenfalls substituiertes Heteroaryl oder ein Rest mit Donor- oder Akzeptorwirkung;
o, p unabhängig voneinander 0, 1, 2, 3 oder 4, bevorzugt 0, 1 oder 2;
L verbrückende Gruppe ausgewählt aus der Gruppe bestehend aus 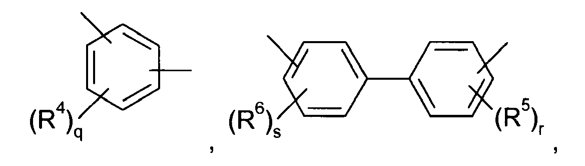 -CH₂-(B)ⱼ- und gegebenenfalls substituiertem Heteroarylen;
R⁴, R⁵, R⁶ jeweils unabhängig voneinander gegebenenfalls substituiertes Alkyl, gegebenenfalls substituiertes Aryl, gegebenenfalls substituiertes He- teroaryl oder Substituenten mit Donor- oder Akzeptorwirkung;
q, r, s unabhängig voneinander 0, 1, 2, 3 oder 4, bevorzugt 0, 1 oder 2;
B eine Alkylenbrücke -CₖH₂ₖ-CH₂-, worin eine oder mehrere nicht be- nachbarte CH₂-Gruppen der Einheit -CₖH₂ₖ- durch Sauerstoff oder NR⁷ ersetzt sein können;
R⁷ Wasserstoff oder Alkyl;
k 1, 2, 3, 4, 5, 6, 7 oder 8; und
j 0 oder 1; und
Y Halogen, bevorzugt ausgewählt aus der Gruppe bestehend aus F, Cl und Br, besonders bevorzugt Br;
durch Umsetzung von Phenothiazin oder eines Phenothiazinderivats der Formel (III) 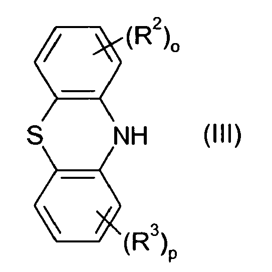 worin R², R³, o und p die vorstehend genannten Bedeutungen aufweisen, mit einer bifunktionellen Verbindung der Formel (IV)
Z-L-Y (IV)
worin L und Y die vorstehend genannten Bedeutungen aufweisen und
Z Iod, Fluor, Brom oder Tosyl bedeutet;
(ii) Herstellung von Phenothiazinderivaten der Formel (V) 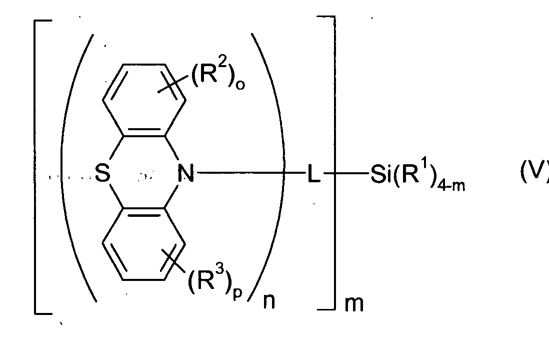 worin die Reste und Indizes die vorstehend genannten Bedeutungen aufweisen, und
m 1, 2, 3 oder 4, und
n 1 oder 2 bedeuten,
durch Umsetzung des Phenothiazinderivats (II) mit einem Halogenalkyl/arylsilan der allgemeinen Formel (VIa) oder mit einem Alkoxysilan der allgemeinen Formel (VIb)
(R⁸)ₜSi(Hal)₄₋ₜ (VIa)
(R⁸)ₜSi(OR⁹)₄₋ₜ (VIb)
worin bedeuten
R⁸ gegebenenfalls substsituiertes Aryl, gegebenenfalls substituiertes He- teroaryl oder gegebenenfalls substituiertes Alkyl, bevorzugt Methyl;
Hal Halogen, bevorzugt Cl; und
t 1, 2 oder 3; und
R⁹ Alkyl, bevorzugt Ethyl oder Methyl;
(iii) Herstellung der Phenothiazin-S-oxid- oder -S,S-dioxid-Derivate der Formel (I)
durch Umsetzung der Phenothiazinderivate der Formel (V) mit einem Oxidationsmittel.

11. Verwendung von Verbindungen nach einem der Ansprüche 1 bis 4 in OLEDs, bevorzugt als Matrixmaterialien und/oder Blockermaterialien für Triplettemitter.

12. Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Digitalkameras, Fahrzeugen sowie Zielanzeigen an Bussen und Bahnen und Beleuchtungseinheiten enthaltend mindestens eine organische Leuchtdiode gemäß einem der Ansprüche 5 bis 7.

## Claims

1. A compound of the general formula I 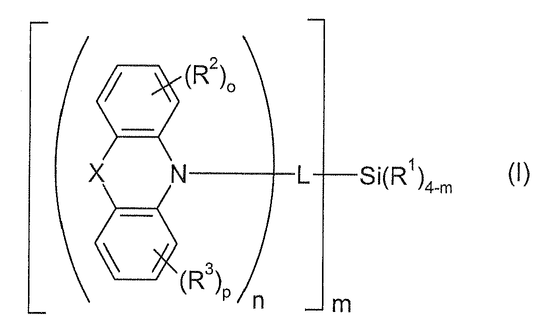 in which
X is SO₂ or SO, preferably SO₂;
R¹ is in each case independently optionally substituted aryl, optionally substituted heteroaryl or optionally substituted alkyl;
R² R³ are in each case independently optionally substituted alkyl, optionally substituted aryl, optionally substituted heteroaryl or substituents having donor or acceptor action;
m is 1, 2, 3 or 4, preferably 2, 3 or 4;
n is 1 or 2;
o, p are each independently 0, 1, 2, 3 or 4, preferably 0, 1 or 2;
L is a bridging group selected from the group consisting of 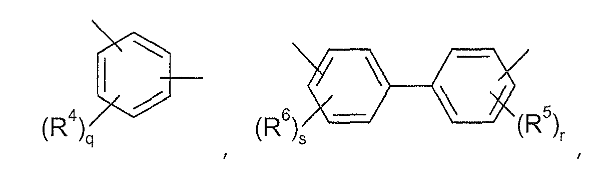 -CH₂-(B)ⱼ- and optionally substituted heteroarylene;
R⁴, R⁵, R⁶ are in each case independently optionally substituted alkyl, optionally substituted aryl, optionally substituted heteroaryl or substituents having donor or acceptor action;
q, r, s are each independently 0, 1, 2, 3 or 4, preferably 0, 1 or 2;
B is an alkylene bridge -CₖH₂ₖ-CH₂-, in which one or more nonadjacent CH₂ groups of the -CₖH₂ₖ- unit may be replaced by oxygen or NR⁷;
R⁷ is hydrogen or alkyl;
k is 1, 2, 3, 4, 5, 6, 7 or 8; and
j is 0 or 1.

2. A compound according to claim 1, in which:
X is SO₂;
m is 2, 3 or 4;
o, p are each 0, 1 or 2;
L 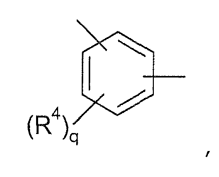 preferably 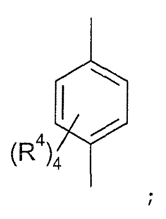 and
q is 0, 1 or 2.

3. A compound according to claim 1 or 2, wherein at least two of the L or R¹ radicals or groups bonded to the Si are aromatic radicals or groups.

4. A compound according to claim 1, in which:
L 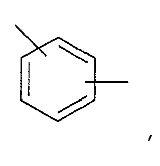 preferably 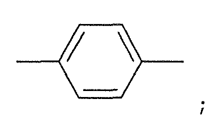 and
o, p and q are each 0.

5. An OLED comprising at least one compound according to any of claims 1 to 4.

6. The OLED according to claim 5, wherein the at least one compound according to any one of claims 1 to 4 is used as a matrix material and/or blocker material.

7. The OLED according to claim 6, wherein the matrix material and/or blocker material is used together with a triplet emitter.

8. A light-emitting layer comprising at least one compound according to any one of claims 1 to 4 and at least one triplet emitter.

9. A hole and/or exciton blocker layer comprising at least one compound according to any one of claims 1 to 4.

10. A process for preparing compounds according to any of claims 1 to 4, comprising the steps of:
(i) preparing a phenothiazine derivative (II) 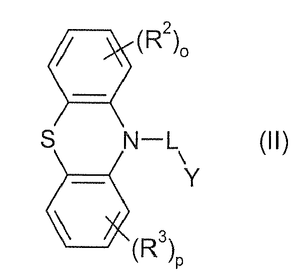 in which
R², R³ are in each case independently optionally substituted alkyl, optionally substituted aryl, optionally substituted heteroaryl or a radical having donor or acceptor action;
o, p are each independently 0, 1, 2, 3 or 4, preferably 0, 1 or 2;
L is a bridging group selected from the group consisting of 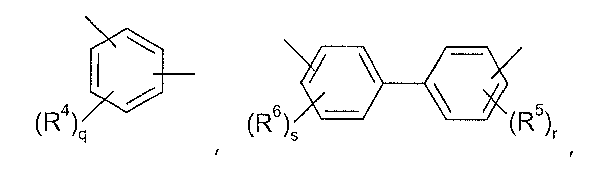 -CH₂-(B)ⱼ- and optionally substituted heteroarylene;
R⁴, R⁵, R⁶ are in each case independently optionally substituted alkyl, optionally substituted aryl, optionally substituted heteroaryl or substituents having donor or acceptor action;
q, r, s are each independently 0, 1, 2, 3 or 4, preferably 0, 1 or 2;
B is an alkylene bridge -CₖH₂ₖ-CH₂, in which one or more nonadjacent CH₂ groups of the - CₖH₂ₖ- unit may be replaced by oxygen or NR⁷;
R⁷ is hydrogen or alkyl;
k is 1, 2, 3, 4, 5, 6, 7 or 8; and
j is 0 or 1; and
Y is halogen, preferably selected from the group consisting of F, Cl and Br, more preferably Br;
by reacting phenothiazine or a phenothiazine derivative of the formula (III) 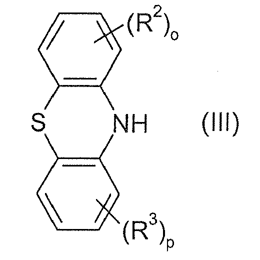 in which R², R³, o and p are each as defined above with a bifunctional compound of the formula (IV)
Z-L-Y (IV)
in which L and Y are each as defined above and
Z is iodine, fluorine, bromine or tosyl;
(ii) preparation of phenothiazine derivatives of the formula (V) 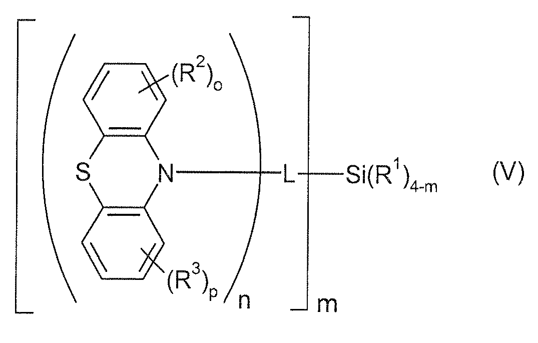 in which the radicals and indices are each as defined above, and
m is 1, 2, 3 or 4, and
n is 1 or 2,
by reacting the phenothiazine derivative (II) with a haloalkyl/arylsilane of the general formula (VIa) or with an alkoxysilane of the general formula (VIb)
(R⁸) ₜSi (Hal ) ₄₋ₜ (VIa)
(R ⁸) ₜSi (OR⁹) ₄₋ₜ (VIb)
in which
R⁸ is optionally substituted aryl, optionally substituted heteroaryl or optionally substituted alkyl, preferably methyl;
Hal is halogen, preferably Cl; and
t is 1, 2 or 3; and
R⁹ is alkyl, preferably ethyl or methyl;
(iii) preparing the phenothiazine S-oxide or S,S- dioxide derivatives of the formula (I)
by reacting the phenothiazine derivatives of the formula (V) with an oxidizing agent.

11. The use of compounds according to any one of claims 1 to 4 in OLEDs, preferably as matrix materials and/or as blocker materials for triplet emitters.

12. A device selected from the group consisting of stationary visual display units such as visual display units of computers, televisions, visual display units in printers, kitchen appliances and advertising panels, illuminations, information panels and mobile visual display units such as visual display units in cellphones, laptops, digital cameras, vehicles and destination displays on buses and trains and illumination units, comprising at least one organic light-emitting diode according to any one of claims 5 to 7.

## Revendications

1. Composés de formule générale I 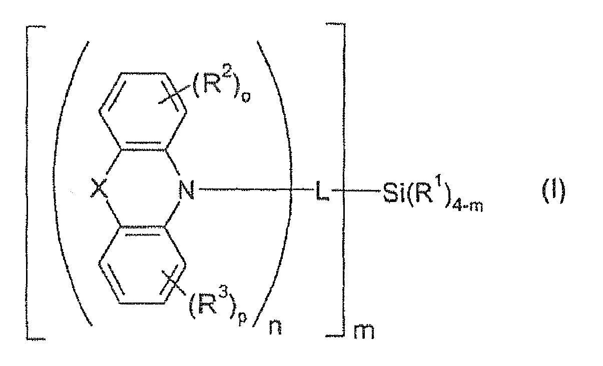 dans laquelle
X représente SO₂ ou SO, de préférence SO₂ ;
R¹ représentent chacun, indépendamment les uns des autres, un groupe aryle éventuellement substitué, hétéroaryle éventuellement substitué ou alkyle éventuellement substitué ;
R², R³ représentent chacun, indépendamment l'un de l'autre, un groupe alkyle éventuellement substitué, aryle éventuellement substitué, hétéroaryle éventuellement substitué ou des substituants agissant comme donneur ou accepteur ;
m représente 1, 2, 3 ou 4, de préférence 2, 3 ou 4 ;
n représente 1 ou 2 ;
o, p représentent, indépendamment l'un de l'autre, 0, 1, 2, 3 ou 4, de préférence 0, 1 ou 2 ;
L représente un groupe pontant choisi dans l'ensemble constitué par 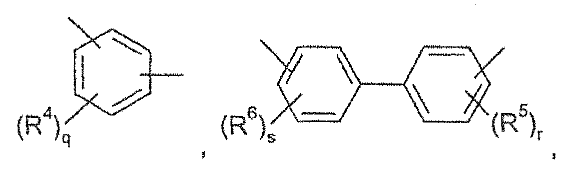 -CH₂-(B)_{J}- et un groupe hétéroarylène éventuellement substitué ;
R⁴, R⁵, R⁶ représentent, chacun indépendamment, un groupe alkyle éventuellement substitué, aryle éventuellement substitué, hétéroaryle éventuellement substitué ou des substituants agissant comme donneur ou accepteur ;
q, r, s représentent, chacun indépendamment, 0, 1, 2, 3 ou 4, de préférence 0, 1 ou 2 ;
B représente un pont alkylène -CₖH₂ₖ-CH₂-, dans lequel un ou plusieurs groupes CH₂ non contigus de l'unité -CₖH₂ₖ- peuvent être remplacés par un atome d'oxygène ou NR⁷ ;
R⁷ représente un atome d'hydrogène ou un groupe alkyle ;
k représente 1, 2, 3, 4, 5, 6, 7 ou 8 ; et
j représente 0 ou 1.

2. Composés selon la revendication 1, dans lesquels :
X représente SO₂;
m représente 2, 3 ou 4 ;
o, p représente 0, 1 ou 2 ;
L représente 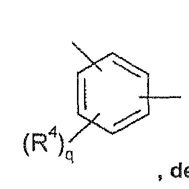 , de preference 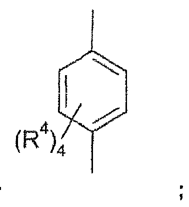 et
q représente 0, 1 ou 2.

3. Composés selon la revendication 1 ou 2, **caractérisés en ce qu'**au moins deux des groupes ou radicaux L ou R¹ liés à Si sont des groupes ou radicaux aromatiques.

4. Composés selon la revendication 1, dans lesquels:
L représente 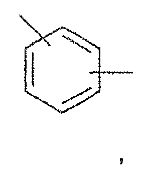 , de preference 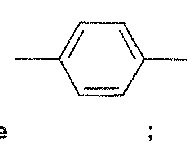 et
o, p et q représentent 0.

5. OLED (diode électroluminescente organique) contenant un composé selon l'une quelconque des revendications 1 à 4,

6. OLED selon la revendication 5, **caractérisée en ce que** ledit au moins un composé selon l'une quelconque des revendications 1 à 4 est utilisé en tant que matériau de matrice et/ou matériau bloquant.

7. OLED selon la revendication 6, **caractérisée en ce que** le matériau de matrice et/ou le matériau bloquant est/sont utilisé(s) conjointement avec un émetteur de triplets.

8. Couche photo-émettrice contenant au moins un composé selon l'une quelconque des revendications 1 à 4 et au moins un émetteur de triplets.

9. Couche de blocage de trous et/ou d'excitons, contenant au moins un composé selon l'une quelconque des revendications 1 à 4.

10. Procédé pour la préparation de composés selon l'une quelconque des revendications 1 à 4, comprenant les étapes :
(i) Préparation d'un dérivé de phénothiazine (II) 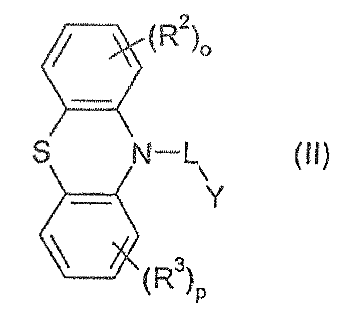 dans lequel
R², R³ représentent chacun, indépendamment l'un de l'autre, un groupe alkyle éventuellement substitué, aryle éventuellement substitué ou hétéroaryle éventuellement substitué ou un radical agissant comme donneur ou accepteur ;
o, p représentent, indépendamment l'un de l'autre, 0, 1, 2, 3 ou 4, de préférence 0, 1 ou 2 ;
L représente un groupe pontant choisi dans l'ensemble constitué par 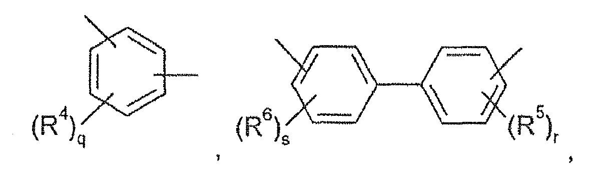 -CH₂-(B)ⱼ- et un groupe hétéroarylène éventuellement substitué ;
R⁴, R⁵, R⁶ représentent, chacun indépendamment, un groupe alkyle éventuellement substitué, aryle éventuellement substitué, hétéroaryle éventuellement substitué ou des substituants agissant comme donneur ou accepteur ;
q, r, s représentent, chacun indépendamment, 0, 1, 2, 3 ou 4, de préférence 0, 1 ou 2 ;
B représente un pont alkylène -CₖH₂ₖ-CH₂-, dans lequel un ou plusieurs groupes CH₂ non contigus de l'unité -CₖH₂ₖ- peuvent être remplacés par un atome d'oxygène ou NR⁷ ;
R⁷ représente un atome d'hydrogène ou un groupe alkyle ;
k représente 1, 2, 3, 4, 5, 6, 7 ou 8 ; et
j représente 0 ou 1 ; et
Y représente un atome d'halogène, de préférence choisi dans l'ensemble constitué par F, Cl et Br ; de façon particulièrement préférée Br ;
par mise en réaction de phénothiazine ou d'un dérivé de phénothiazine de formule (III) 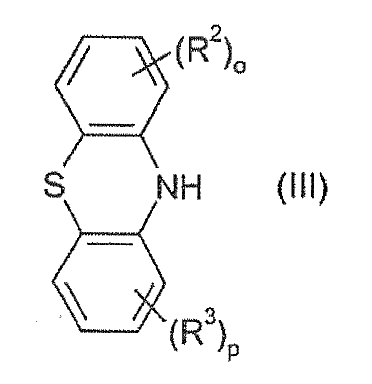 dans laquelle R², R³, o et p ont les significations données précédemment, avec un composé bifonctionnel de formule (IV)
Z-L-Y (IV)
dans laquelle L et Y ont les significations données précédemment et
Z représente un atome d'iode, de fluor, de brome ou le groupe tosyle ;
(ii) Préparation de dérivés de phénothiazine de formule (V) 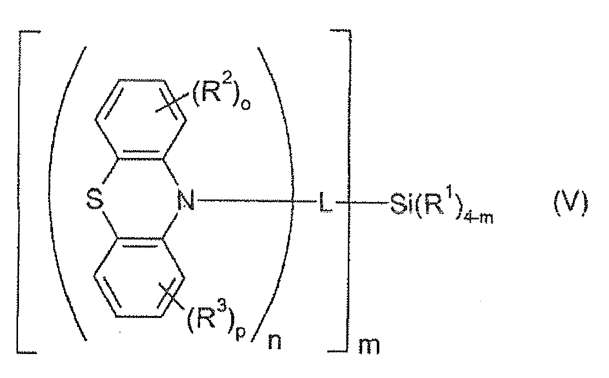 dans laquelle les radicaux et indices ont les significations indiquées précédemment, et
m représente 1, 2, 3 ou 4, et
n représente 1 ou 2,
par mise en réaction du dérivé de phénothiazine (II) avec un halogénoalkyl/arylsilane de formule générale (VIa) ou avec un alcoxysilane de formule générale (VIb)
(R⁸) ₜSi (Hal) ₄₋ₜ (VIa)
(R⁸) ₜSi (OR⁹) ₄₋ₜ (VIb)
où
R⁸ représente un groupe alkyle éventuellement substitué, hétéroaryle éventuellement substitué ou alkyle éventuellement substitué, de préférence méthyle ;
Hal représente un atome d'halogène, de préférence de chlore ; et
t représente 1, 2 ou 3 ; et
R⁹ représente un groupe alkyle, de préférence éthyle ou méthyle ;
(iii) Préparation des dérivés de phénothiazine-S- oxyde ou -S,S-dioxyde de formule (I)
par mise en réaction des dérivés de phénothiazine de formule (V) avec un oxydant.

11. Utilisation de composés selon l'une quelconque des revendications 1 à 4 dans des OLED, de préférence en tant que matériaux de matrice et/ou matériaux bloquants pour émetteurs de triplets.

12. Dispositif choisi dans le groupe constitué par des écrans fixes tels que des écrans d'ordinateurs, des téléviseurs, des écrans dans des imprimantes, des appareils de cuisine ainsi que des tableaux publicitaires, des éclairages, des tableaux indicateurs et des écrans mobiles tels que des écrans dans des téléphones portables, des ordinateurs portables, des appareils photographiques numériques, des véhicules ainsi que des afficheurs de destination dans des autobus et des trains, et des unités d'éclairage contenant au moins une diode luminescente organique selon l'une quelconque des revendications 5 à 7.
